# EUROPEAN PATENT APPLICATION

(11) **EP 4 806 515 A2**
(43) Date of publication of application: **16.09.2026**
(21) Application number: 26181500.5
(22) Date of filing: 12.04.2019
(51) Int. Cl.: A61P 35/02

(54) **PIM KINASE INHIBITORS FOR TREATMENT OF MYELOPROLIFERATIVE NEOPLASMS AND FIBROSIS ASSOCIATED WITH CANCER**

(30) Priority: 13.04.2018 US 201862657563 P; 13.04.2018 US 201862657540 P; 09.10.2018 US 201862743469 P; 30.10.2018 US 201862753025 P; 30.10.2018 US 201862753023 P
(62) Document number(s) of the earlier application(s) in accordance with Art. 76 EPC: 19785217.1 / 3 773 560
(71) Applicant: Sumitomo Pharma America, Inc., Marlborough, Massachusetts 01752 (US)
(72) Inventor: FOULKS, Jason Marc, Cambridge, 02139 (US); WARNER, Steven L., Cambridge, 02139 (US)
(74) Representative: Carpmaels & Ransford LLP

(57) **Abstract**

Methods for treatment of myeloproliferative neoplasms and/or fibrosis associated with cancer are provided. The disclosed methods comprise administering a PIM kinase inhibitor, and optionally a JAK kinase inhibitor or other therapeutic agent, to a mammal in need thereof.

## Description

### RELATED APPLICATIONS

This application claims the benefit of U.S. Provisional Application No. 62/657,540, filed on April 13, 2018, U.S. Provisional Application No. 62/657,563, filed on April 13, 2018, U.S. Provisional Application No. 62/743,469, filed on October 9, 2018, U.S. Provisional Application No. 62/753,023, filed on October 30, 2018, and U.S. Provisional Application No. 62/753,025, filed on October 30, 2018. The entire teachings of the above applications are incorporated herein by reference.

### BACKGROUND

Despite meaningful advancements in the treatment of many hematological malignancy indications, significant unmet needs persist. Drug resistance and poor overall survival remain a substantial challenge that will require the development and evaluation of novel therapeutic agents.

PIM kinases are frequently overexpressed in various hematologic and solid tumors, allowing cancer cells to evade apoptosis and promoting tumor growth. In inflammatory disorders, PIM-1 kinase has been shown to mediate interleukin-22 signaling in cell-based and animal models.

Thus, there is a need for treatment regimens that exploit the activity of PIM kinase inhibitors to treat hematological malignancies.

### SUMMARY

This disclosure is based, at least in part, on the discovery that the compound of structural formula I is a PIM kinase inhibitor, and demonstrates profound preclinical activity in models of leukemia and myeloproliferative disorders (*e.g.*, myelofibrosis).

Accordingly, provided herein is a method for treating a myeloproliferative neoplasm (*e.g*., myelofibrosis) in a mammal in need thereof. The method comprises administering to the mammal from about 250 mg to about 2.5 g (*e.g.,* from about 300 mg to about 1.5 g, from about 450 mg to about 1.5 g) per day of a compound represented by structural formula 1: 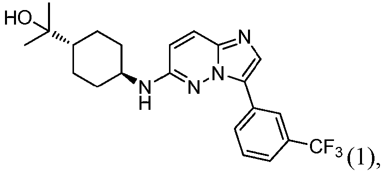 or a pharmaceutically acceptable salt thereof; and an effective amount of ruxolitinib, or a pharmaceutically acceptable salt thereof.

Also provided herein is a pharmaceutical composition comprising a pharmaceutically acceptable carrier or excipient; a compound of structural formula 1, or a pharmaceutically acceptable salt thereof; and ruxolitinib, or a pharmaceutically acceptable salt thereof.

Also provided herein is a kit comprising a compound of structural formula 1, or a pharmaceutically acceptable salt thereof; ruxolitinib, or a pharmaceutically acceptable salt thereof; and written instructions for administering the compound of structural formula 1, or a pharmaceutically acceptable salt thereof, in combination with ruxolitinib, or a pharmaceutically acceptable salt thereof, to treat a myeloproliferative neoplasm (*e.g.*, myelofibrosis).

These and other aspects of embodiments of the disclosure will be apparent upon reference to the following detailed description. To this end, various references are set forth herein which describe in more detail certain background information, procedures, compounds and/or compositions, and are each hereby incorporated by reference in their entirety.

### BRIEF DESCRIPTION OF THE DRAWINGS

In the figures, identical reference numbers identify similar elements. The sizes and relative positions of elements in the figures are not necessarily drawn to scale and some of these elements are enlarged and positioned to improve figure legibility. Further, the particular shapes of the elements as drawn are not intended to convey any information regarding the actual shape of the particular elements, and have been solely selected for ease of recognition in the figures.
FIGs. 1A-C show increased expression of PIM1 mRNA in myeloproliferative neoplasm hematopoietic progenitors and long-term hematopoietic stem cells.
FIGs. 2A-C show PIM1 protein expression in myelofibrosis patients as immunoblotting results.
FIGs. 3A-C show inhibition of hematopoietic cells for PIM1 knockdown compared to wild-type JAK2 expressing cells.
FIGs. 4A-E illustrate Compound 1 inducing selective inhibition of proliferation in cells expressing JAK2 V617F.
FIGs. 5A-B depict myeloproliferative neoplasm inhibition in CK34+ cells when treated with Compound 1.
FIGs. 6A-6F show a synergistic relationship between Compound 1 (a PIM kinase inhibitor) and ruxolitinib (a JAK2 inhibitor) for inducing apoptosis in hematopoietic cells expressing JAK2 V617F.
FIGs. 7A-C illustrate that Compound 1 overcomes resistance to a JAK2 inhibitor (ruxolitinib) in cells expressing JAK2 V617F.
FIG. 8 presents spleen and bone marrow samples of knock-in mice expressing JAK2 V617F that develop high-grade myelofibrosis.
FIG. 9A-E are histograms comparing treatment with Compound 1 alone or in combination with ruxolitinib to improve blood cell counts (white blood cells and neutrophils) and spleen size in a myelofibrosis mouse model.
FIG. 10 is a depiction of tissue samples for comparing treatment using Compound 1 alone or in combination with ruxolitinib and the related reduction of fibrosis in myelofibrosis mouse models.
FIGs. 11A-E show genes related to TNFα and WNT signaling pathways are downregulated in JAK2 V617F expressing hematopoietic progenitors after treatment with Compound 1 alone or in combination with ruxolitinib.
FIG. 12 shows a plot of Compound 1 having anti-proliferative activity against prostate cancer cells in a colony formation assay.
FIG. 13 shows the actual results from the colony formation assay.
FIG. 14 is a plot of tumor volume against the number of days for a prostate adenocarcinoma xenograft model.
FIG. 15 illustrates the potency of Compound 1 compared to Compound A with respect to inhibition of p-BAD.

### DETAILED DESCRIPTION

In the following description, certain specific details are set forth in order to provide a thorough understanding of various embodiments of the disclosure. However, one skilled in the art will understand that embodiments of the disclosure may be practiced without these details.

Unless the context requires otherwise, throughout the present specification and claims, the word "comprise" and variations thereof, such as "comprises" and "comprising," are to be construed in an open, inclusive sense (i.e., as "including, but not limited to").

Reference throughout this specification to "one embodiment" or "an embodiment" means that a particular feature, structure or characteristic described in connection with the embodiment is included in at least one embodiment of the present disclosure. Thus, the appearances of the phrases "in one embodiment" or "in an embodiment" in various places throughout this specification are not necessarily all referring to the same embodiment. Furthermore, the particular features or characteristics may be combined in any suitable manner in one or more embodiments.

"Amino" refers to the -NH₂ radical.

"Cyano" refers to the CN radical.

"Hydroxy" or "hydroxyl" refers to the OH radical.

"Nitro" refers to the NO₂ radical.

"Alkyl" refers to a straight or branched hydrocarbon chain radical consisting solely of carbon and hydrogen atoms, which is saturated or unsaturated (*i.e.,* contains one or more double and/or triple bonds), having from one to twelve carbon atoms (C₁-C₁₂ alkyl), preferably one to eight carbon atoms (C₁-C₈ alkyl) or one to six carbon atoms (C₁-C₆ alkyl), and which is attached to the rest of the molecule by a single bond, e.g., methyl, ethyl, n-propyl, 1-methylethyl(iso propyl), n-butyl, n-pentyl, 1,1-dimethylethyl (t-butyl), 3-methylhexyl, 2-methylhexyl, ethenyl, prop-1-enyl, but-1-enyl, pent-1-enyl, penta-1,4-dienyl, ethynyl, propynyl, butynyl, pentynyl, hexynyl, and the like. In embodiments, "alkyl" is saturated alkyl. Unless stated otherwise specifically in the specification, alkyl groups are optionally substituted.

"Alkoxy" refers to a radical of the formula -ORₐ where Rₐ is an alkyl radical as defined above containing one to twelve carbon atoms. "Haloalkoxy" is an alkoxy moiety comprising at least one halo substituent. Unless stated otherwise specifically in the specification, alkoxy and haloalkoxy groups may be optionally substituted.

"Alkylamino" or "alkylaminyl" refers to a radical of the formula -NHRₐ or - NRₐRₐ where each Rₐ is, independently, an alkyl radical as defined above containing one to twelve carbon atoms. Unless stated otherwise specifically in the specification, an alkylamino group may be optionally substituted.

"Alkylsulfonamidyl" refers to a radical of the formula -S(O₂)NHRₐ or - S(O₂)NRₐRₐ where each Rₐ is, independently, an alkyl radical as defined above containing one to twelve carbon atoms. Unless stated otherwise specifically in the specification, an alkylsulfonamidyl group may be optionally substituted.

"Alkylcarbonyl" is radical of formula -C(=O)Rₐ, wherein Rₐ is an alkyl group. Unless stated otherwise specifically in the specification, alkylcarbonyl groups are optionally substituted.

"Carbocyclic" refers to a ring, wherein each atom forming the ring is carbon. Aryl and cycloalkyl groups are carbocyclic.

"Halo" or "halogen" refers to bromo, chloro, fluoro or iodo.

"Haloalkyl" refers to an alkyl radical, as defined above, that is substituted by one or more halo radicals, as defined above, e.g., trifluoromethyl, difluoromethyl, trichloromethyl, 2,2,2-trifluoroethyl, 1,2-difluoroethyl, 3-bromo-2-fluoropropyl, 1,2-dibromoethyl, and the like. Unless stated otherwise specifically in the specification, a haloalkyl group may be optionally substituted.

"Heterocyclyl" or "heterocyclic ring" refers to a stable 3- to 18-membered non-aromatic ring radical which consists of two to twelve carbon atoms and from one to six heteroatoms selected from the group consisting of nitrogen, oxygen and sulfur. Unless stated otherwise specifically in the specification, the heterocyclyl radical may be a monocyclic, bicyclic, tricyclic or tetracyclic ring system, which may include fused or bridged ring systems; and the nitrogen, carbon or sulfur atoms in the heterocyclyl radical may be optionally oxidized; the nitrogen atom may be optionally quaternized; and the heterocyclyl radical may be partially or fully saturated. Examples of such heterocyclyl radicals include, but are not limited to, dioxolanyl, thienyl[1,3]dithianyl, decahydroisoquinolyl, imidazolinyl, imidazolidinyl, isothiazolidinyl, isoxazolidinyl, morpholinyl, octahydroindolyl, octahydroisoindolyl, 2-oxopiperazinyl, 2-oxopiperidinyl, 2-oxopyrrolidinyl, oxazolidinyl, piperidinyl, piperazinyl, 4-piperidonyl, pyrrolidinyl, pyrazolidinyl, quinuclidinyl, thiazolidinyl, tetrahydrofuryl, trithianyl, tetrahydropyranyl, thiomorpholinyl, thiamorpholinyl, 1-oxo-thiomorpholinyl, and 1,1-dioxo-thiomorpholinyl. Unless stated otherwise specifically in the specification. Unless stated otherwise specifically in the specification, a heterocyclyl group may be optionally substituted.

The term "substituted" used herein means any of the above groups wherein at least one hydrogen atom is replaced by a bond to a non-hydrogen atoms such as, but not limited to: a halogen atom such as F, Cl, Br, and I; an oxygen atom in groups such as hydroxyl groups, alkoxy groups, and ester groups; a sulfur atom in groups such as thiol groups, thioalkyl groups, sulfone groups, sulfonyl groups, and sulfoxide groups; a nitrogen atom in groups such as amines, amides, alkylamines, dialkylamines, arylamines, alkylarylamines, diarylamines, N-oxides, imides, and enamines; a silicon atom in groups such as trialkylsilyl groups, dialkylarylsilyl groups, alkyldiarylsilyl groups, and triarylsilyl groups; and other heteroatoms in various other groups. "Substituted" also means any of the above groups in which one or more hydrogen atoms are independently replaced by a higher-order bond (*e.g.,* a double- or triple-bond) to a heteroatom such as oxygen in oxo, carbonyl, carboxyl, and ester groups; and nitrogen in groups such as imines, oximes, hydrazones, and nitriles. For example, "substituted" includes any of the above groups in which one or more hydrogen atoms are independently replaced with -NR_{g}Rₕ, -NR_{g}C(=O)Rₕ, -NR_{g}C(=O)NR_{g}Rₕ, - NR_{g}C(=O)ORₕ, -NR_{g}SO₂Rₕ, -OC(=O)NR_{g}Rₕ, -OR_{g}, -SR_{g}, -SOR_{g}, -SO₂R_{g}, -OSO₂R_{g}, - SO₂OR_{g}, =NSO₂R_{g}, and/or -SO₂NR_{g}Rₕ. "Substituted" also means any of the above groups in which one or more hydrogen atoms are independently replaced with -C(=O)R_{g}, -C(=O)OR_{g}, -C(=O)NR_{g}Rₕ, -CH₂SO₂R_{g}, and/or -CH₂SO₂NR_{g}Rₕ. In the foregoing, R_{g} and Rₕ are the same or different and independently hydrogen, alkyl, alkoxy, alkylamino, thioalkyl, aryl, aralkyl, cycloalkyl, cycloalkylalkyl, haloalkyl, heterocyclyl, N-heterocyclyl, heterocyclylalkyl, heteroaryl, N-heteroaryl and/or heteroarylalkyl. "Substituted" further means any of the above groups in which one or more hydrogen atoms are independently replaced by a bond to an amino, cyano, hydroxyl, imino, nitro, oxo, thioxo, halo, alkyl, alkoxy, alkylamino, thioalkyl, aryl, aralkyl, cycloalkyl, cycloalkylalkyl, haloalkyl, heterocyclyl, *N*-heterocyclyl, heterocyclylalkyl, heteroaryl, N-heteroaryl and/or heteroarylalkyl group. In addition, each of the foregoing substituents may also be optionally substituted with one or more of the above substituents.

"Compounds of the disclosure" or "disclosed compounds" refer to compounds targeting PIM kinase or JAK, for example a compound of structure (I), (II), or (III), or a compound targeting PIM kinase or JAK kinase known in the art or incorporated by reference.

"PIM kinase inhibitor," as used herein, refers to a compound that inhibits the activity of a Proviral Insertion in Murine Lymphomas (PIM) kinase. PIM kinase refers to a family of serine/threonine kinases that regulate several signaling pathways that are fundamental to cancer development and progression. The PIM family includes PIM1, PIM2, and PIM3. A PIM inhibitor can have activity on all PIM family members or one or more subtypes of the PIM family. A PIM inhibitor can be selected for action on a specific subtype of the PIM family, for example a PIM inhibitor can act at a lower concentration on PIM1 of the PIM family than on other members of the PIM family. More specifically, a PIM inhibitor can selectively act on PIM1 compared to its action on, for example, PIM3. In some embodiments, a PIM inhibitor inhibits one or more PIM subtypes comprising PIM1.

A "PIM1 inhibitor," for example, refers to a compound that functions as an inhibitor to PIM1. In some embodiments, a PIM1 inhibitor is selective for PIM1 (*e.g.,* acts at a lower concentration) compared to other PIM subtypes.

"JAK inhibitor," as used herein, refers to a compound that inhibits the activity of a Janus kinase. Janus kinase refers to a family of intracellular, nonreceptor tyrosine kinases that transduce cytokine-mediated signals via the JAK-STAT pathway. The JAK family includes JAK1, JAK2, JAK3, and TYK2. A JAK inhibitor can have activity on all JAK family members or one or more subtypes of the JAK family. A JAK inhibitor can be selected for action on a specific subtype of the JAK family, for example, a JAK inhibitor can act at a lower concentration on JAK2 of the JAK family than on other members of the JAK family. More specifically, a JAK inhibitor can selectively act on JAK2 compared to its action on, for example, JAK1. In some embodiments, a JAK inhibitor inhibits one or more JAK subtypes comprising JAK2.

A "JAK2 inhibitor," for example, refers to a compound that functions as an inhibitor to JAK2. In some embodiments, a JAK2 inhibitor is selective for JAK2 (e.g., acts at a lower concentration) compared to other JAK subtypes.

Embodiments of the present disclosure also include administration of prodrugs of the disclosed compounds. "Prodrug" is meant to indicate a compound that may be converted under physiological conditions or by solvolysis to a biologically active compound of the disclosure. Thus, the term "prodrug" refers to a metabolic precursor of a compound of the disclosure that is pharmaceutically acceptable. A prodrug may be inactive when administered to a subject in need thereof, but is converted in vivo to an active compound of the disclosure. Prodrugs are typically rapidly transformed in vivo to yield the parent compound of the disclosure, for example, by hydrolysis in blood. The prodrug compound often offers advantages of solubility, tissue compatibility or delayed release in a mammalian organism (*see,* Bundgard, H., Design of Prodrugs (1985), pp. 7 9, 21 24 (Elsevier, Amsterdam)). A discussion of prodrugs is provided in Higuchi, T., et al., A.C.S. Symposium Series, Vol. 14, and in Bioreversible Carriers in Drug Design, Ed. Edward B. Roche, American Pharmaceutical Association and Pergamon Press, 1987.

The term "prodrug" is also meant to include any covalently bonded carriers, which release the active compound of the disclosure in vivo when such prodrug is administered to a mammalian subject. Prodrugs of a compound of the disclosure may be prepared by modifying functional groups present in the compound of the disclosure in such a way that the modifications are cleaved, either in routine manipulation or in vivo, to the parent compound of the disclosure. Prodrugs include compounds of the disclosure wherein a hydroxy, amino or mercapto group is bonded to any group that, when the prodrug of the compound of the disclosure is administered to a mammalian subject, cleaves to form a free hydroxy, free amino or free mercapto group, respectively. Examples of prodrugs include, but are not limited to, acetate, formate and benzoate derivatives of alcohol or amide derivatives of amine functional groups in the compounds of the disclosure, and the like.

Embodiments of the disclosure are also meant to encompass administration of all pharmaceutically acceptable compounds of the disclosed compounds being isotopically-labelled by having one or more atoms replaced by an atom having a different atomic mass or mass number. Examples of isotopes that can be incorporated into the disclosed compounds include isotopes of hydrogen, carbon, nitrogen, oxygen, phosphorous, fluorine, chlorine, and iodine, such as ²H, ³H, ¹¹C, ¹³C, ¹⁴C, ¹³N, ¹⁵N, ¹⁵O, ¹⁷O, ¹⁸O, ³¹P, ³¹P, ³⁵S, ¹⁸F, ³⁶Cl, ¹²³I, and ¹²⁵I. These radiolabeled compounds could be useful to help determine or measure the effectiveness of the compounds, by characterizing, for example, the site or mode of action, or binding affinity to a pharmacologically important site of action. Certain isotopically-labeled compounds of structure (I), (II) or (III), for example, those incorporating a radioactive isotope, are useful in drug and/or substrate tissue distribution studies. The radioactive isotopes tritium, *i.e.,* ³H, and carbon-14, *i.e.,* ¹⁴C, are particularly useful for this purpose in view of their ease of incorporation and ready means of detection.

Substitution with heavier isotopes such as deuterium, *i.e.,* ²H, may afford certain therapeutic advantages resulting from greater metabolic stability, for example, increased in vivo half-life or reduced dosage requirements, and hence may be preferred in some circumstances.

Substitution with positron emitting isotopes, such as ¹¹C, ¹⁸F, ¹⁵O and ¹³N, can be useful in Positron Emission Topography (PET) studies for examining substrate receptor occupancy. Isotopically-labeled compounds of structure (I), (II) or (III) can generally be prepared by conventional techniques known to those skilled in the art or by processes analogous to those described in the Preparations and Examples as set out below using an appropriate isotopically-labeled reagent in place of the non-labeled reagent.

Embodiments of the disclosure are also meant to encompass the in vivo metabolic products of the disclosed compounds. Such products may result from, for example, the oxidation, reduction, hydrolysis, amidation, esterification, and the like of the administered compound, primarily due to enzymatic processes. Accordingly, embodiments include compounds produced by a process comprising administering a compound of this disclosure to a mammal for a period of time sufficient to yield a metabolic product thereof. Such products are typically identified by administering a radio-labelled compound of the disclosure in a detectable dose to an animal, such as a rat, mouse, guinea pig, monkey, or to a human, allowing sufficient time for metabolism to occur, and isolating its conversion products from the urine, blood or other biological samples.

"Stable compound" and "stable structure" are meant to indicate a compound that is sufficiently robust to survive isolation to a useful degree of purity from a reaction mixture, and formulation into an efficacious therapeutic agent.

"Subject" includes humans, domestic animals, such as laboratory animals (*e.g.,* dogs, monkeys, rats, mice, *etc*.), household pets (*e.g.,* cats, dogs, rabbits, *etc.*), and livestock (*e.g.,* pigs, cattle, sheep, goats, horses, *etc*.), and non-domestic animals (*e.g.,* bears, elephants, porcupines, *etc*.). In embodiments, a subject is a mammal. In embodiments, a subject is a human.

"Mammal" includes humans and both domestic animals such as laboratory animals and household pets (*e.g.,* cats, dogs, swine, cattle, sheep, goats, horses, rabbits), and non-domestic animals such as wildlife and the like.

"Optional" or "optionally" means that the subsequently described event or circumstances may or may not occur, and that the description includes instances where said event or circumstance occurs and instances in which it does not. For example, "optionally substituted aryl" means that the aryl radical may or may not be substituted and that the description includes both substituted aryl radicals and aryl radicals having no substitution.

"Pharmaceutically acceptable carrier, diluent or excipient" includes, without limitation, any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, or emulsifier which has been approved by the United States Food and Drug Administration as being acceptable for use in humans or domestic animals.

"Pharmaceutically acceptable salt" includes both acid and base addition salts. Even if not specifically described in each instance, unless otherwise indicated (*e.g.,* by the context), use of a therapeutic agent described herein (*e.g.,* PIM kinase inhibitor, JAK kinase inhibitor) optionally comprises use of a pharmaceutically acceptable salt of the therapeutic agent instead of, or in addition to, the parent compound.

"Pharmaceutically acceptable acid addition salt" refers to those salts which retain the biological effectiveness and properties of the free bases, which are not biologically or otherwise undesirable, and which are formed with inorganic acids such as, but not limited to, hydrochloric acid, hydrobromic acid, sulfuric acid, nitric acid, phosphoric acid and the like, and organic acids such as, but not limited to, acetic acid, 2,2-dichloroacetic acid, adipic acid, alginic acid, ascorbic acid, aspartic acid, benzenesulfonic acid, benzoic acid, 4-acetamidobenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxo-glutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid, undecylenic acid, and the like.

"Pharmaceutically acceptable base addition salt" refers to those salts which retain the biological effectiveness and properties of the free acids, and which are not biologically or otherwise undesirable. These salts are prepared from addition of an inorganic base or an organic base to the free acid. Salts derived from inorganic bases include, but are not limited to, the sodium, potassium, lithium, ammonium, calcium, magnesium, iron, zinc, copper, manganese, aluminum salts and the like. Preferred inorganic salts are the ammonium, sodium, potassium, calcium, and magnesium salts. Salts derived from organic bases include, but are not limited to, salts of primary, secondary, and tertiary amines, substituted amines including naturally occurring substituted amines, cyclic amines and basic ion exchange resins, such as ammonia, isopropylamine, trimethylamine, diethylamine, triethylamine, tripropylamine, diethanolamine, ethanolamine, deanol, 2 dimethylaminoethanol, 2 diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purines, piperazine, piperidine, N ethylpiperidine, polyamine resins and the like. Particularly preferred organic bases are isopropylamine, diethylamine, ethanolamine, trimethylamine, dicyclohexylamine, choline and caffeine.

A "pharmaceutical composition" refers to a formulation of a compound of the disclosure and a medium generally accepted in the art for the delivery of a biologically active compound to mammals, e.g., humans. Such a medium includes all pharmaceutically acceptable carriers, diluents or excipients for the compound of the disclosure.

"Effective amount" or "therapeutically effective amount" refers to that amount of a compound of the disclosure which, when administered to a subject (*e.g.,* a mammal, preferably a human), is sufficient to effect treatment, as defined below, of a cancer, such as a b-cell malignancy) in the subject, preferably a human. The amount of a compound of the disclosure which constitutes a "therapeutically effective amount" will vary depending on the compound, the condition and its severity, the manner of administration, and the age of the mammal to be treated, but can be determined routinely by one of ordinary skill in the art having regard to his own knowledge and to this disclosure.

"Treating" or "treatment," as used herein, covers the treatment of the disease or condition of interest in a mammal, preferably a human, having the disease or condition of interest, and includes: (i) preventing the disease or condition from occurring in a mammal, in particular, when such mammal is predisposed to the condition but has not yet been diagnosed as having it; (ii) inhibiting the disease or condition, i.e., arresting its development; (iii) relieving the disease or condition, i.e., causing regression of the disease or condition; or (iv) relieving the symptoms resulting from the disease or condition, *i.e.,* relieving pain without addressing the underlying disease or condition.

With respect to myelofibrosis, "treating" or "treatment" includes achieving complete or partial remission, cytogenetic remission or molecular remission of the myelofibrosis, and achieving clinical improvement, an anemia response, a spleen response, a symptoms response or stable disease, as those terms are defined in Tefferi, A., et al., Blood 2013, 122:1395-1398 (*see,* in particular, Table 1), the relevant teachings of which are incorporated herein by reference in their entireties.

As used herein, the terms "disease" and "condition" may be used interchangeably or may be different in that the particular malady or condition may not have a known causative agent (so that etiology has not yet been worked out) and it is therefore not yet recognized as a disease but only as an undesirable condition or syndrome, wherein a more or less specific set of symptoms has been identified by clinicians.

A "cancer," including a "tumor," refers to an uncontrolled growth of cells and/or abnormal increased cell survival and/or inhibition of apoptosis which interferes with the normal functioning of the bodily organs and systems. "Cancer" (e.g., a tumor) includes solid and non-solid cancers. A subject that has a cancer or a tumor has an objectively measurable number of cancer cells present in the subject's body. "Cancers" include benign and malignant cancers (e.g., benign and malignant tumors, respectively), as well as dormant tumors or micrometastases.

"Fibrosis associated with cancer" refers to a cancer (e.g., a tumor) having a fibrotic component. The methods disclosed herein are meant to include subjects having fibrosis associated with cancer and a separate fibrotic disease (e.g., pulmonary fibrosis), so long as the subject also has a fibrosis associated with cancer. Examples of cancers having a fibrotic component include, but are not limited to, pancreatic cancer (e.g., pancreatic ductal adenocarcinoma), liver cancer, kidney cancer, renal cell cancer, lung cancer (e.g., large cell lung cancer, squamous cell carcinoma), carcinoma of an internal organ (e.g., pancreas, lung, kidney, liver), sarcoma (e.g., soft tissue sarcoma), malignant fibrous histiocytoma, fibrosarcoma (e.g., dermatofibrosarcoma protuberans), hepatocellular carcinoma, breast cancer (e.g., inflammatory breast cancer), endometrial cancer, ovarian cancer (e.g., high grade serious ovarian carcinoma) and uterine sarcoma (e.g., uterine leiomyosarcoma). Examples of solid tumors having a fibrotic component include, but are not limited to, kidney, liver, lung, breast, ovarian, endometrial, uterine and pancreas.

"Metastasis" refers to the spread of cancer from its primary site to other places in the body. "Metastases" are cancers which migrate from their original location and seed vital organs, which can eventually lead to the death of the subject through the functional deterioration of the affected organs. Metastasis is a sequential process, where cancer cells can break away from a primary tumor, penetrate into lymphatic and blood vessels, circulate through the bloodstream, and grow in a distant focus (metastasize) in normal tissues elsewhere in the body. At the new site, the cells establish a blood supply and can grow to form a life-threatening mass. Metastasis can be local or distant. Both stimulatory and inhibitory molecular pathways within the tumor cell regulate this behavior, and interactions between the tumor cell and host cells in the new site are also significant.

The compounds of the disclosure, or their pharmaceutically acceptable salts, may contain one or more asymmetric centers and may thus give rise to enantiomers, diastereomers, and other stereoisomeric forms that may be defined, in terms of absolute stereochemistry, as (R)-or (S)-, or as (D)- or (L)- for amino acids. The present disclosure is meant to include all such possible isomers, as well as their racemic and optically pure forms. Optically active (+) and (-), (R)- and (S)-, or (D)- and (L)-isomers may be prepared using chiral synthons or chiral reagents, or resolved using conventional techniques, for example, chromatography and fractional crystallization. Conventional techniques for the preparation/isolation of individual enantiomers include chiral synthesis from a suitable optically pure precursor or resolution of the racemate (or the racemate of a salt or derivative) using, for example, chiral high-pressure liquid chromatography (HPLC). When the compounds described herein contain olefinic double bonds or other centers giving rise to geometric asymmetry, and unless specified otherwise, it is intended that the compounds include both E and Z geometric isomers. Likewise, all tautomeric forms are also intended to be included.

A "stereoisomer" refers to a compound made up of the same atoms bonded by the same bonds but having different three-dimensional structures, which are not interchangeable. The present disclosure contemplates various stereoisomers and mixtures thereof, and includes "enantiomers," which refers to two stereoisomers whose molecules are non-superimposable mirror images of one another.

### I. Methods

Accordingly, in one embodiment a method for treating myeloproliferative neoplasms in a mammal in need thereof is provided, the method comprising administering to the mammal an effective amount of a PIM kinase inhibitor. Another embodiment provides a method for decreasing proliferation of hematopoietic cells in a mammal, the method comprising contacting the cells with a PIM kinase inhibitor (*e.g*., an effective amount of a PIM kinase inhibitor).

Yet another embodiment provides a method for treating a subject having or at risk of developing fibrosis associated with cancer, the method comprising administering to the subject a composition comprising a therapeutically effective amount of Compound 1: 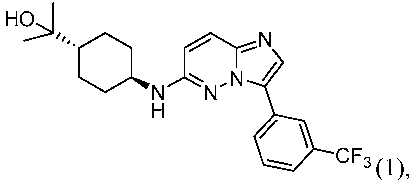 or a pharmaceutically acceptable salt thereof.

In some embodiments, the methods described herein involve identifying a subject being at risk of developing fibrosis associated with cancer. In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with cancer. In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with cancer.

In some embodiments, provided are methods for prophylactically treating a fibrosis associated with cancer, comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer, comprising administering Compound 1, or a pharmaceutically acceptable salt thereof (*e.g.,* an effective amount of Compound 1, or a pharmaceutically acceptable salt thereof), to a subject in need thereof. Some embodiments provide a method for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of cancer tissue and fibrosis associated therewith, the method including contacting cancer tissue and fibrosis associated therewith with Compound 1, or a pharmaceutically acceptable salt thereof, in an amount sufficient to inhibit formation or deposition of fibrosis in the cancer tissue.

In another aspect, a method is provided for treating a subject having or at risk of developing fibrosis associated with cancer, the method comprising administering to the subject in need thereof a therapeutically effective amount of Compound 1, or a pharmaceutically acceptable salt thereof.

Another embodiment provides a method for treating a subject having or at risk of developing fibrosis associated with pancreatic cancer, the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with pancreatic cancer. In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with pancreatic cancer. In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with pancreatic cancer.

In some embodiments, provided are methods for prophylactically treating a fibrosis associated with cancer comprising administering Compound 1, or a pharmaceutically acceptable salt thereof, to a subject in need thereof, wherein the fibrosis associated with cancer includes fibrosis associated with pancreatic cancer. In some embodiments, provided are methods for prophylactically treating fibrosis associated with pancreatic cancer comprising administering Compound 1, or a pharmaceutically acceptable salt thereof, to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is pancreatic cancer. Some embodiments provide a method for treating fibrosis associated with pancreatic cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with pancreatic cancer. In some embodiments, provided are methods for preventing fibrosis associated with pancreatic cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of pancreatic cancer tissue and fibrosis associated therewith, the method including contacting pancreatic cancer tissue and fibrosis associated therewith with Compound 1, or a pharmaceutically acceptable salt thereof, in an amount sufficient to inhibit formation or deposition of fibrosis in the pancreatic cancer tissue.

In another aspect, a method is provided for treating a subject having or at risk of developing fibrosis associated with pancreatic cancer, the method comprising administering to the subject in need thereof a therapeutically effective amount of Compound 1, or a pharmaceutically acceptable salt thereof.

Some embodiments provide a method for treating a subject having or at risk of developing fibrosis associated with pancreatic ductal adenocarcinoma, the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with pancreatic ductal adenocarcinoma. In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with pancreatic ductal adenocarcinoma. In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with pancreatic ductal adenocarcinoma.

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with pancreatic ductal adenocarcinoma. In some embodiments, provided are methods for prophylactically treating fibrosis associated with pancreatic ductal adenocarcinoma comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is pancreatic ductal adenocarcinoma. Some embodiments provide a method for treating fibrosis associated with pancreatic ductal adenocarcinoma comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with pancreatic ductal adenocarcinoma. In some embodiments, provided are methods for preventing fibrosis associated with pancreatic ductal adenocarcinoma comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of pancreatic ductal adenocarcinoma tissue and fibrosis associated therewith, the method including contacting pancreatic ductal adenocarcinoma tissue and/or fibrosis associated therewith with Compound 1, or a pharmaceutically acceptable salt thereof, in an amount sufficient to inhibit formation or deposition of fibrosis in the cancer tissue.

In another aspect, a method is provided for treating a subject having or at risk of developing fibrosis associated with pancreatic ductal adenocarcinoma, the method comprising administering to the subject in need thereof a therapeutically effective amount of Compound 1, or a pharmaceutically acceptable salt thereof.

Another embodiment provides a method for treating a subject having or at risk of developing fibrosis associated with a solid tumor (*e.g.*, kidney, liver, lung, breast, ovarian, endometrial, uterine, and/or pancreatic cancer), the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with a solid tumor (*e.g*., kidney, liver, lung, breast, ovarian, endometrial, uterine, and/or pancreatic cancer). In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with a solid tumor (*e.g*., kidney, liver, lung, breast, ovarian, endometrial, uterine, and/or pancreatic cancer). In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with a solid tumor (*e.g.,* kidney, liver, lung, breast, ovarian, endometrial, uterine, and/or pancreatic cancer).

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with a solid tumor (*e.g.,* kidney, liver, lung, breast, ovarian, endometrial, uterine, and/or pancreatic cancer). In some embodiments, provided are methods for prophylactically treating fibrosis associated with a solid tumor (*e.g*., kidney, liver, lung, breast, ovarian, endometrial, uterine, and/or pancreatic cancer) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is a solid tumor (*e.g.,* kidney, liver, lung, breast, ovarian, endometrial, uterine, and/or pancreatic cancer). Some embodiments provide a method for treating fibrosis associated with a solid tumor (*e.g.*, kidney, liver, lung, breast, ovarian, endometrial, uterine, and/or pancreatic cancer) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with a solid tumor (*e.g.*, kidney, liver, lung, breast, ovarian, endometrial, uterine, and/or pancreatic cancer). In some embodiments, provided are methods for preventing fibrosis associated with a solid tumor (*e.g.*, kidney, liver, lung, breast, ovarian, endometrial, uterine, and/or pancreatic cancer) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of solid tumor tissue and fibrosis associated therewith, the method including contacting solid tumor tissue and/or fibrosis associated therewith with Compound 1, or a pharmaceutically acceptable salt thereof, in an amount sufficient to inhibit formation or deposition of fibrosis in the solid tumor tissue.

In another aspect, a method is provided for treating a subject having or at risk of developing fibrosis associated with a solid tumor (e.g., kidney, liver, lung, breast, ovarian, endometrial, uterine, and/or pancreatic cancer), the method comprising administering to the subject in need thereof a therapeutically effective amount of Compound 1, or a pharmaceutically acceptable salt thereof.

Certain embodiments provide a method for treating a subject having or at risk of developing fibrosis associated with liver cancer, the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with liver cancer. In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with liver cancer. In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with liver cancer.

In some embodiments, provided are methods for prophylactically treating a fibrosis associated with cancer comprising administering Compound 1, or a pharmaceutically acceptable salt thereof, to a subject in need thereof (*e.g.,* an effective amount of Compound 1, or a pharmaceutically acceptable salt thereof), wherein the fibrosis associated with cancer includes fibrosis associated with liver cancer. In some embodiments, provided are methods for prophylactically treating fibrosis associated with liver cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is liver cancer. Some embodiments provide a method for treating fibrosis associated with liver cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with liver cancer. In some embodiments, provided are methods for preventing fibrosis associated with liver cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of liver cancer tissue and fibrosis associated therewith, the method including contacting liver cancer tissue and/or fibrosis associated therewith with Compound 1, or a pharmaceutically acceptable salt thereof, in an amount sufficient to inhibit formation or deposition of fibrosis in the liver cancer tissue.

In another aspect, a method is provided for treating a subject having or at risk of developing fibrosis associated with liver cancer, the method comprising administering to the subject in need thereof a therapeutically effective amount of Compound 1, or a pharmaceutically acceptable salt thereof.

Another embodiment provides a method for treating a subject having or at risk of developing fibrosis associated with kidney cancer, the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with kidney cancer. In some embodiments, the methods described herein further include administering Compound 1, or a pharmaceutically acceptable salt thereof, to a subject identified as being at risk of developing fibrosis associated with kidney cancer. In some embodiments, the methods further include administering Compound 1, or a pharmaceutically acceptable salt thereof, to a subject suspected to have fibrosis associated with kidney cancer.

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with kidney cancer. In some embodiments, provided are methods for prophylactically treating fibrosis associated with kidney cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is kidney cancer. Some embodiments provide a method for treating fibrosis associated with kidney cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with kidney cancer. In some embodiments, provided are methods for preventing fibrosis associated with kidney cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of kidney cancer tissue and fibrosis associated therewith, the method including contacting kidney cancer tissue and/or fibrosis associated therewith with Compound 1, or a pharmaceutically acceptable salt thereof, in an amount sufficient to inhibit formation or deposition of fibrosis in the kidney cancer tissue.

In another aspect, a method is provided for treating a subject having or at risk of developing fibrosis associated with kidney cancer, the method comprising administering to the subject in need thereof a therapeutically effective amount of Compound 1, or a pharmaceutically acceptable salt thereof.

Another embodiment provides a method for treating a subject having or at risk of developing fibrosis associated with renal cell cancer, the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with renal cell cancer. In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with renal cell cancer. In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with renal cell cancer.

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with renal cell cancer. In some embodiments, provided are methods for prophylactically treating fibrosis associated with renal cell cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is renal cell cancer. Some embodiments provide a method for treating fibrosis associated with renal cell cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof *(e.g.,* an effective amount of Compound 1, or a pharmaceutically acceptable salt thereof), to a subject in need thereof, wherein the fibrosis associated with cancer is fibrosis associated with renal cell cancer. In some embodiments, provided are methods for preventing fibrosis associated with renal cell cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of renal cell cancer tissue and fibrosis associated therewith, the method including contacting renal cell cancer tissue and/or fibrosis associated therewith with Compound 1, or a pharmaceutically acceptable salt thereof, in an amount sufficient to inhibit formation or deposition of fibrosis in the renal cell cancer tissue.

In another aspect, a method is provided for treating a subject having or at risk of developing fibrosis associated with renal cell cancer, the method comprising administering to the subject in need thereof a therapeutically effective amount of Compound 1, or a pharmaceutically acceptable salt thereof.

Certain embodiments provide a method for treating a subject having or at risk of developing fibrosis associated with lung cancer, the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with lung cancer. In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with lung cancer. In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with lung cancer.

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with lung cancer. In some embodiments, provided are methods for prophylactically treating fibrosis associated with lung cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is lung cancer. Some embodiments provide a method for treating fibrosis associated with lung cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with lung cancer. In some embodiments, provided are methods for preventing fibrosis associated with lung cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of lung cancer tissue and fibrosis associated therewith, the method including contacting lung cancer tissue and/or fibrosis associated therewith with Compound 1, or a pharmaceutically acceptable salt thereof, in an amount sufficient to inhibit formation or deposition of fibrosis in the lung cancer tissue.

In another aspect, a method is provided for treating a subject having or at risk of developing fibrosis associated with lung cancer, the method comprising administering to the subject in need thereof a therapeutically effective amount of Compound 1, or a pharmaceutically acceptable salt thereof.

Another embodiment provides a method for treating a subject having or at risk of developing fibrosis associated with a combination of pancreatic cancer, liver cancer, lung cancer, and/or renal cell cancers, the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with a combination of pancreatic cancer, liver cancer, lung cancer, and/or renal cell cancers. In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with a combination of pancreatic cancer, liver cancer, lung cancer, and/or renal cell cancers. In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with a combination of pancreatic cancer, liver cancer, lung cancer, and/or renal cell cancers.

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with a combination of pancreatic cancer, liver cancer, lung cancer, and/or renal cell cancers. In some embodiments, provided are methods for prophylactically treating fibrosis associated with a combination of pancreatic cancer, liver cancer, lung cancer, and/or renal cell cancers comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is a combination of pancreatic cancer, liver cancer, lung cancer, and/or renal cell cancers. Some embodiments provide a method for treating fibrosis associated with a combination of pancreatic cancer, liver cancer, lung cancer, and/or renal cell cancers comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof, to a subject in need thereof (*e.g.,* an effective amount of Compound 1, or a pharmaceutically acceptable salt thereof), wherein the fibrosis associated with cancer is fibrosis associated with a combination of pancreatic cancer, liver cancer, lung cancer, and/or renal cell cancers. In some embodiments, provided are methods for preventing fibrosis associated with a combination of pancreatic cancer, liver cancer, lung cancer, and/or renal cell cancers comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of pancreatic cancer, liver cancer, lung cancer, and/or renal cell cancer tissue and fibrosis associated therewith, the method including contacting pancreatic cancer, liver cancer, lung cancer, and/or renal cell cancer tissue and/or fibrosis associated therewith with Compound 1, or a pharmaceutically acceptable salt thereof, in an amount sufficient to inhibit formation or deposition of fibrosis in the pancreatic cancer, liver cancer, lung cancer, and/or renal cell cancer tissue.

In another aspect, a method is provided for treating a subject having or at risk of developing fibrosis associated with a combination of pancreatic cancer, liver cancer, lung cancer, and/or renal cell cancers, the method comprising administering to the subject in need thereof a therapeutically effective amount of Compound 1, or a pharmaceutically acceptable salt thereof.

Another embodiment provides a method for treating a subject having or at risk of developing fibrosis associated with carcinoma of an internal organ (*e.g.,* pancreas, lung, kidney and/or liver), the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with carcinoma of an internal organ (*e.g*., pancreas, lung, kidney and/or liver). In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with carcinoma of an internal organ (*e.g*., pancreas, lung, kidney and/or liver). In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with carcinoma of an internal organ (*e.g*., pancreas, lung, kidney and/or liver).

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with carcinoma of an internal organ (*e.g*., pancreas, lung, kidney and/or liver). In some embodiments, provided are methods for prophylactically treating fibrosis associated with carcinoma of an internal organ (*e.g.*, pancreas, lung, kidney and/or liver) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is carcinoma of an internal organ (*e.g.*, pancreas, lung, kidney and/or liver). Some embodiments provide a method for treating fibrosis associated with carcinoma of an internal organ (*e.g*., pancreas, lung, kidney and/or liver) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with carcinoma of an internal organ (*e.g*., pancreas, lung, kidney and/or liver). In some embodiments, provided are methods for preventing fibrosis associated with carcinoma of an internal organ (*e.g*., pancreas, lung, kidney and/or liver) comprising administering Compound 1, or a pharmaceutically acceptable salt thereof *(e.g.,* an effective amount of Compound 1, or a pharmaceutically acceptable salt thereof), to a subject in need thereof.

In some embodiments, provided are methods for inhibiting formation or deposition of carcinoma (*e.g.,* pancreas, lung, kidney and/or liver) tissue and fibrosis associated therewith, the method including contacting carcinoma (*e.g.,* pancreas, lung, kidney and/or liver) tissue and/or fibrosis associated therewith with Compound 1, or a pharmaceutically acceptable salt thereof, in an amount sufficient to inhibit formation or deposition of fibrosis in the carcinoma (*e.g.,* pancreas, lung, kidney and/or liver) tissue.

In some embodiments, provided are methods for inhibiting formation or deposition of carcinoma tissue fibrosis, the method including contacting carcinoma tissue with Compound 1, or a pharmaceutically acceptable salt thereof, in an amount sufficient to inhibit formation or deposition of fibrosis in the carcinoma tissue. In some embodiments, the carcinoma tissue is carcinoma of an internal organ (*e.g.*, pancreas, lung, kidney and/or liver).

In another aspect, a method is provided for treating a subject having or at risk of developing fibrosis associated with carcinoma of an internal organ (*e.g.,* pancreas, lung, kidney and/or liver), the method comprising administering to the subject in need thereof a therapeutically effective amount of Compound 1, or a pharmaceutically acceptable salt thereof.

Another embodiment provides a method for treating a subject having or at risk of developing fibrosis associated with sarcoma (*e.g.*, soft tissue sarcoma), the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with sarcoma (*e.g*., soft tissue sarcoma). In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with sarcoma (*e.g.,* soft tissue sarcoma). In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with sarcoma (*e.g*., soft tissue sarcoma).

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with sarcoma (*e.g*., soft tissue sarcoma). In some embodiments, provided are methods for prophylactically treating fibrosis associated with sarcoma (*e.g*., soft tissue sarcoma) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is sarcoma (*e.g.,* soft tissue sarcoma). Some embodiments provide a method for treating fibrosis associated with sarcoma (*e.g.,* soft tissue sarcoma) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with sarcoma (*e.g.,* soft tissue sarcoma). In some embodiments, provided are methods for preventing fibrosis associated with sarcoma (*e.g.,* soft tissue sarcoma) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of sarcoma (*e.g.,* soft tissue sarcoma) tissue and fibrosis associated therewith, the method including contacting sarcoma (*e.g.,* soft tissue sarcoma) tissue and/or fibrosis associated therewith with Compound 1, or a pharmaceutically acceptable salt thereof, in an amount sufficient to inhibit formation or deposition of fibrosis in the sarcoma (e.g., soft tissue sarcoma) tissue.

In another aspect, a method is provided for treating a subject having or at risk of developing fibrosis associated with sarcoma (e.g., soft tissue sarcoma), the method comprising administering to the subject in need thereof a therapeutically effective amount of Compound 1, or a pharmaceutically acceptable salt thereof.

Another embodiment provides a method for treating a subject having or at risk of developing fibrosis associated with malignant fibrous histiocytoma, the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with malignant fibrous histiocytoma. In some embodiments, the methods described herein further include administering Compound 1, or a pharmaceutically acceptable salt thereof *(e.g.,* an effective amount of Compound 1, or a pharmaceutically acceptable salt thereof), to a subject identified as being at risk of developing fibrosis associated with malignant fibrous histiocytoma. In some embodiments, the methods further include administering Compound 1, or a pharmaceutically acceptable salt thereof *(e.g.,* an effective amount of Compound 1, or a pharmaceutically acceptable salt thereof), to a subject suspected to have fibrosis associated with malignant fibrous histiocytoma.

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with malignant fibrous histiocytoma. In some embodiments, provided are methods for prophylactically treating fibrosis associated with malignant fibrous histiocytoma comprising administering Compound 1, or a pharmaceutically acceptable salt thereof (*e.g.*, an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is malignant fibrous histiocytoma. Some embodiments provide a method for treating fibrosis associated with malignant fibrous histiocytoma comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with malignant fibrous histiocytoma. In some embodiments, provided are methods for preventing fibrosis associated with malignant fibrous histiocytoma comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of malignant fibrous histiocytoma tissue and fibrosis associated therewith, the method including contacting malignant fibrous histiocytoma tissue and/or fibrosis associated therewith with Compound 1, or a pharmaceutically acceptable salt thereof, in an amount sufficient to inhibit formation or deposition of fibrosis in the malignant fibrous histiocytoma tissue.

In another aspect, a method is provided for treating a subject having or at risk of developing fibrosis associated with malignant fibrous histiocytoma, the method comprising administering to the subject in need thereof a therapeutically effective amount of Compound 1, or a pharmaceutically acceptable salt thereof.

Another embodiment provides a method for treating a subject having or at risk of developing fibrosis associated with fibrosarcoma, the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with fibrosarcoma. In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with fibrosarcoma. In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with fibrosarcoma.

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with fibrosarcoma. In some embodiments, provided are methods for prophylactically treating fibrosis associated with fibrosarcoma comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is fibrosarcoma. Some embodiments provide a method for treating fibrosis associated with fibrosarcoma comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with fibrosarcoma. In some embodiments, provided are methods for preventing fibrosis associated with fibrosarcoma comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of fibrosarcoma tissue and fibrosis associated therewith, the method including contacting fibrosarcoma tissue and/or fibrosis associated therewith with Compound 1, or a pharmaceutically acceptable salt thereof, in an amount sufficient to inhibit formation or deposition of fibrosis in the fibrosarcoma tissue.

In another aspect, a method is provided for treating a subject having or at risk of developing fibrosis associated with fibrosarcoma, the method comprising administering to the subject in need thereof a therapeutically effective amount of Compound 1, or a pharmaceutically acceptable salt thereof.

Another embodiment provides a method for treating a subject having or at risk of developing fibrosis associated with dermatofibrosarcoma protuberans, the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with dermatofibrosarcoma protuberans. In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with dermatofibrosarcoma protuberans. In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with dermatofibrosarcoma protuberans.

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with dermatofibrosarcoma protuberans. In some embodiments, provided are methods for prophylactically treating fibrosis associated with dermatofibrosarcoma protuberans comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is dermatofibrosarcoma protuberans. Some embodiments provide a method for treating fibrosis associated with dermatofibrosarcoma protuberans comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof, to a subject in need thereof (*e.g.,* an effective amount of Compound 1, or a pharmaceutically acceptable salt thereof), wherein the fibrosis associated with cancer is fibrosis associated with dermatofibrosarcoma protuberans. In some embodiments, provided are methods for preventing fibrosis associated with dermatofibrosarcoma protuberans comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of dermatofibrosarcoma protuberans tissue and fibrosis associated therewith, the method including contacting dermatofibrosarcoma protuberans tissue and/or fibrosis associated therewith with Compound 1, or a pharmaceutically acceptable salt thereof, in an amount sufficient to inhibit formation or deposition of fibrosis in the dermatofibrosarcoma protuberans tissue.

In another aspect, a method is provided for treating a subject having or at risk of developing fibrosis associated with dermatofibrosarcoma protuberans, the method comprising administering to the subject in need thereof a therapeutically effective amount of Compound 1, or a pharmaceutically acceptable salt thereof.

Another embodiment provides a method for treating a subject having or at risk of developing fibrosis associated with large cell lung cancer (*e.g*., squamous cell carcinoma), the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with large cell lung cancer (*e.g.,* squamous cell carcinoma). In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with large cell lung cancer (*e.g*., squamous cell carcinoma). In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with large cell lung cancer (*e.g.*, squamous cell carcinoma).

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with large cell lung cancer (*e.g*., squamous cell carcinoma). In some embodiments, provided are methods for prophylactically treating fibrosis associated with large cell lung cancer (*e.g*., squamous cell carcinoma) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is large cell lung cancer (*e.g*., squamous cell carcinoma). Some embodiments provide a method for treating fibrosis associated with large cell lung cancer (*e.g*., squamous cell carcinoma) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with large cell lung cancer (*e.g*., squamous cell carcinoma). In some embodiments, provided are methods for preventing fibrosis associated with large cell lung cancer (*e.g*., squamous cell carcinoma) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of large cell lung cancer (*e.g*., squamous cell carcinoma) tissue and fibrosis associated therewith, the method including contacting large cell lung cancer (*e.g.,* squamous cell carcinoma) tissue and/or fibrosis associated therewith with Compound 1, or a pharmaceutically acceptable salt thereof, in an amount sufficient to inhibit formation or deposition of fibrosis in the large cell lung cancer (*e.g*., squamous cell carcinoma) tissue.

In another aspect, a method is provided for treating a subject having or at risk of developing fibrosis associated with large cell lung cancer (e.g., squamous cell carcinoma), the method comprising administering to the subject in need thereof a therapeutically effective amount of Compound 1, or a pharmaceutically acceptable salt thereof.

Another embodiment provides a method for treating a subject having or at risk of developing fibrosis associated with hepatocellular carcinoma, the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with hepatocellular carcinoma. In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with hepatocellular carcinoma. In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with hepatocellular carcinoma.

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with hepatocellular carcinoma. In some embodiments, provided are methods for prophylactically treating fibrosis associated with hepatocellular carcinoma comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is hepatocellular carcinoma. Some embodiments provide a method for treating fibrosis associated with hepatocellular carcinoma comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with hepatocellular carcinoma. In some embodiments, provided are methods for preventing fibrosis associated with hepatocellular carcinoma comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of hepatocellular carcinoma tissue and fibrosis associated therewith, the method including contacting hepatocellular carcinoma tissue and/or fibrosis associated therewith with Compound 1, or a pharmaceutically acceptable salt thereof, in an amount sufficient to inhibit formation or deposition of fibrosis in the hepatocellular carcinoma tissue.

In another aspect, a method is provided for treating a subject having or at risk of developing fibrosis associated with hepatocellular carcinoma, the method comprising administering to the subject in need thereof a therapeutically effective amount of Compound 1, or a pharmaceutically acceptable salt thereof.

Another embodiment provides a method for treating a subject having or at risk of developing myelofibrosis, the method comprising administering to the subject a composition comprising a therapeutically effective amount of Compound 1, or a pharmaceutically acceptable salt thereof.

In some embodiments, the methods described herein involve identifying a subject being at risk of developing myelofibrosis. In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing myelofibrosis. In some embodiments, the methods further include administering Compound 1, or a pharmaceutically acceptable salt thereof (*e.g.,* an effective amount of Compound 1, or a pharmaceutically acceptable salt thereof), to a subject suspected to have myelofibrosis.

In some embodiments, provided are methods for prophylactically treating a myelofibrosis comprising administering Compound 1, or a pharmaceutically acceptable salt thereof (*e.g.,* an effective amount of Compound 1, or a pharmaceutically acceptable salt thereof), to a subject in need thereof. In some embodiments, provided are methods for treating myelofibrosis comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing myelofibrosis comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of myelofibrosis tissue, the method including contacting myelofibrosis tissue with Compound 1, or a pharmaceutically acceptable salt thereof, in an amount sufficient to inhibit formation or deposition of myelofibrosis tissue.

In another aspect, a method is provided for treating a subject having or at risk of developing myelofibrosis, the method comprising administering to the subject in need thereof a therapeutically effective amount of Compound 1, or a pharmaceutically acceptable salt thereof.

Another embodiment provides a method for treating a subject having or at risk of developing fibrosis associated with breast cancer (*e.g*., inflammatory breast cancer), the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with breast cancer (*e.g.,* inflammatory breast cancer). In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with breast cancer (*e.g*., inflammatory breast cancer). In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with breast cancer (*e.g.,* inflammatory breast cancer).

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with breast cancer (*e.g.,* inflammatory breast cancer). In some embodiments, provided are methods for prophylactically treating fibrosis associated with breast cancer (*e.g.*, inflammatory breast cancer) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is breast cancer (*e.g.,* inflammatory breast cancer). Some embodiments provide a method for treating fibrosis associated with breast cancer (*e.g*., inflammatory breast cancer) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with breast cancer (*e.g.*, inflammatory breast cancer). In some embodiments, provided are methods for preventing fibrosis associated with breast cancer (*e.g*., inflammatory breast cancer) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of breast cancer (*e.g*., inflammatory breast cancer) tissue and fibrosis associated therewith, the method including contacting breast cancer (*e.g*., inflammatory breast cancer) tissue and/or fibrosis associated therewith with Compound 1, or a pharmaceutically acceptable salt thereof, in an amount sufficient to inhibit formation or deposition of fibrosis in the breast cancer (*e.g*., inflammatory breast cancer) tissue.

In another aspect, a method is provided for treating a subject having or at risk of developing fibrosis associated with breast cancer (*e.g*., inflammatory breast cancer), the method comprising administering to the subject in need thereof a therapeutically effective amount of Compound 1, or a pharmaceutically acceptable salt thereof.

Another embodiment provides a method for treating a subject having or at risk of developing fibrosis associated with endometrial cancer, the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with endometrial cancer. In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with endometrial cancer. In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with endometrial cancer.

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with endometrial cancer. In some embodiments, provided are methods for prophylactically treating fibrosis associated with endometrial cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is endometrial cancer. Some embodiments provide a method for treating fibrosis associated with endometrial cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with endometrial cancer. In some embodiments, provided are methods for preventing fibrosis associated with endometrial cancer comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of endometrial cancer tissue and fibrosis associated therewith, the method including contacting endometrial cancer tissue and/or fibrosis associated therewith with Compound 1, or a pharmaceutically acceptable salt thereof, in an amount sufficient to inhibit formation or deposition of fibrosis in the endometrial cancer tissue.

In another aspect, a method is provided for treating a subject having or at risk of developing fibrosis associated with endometrial cancer, the method comprising administering to the subject in need thereof a therapeutically effective amount of Compound 1, or a pharmaceutically acceptable salt thereof.

Another embodiment provides a method for treating a subject having or at risk of developing fibrosis associated with ovarian cancer (*e.g.,* high grade serious ovarian carcinoma), the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with ovarian cancer (*e.g.,* high grade serious ovarian carcinoma). In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with ovarian cancer (*e.g.,* high grade serious ovarian carcinoma). In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with ovarian cancer (*e.g.,* high grade serious ovarian carcinoma).

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with ovarian cancer (*e.g.,* high grade serious ovarian carcinoma). In some embodiments, provided are methods for prophylactically treating fibrosis associated with ovarian cancer (*e.g.,* high grade serious ovarian carcinoma) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is ovarian cancer (*e.g.,* high grade serious ovarian carcinoma). Some embodiments provide a method for treating fibrosis associated with ovarian cancer (*e.g.,* high grade serious ovarian carcinoma) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with ovarian cancer (*e.g.,* high grade serious ovarian carcinoma). In some embodiments, provided are methods for preventing fibrosis associated with ovarian cancer (*e.g.,* high grade serious ovarian carcinoma) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of ovarian cancer (*e.g.,* high grade serious ovarian carcinoma) tissue and fibrosis associated therewith, the method including contacting ovarian cancer (*e.g.,* high grade serious ovarian carcinoma) tissue and/or fibrosis associated therewith with Compound 1, or a pharmaceutically acceptable salt thereof, in an amount sufficient to inhibit formation or deposition of fibrosis in the ovarian cancer (*e.g.,* high grade serious ovarian carcinoma) tissue.

In another aspect, a method is provided for treating a subject having or at risk of developing fibrosis associated with ovarian cancer (*e.g.,* high grade serious ovarian carcinoma), the method comprising administering to the subject in need thereof a therapeutically effective amount of Compound 1, or a pharmaceutically acceptable salt thereof.

Another embodiment provides a method for treating a subject having or at risk of developing fibrosis associated with uterine sarcoma (e.g., uterine leiomyosarcoma), the method comprising administering to the subject a composition comprising a therapeutically effective amount of Compound 1, or a pharmaceutically acceptable salt thereof.

In some embodiments, the methods described herein involve identifying a subject being at risk of developing fibrosis associated with uterine sarcoma (*e.g*., uterine leiomyosarcoma). In some embodiments, the methods described herein further include administering Compound 1, or a pharmaceutically acceptable salt thereof (*e.g.,* an effective amount of Compound 1, or a pharmaceutically acceptable salt thereof), to a subject identified as being at risk of developing fibrosis associated with uterine sarcoma (*e.g.,* uterine leiomyosarcoma). In some embodiments, the methods further include administering Compound 1, or a pharmaceutically acceptable salt thereof (*e.g.,* an effective amount of Compound 1, or a pharmaceutically acceptable salt thereof), to a subject suspected to have fibrosis associated with uterine sarcoma (*e.g*., uterine leiomyosarcoma).

In some embodiments, provided are methods for prophylactically treating a fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer includes fibrosis associated with uterine sarcoma (*e.g*., uterine leiomyosarcoma). In some embodiments, provided are methods for prophylactically treating fibrosis associated with uterine sarcoma (*e.g*., uterine leiomyosarcoma) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for treating fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the cancer is uterine sarcoma (*e.g*., uterine leiomyosarcoma). Some embodiments provide a method for treating fibrosis associated with uterine sarcoma (*e.g*., uterine leiomyosarcoma) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for preventing fibrosis associated with cancer comprising administering Compound 1, or a pharmaceutically acceptable salt thereof (*e.g.,* an effective amount of Compound 1, or a pharmaceutically acceptable salt thereof), to a subject in need thereof, wherein the fibrosis associated with cancer is fibrosis associated with uterine sarcoma (*e.g*., uterine leiomyosarcoma). In some embodiments, provided are methods for preventing fibrosis associated with uterine sarcoma (*e.g*., uterine leiomyosarcoma) comprising administering Compound 1, or a pharmaceutically acceptable salt thereof (*e.g.,* an effective amount of Compound 1, or a pharmaceutically acceptable salt thereof), to a subject in need thereof.

In some embodiments, provided are methods for inhibiting formation or deposition of uterine sarcoma (*e.g*., uterine leiomyosarcoma) tissue and fibrosis associated therewith, the method including contacting uterine sarcoma (*e.g*., uterine leiomyosarcoma) tissue and/or fibrosis associated therewith with Compound 1, or a pharmaceutically acceptable salt thereof, in an amount sufficient to inhibit formation or deposition of fibrosis in the uterine sarcoma (e.g., uterine leiomyosarcoma) tissue.

In another aspect, a method is provided for treating a subject having or at risk of developing fibrosis associated with uterine sarcoma (*e.g*., uterine leiomyosarcoma), the method comprising administering to the subject in need thereof a therapeutically effective amount of Compound 1, or a pharmaceutically acceptable salt thereof.

In some related embodiments, the method further comprises administering to the mammal an effective amount of a JAK inhibitor (*i.e.,* the PIM kinase inhibitor is administered concurrently with or sequentially to the JAK inhibitor). In some embodiments, the JAK inhibitor is a JAK2 inhibitor. In some embodiments, the JAK inhibitor is a JAK1 inhibitor.

In certain embodiments, a method for treating a myeloproliferative neoplasm in a mammal in need thereof, the method comprising administering an effective amount of a PIM kinase inhibitor is provided.

In some specific embodiments, the myeloproliferative neoplasm is polycythemia vera. In some other specific embodiments, the myeloproliferative neoplasm is essential thrombocythemia. In still other embodiments, the myeloproliferative neoplasm is myelofibrosis. The structure of the PIM kinase inhibitor and the JAK inhibitor is not particularly limited provided the inhibitor has satisfactory activity against the desired target (i.e., PIM and JAK, respectively). Exemplary PIM kinase inhibitors which are included within the scope of embodiments of the present disclosure include the generic and specific compounds disclosed in PCT Pub. No. WO 2016/161248; WO 2015/019320; WO 2014/033530WO 2014/033631; WO 2014/0200216; WO 2013/175388; WO 2013/013188; WO 2013/020371; WO 2012/154274; WO 2012/129338; WO 2012/080990; WO 2012/120415; WO 2012/004217; WO 2011/057784; WO 2011/079274; WO 2010/0148351; WO 2010/135581; WO 2010/026121; WO 2010/026122; WO 2010/026124; WO 2010/022076; WO 2010/0000978; WO 2010/022081; WO 2009/064486; WO 2009/109576; WO 2008/082839; WO 2008/106692; WO 2008/058126; WO 2007/041712, U.S. Patent No. 7,750,007; 8,168,794 and U.S. Pub. No. 2015/0057265; 2014/0200227; 2014/0329807, US 2008/0261988, the full disclosures of which are hereby incorporated by reference. In some embodiments, the PIM kinase inhibitor is PIM447 or INCB053914. Other PIM kinase inhibitors are known in the art, and such inhibitors are also included in certain embodiments of the disclosure.

A more specific embodiment provides a method for treating a myeloproliferative neoplasm in a mammal in need thereof, the method comprising administering to the mammal an effective amount of a PIM kinase inhibitor, wherein the PIM kinase inhibitor is a compound having one of the following structures (I), (II) or (III): 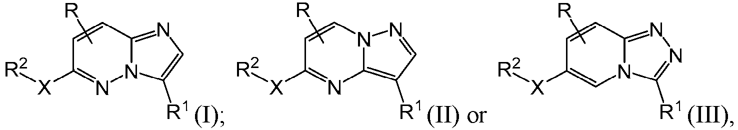 or a stereoisomer or pharmaceutically acceptable salt thereof, wherein:
X is a direct bond, N(R^{a}), S, O, SO or SO₂, wherein R^{a} is H or alkyl;
R is H, amino, cyano, hydroxyl, halo, alkyl, alkylaminyl, haloalkyl, alkoxy or haloalkoxy;
R¹ is phenyl, optionally substituted with 1, 2 or 3 R^{1'}, wherein R^{1'} is, at each occurrence, independently amino, cyano, alkyl, alkylaminyl, alkoxy, halo, haloalkyl, haloalkoxy, hydroxyl, nitro, alkylcarbonyl or alkylsulfonamidyl; and
R² has the following structure: 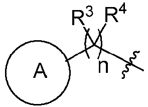 wherein:
   A is an optionally substituted 3-8 membered carbocyclic or heterocyclic ring;
   n is 0, 1, 2, 3 or 4; and
   R³ and R⁴ are, at each occurrence, independently H or alkyl.

In some embodiments, the PIM kinase inhibitor has structure (I). In some embodiments, the PIM kinase inhibitor has structure (II). In some embodiments, the PIM kinase inhibitor has structure (III).

In some embodiments, A is an optionally substituted carbocyclic ring. In certain specific embodiments, A is optionally substituted cyclohexyl. In some more specific embodiments, A is substituted with hydroxyalkyl. In related embodiments, A is cyclohexyl substituted with hydroxyalkyl.

In certain embodiments X is N(R^{a}). In some more specific embodiments, X is NH.

In some embodiments, R¹ is phenyl. In some embodiments, R¹ is phenyl substituted with one R^{1'}. In some embodiments, at least one occurrence of R^{1'} is H. In some embodiments, at least one occurrence of R^{1'} is trifluoromethyl. In some embodiments, R^{1'} is trifluoromethyl.

In some specific embodiments, the PIM kinase inhibitor has one of the following structures:

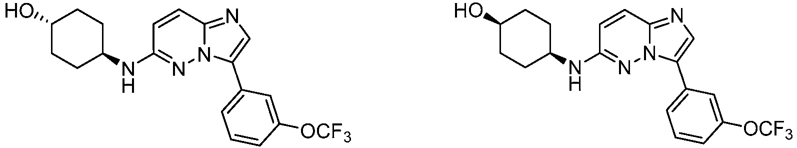

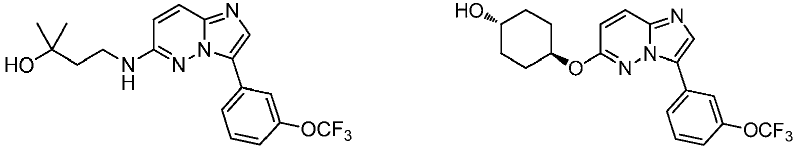

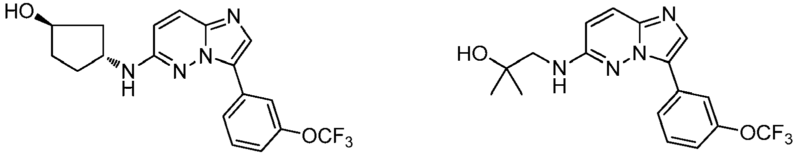

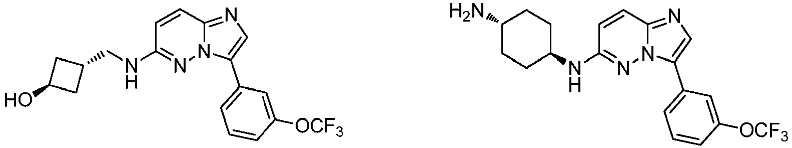

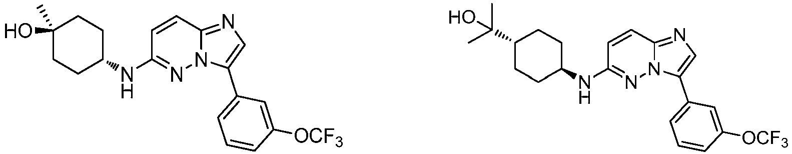

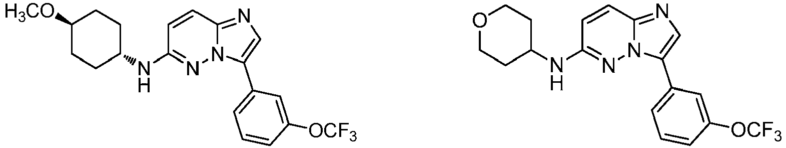

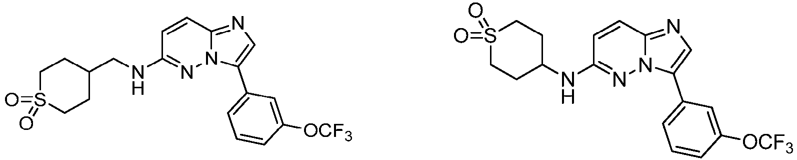

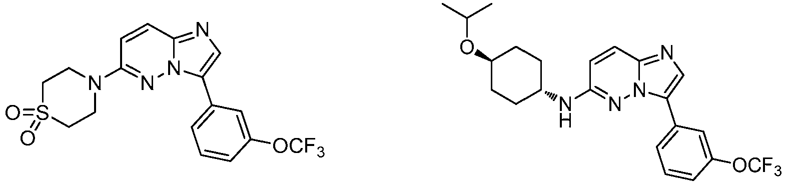

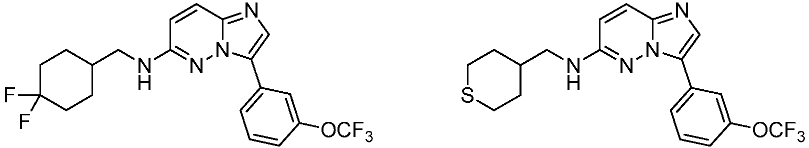

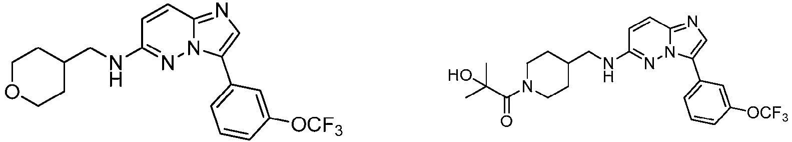

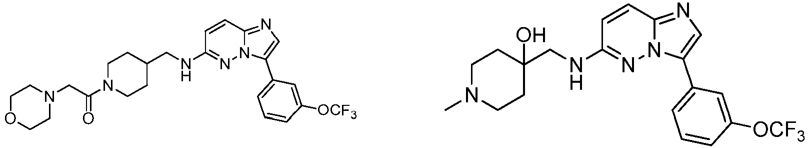

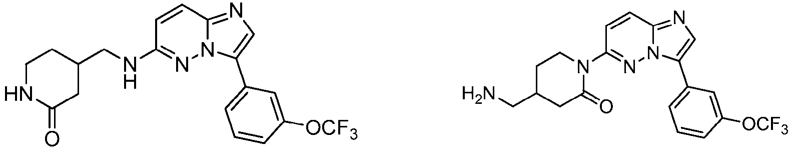

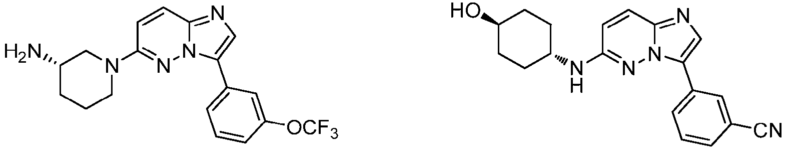

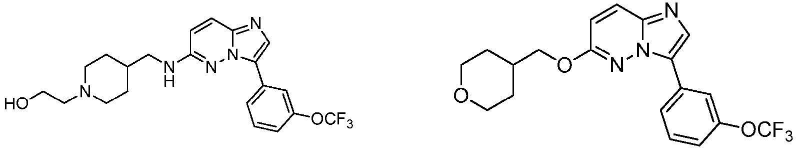

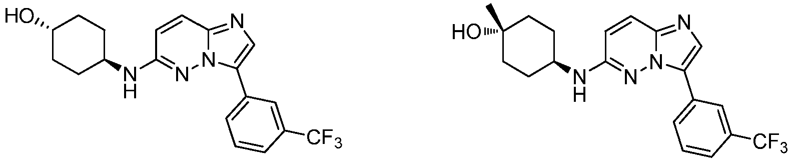

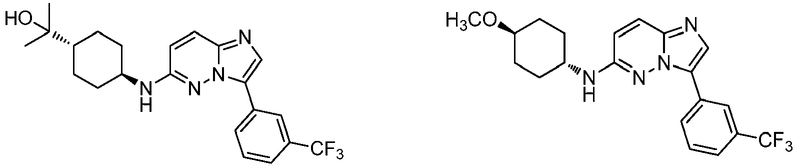

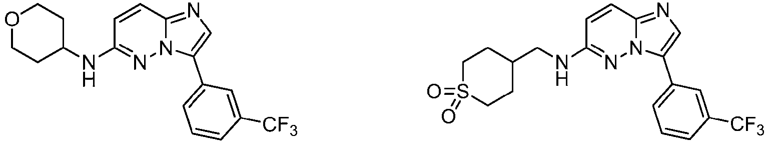

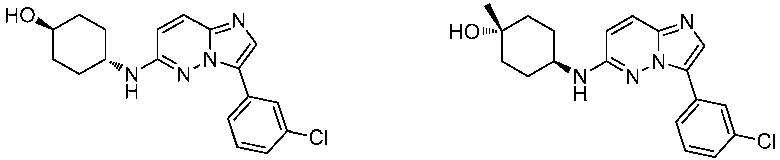

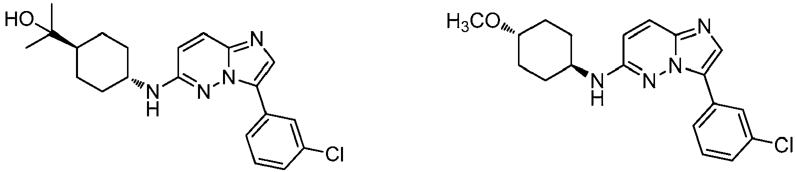

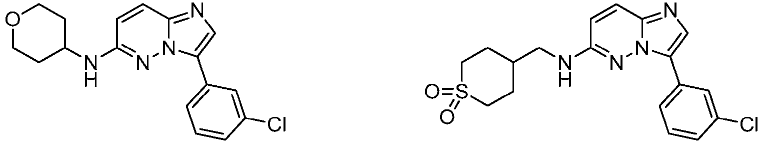

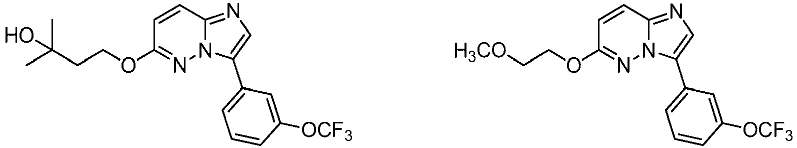

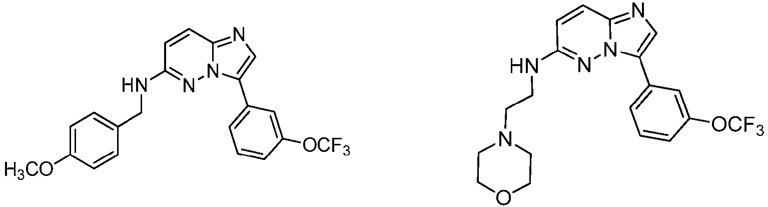

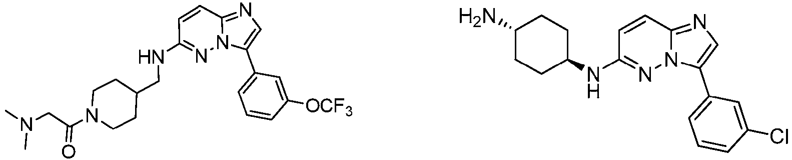

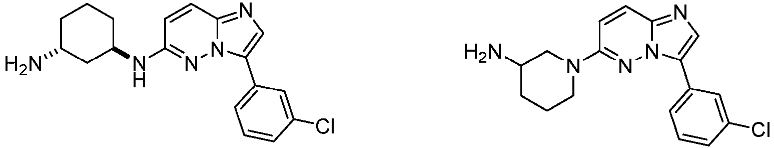

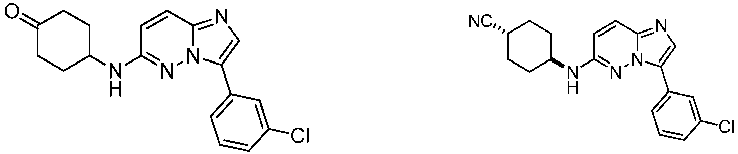

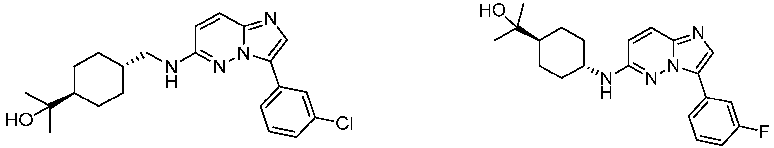

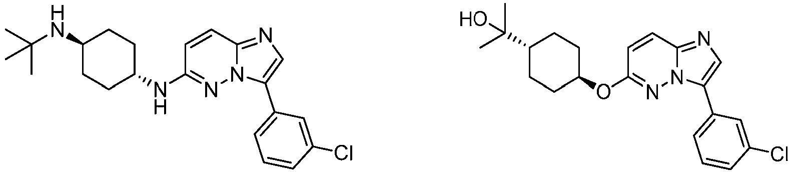

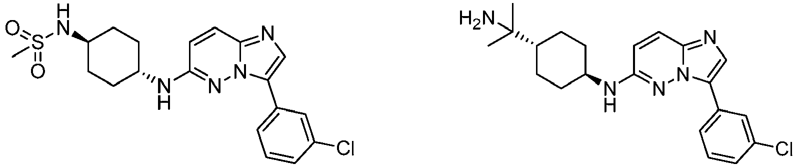

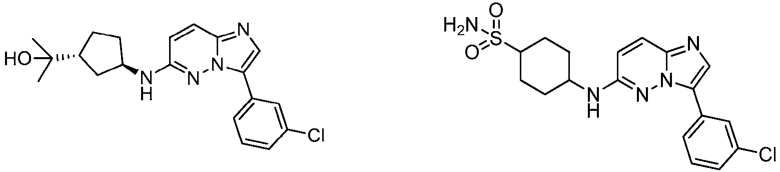

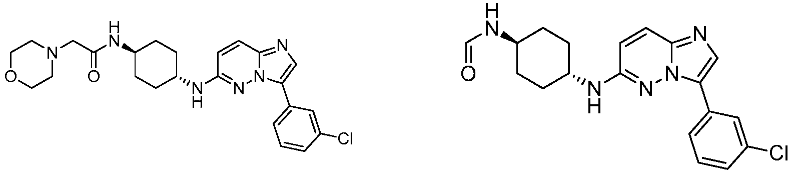

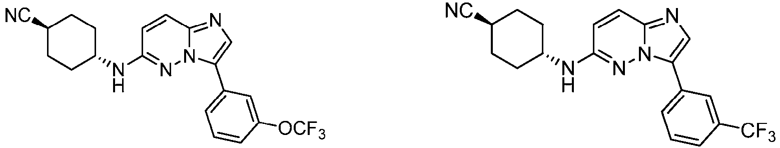

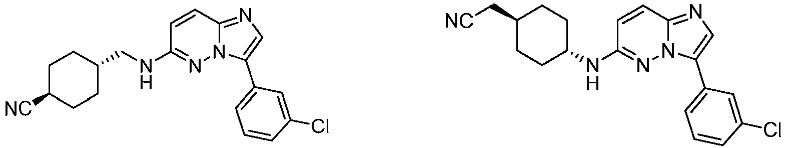

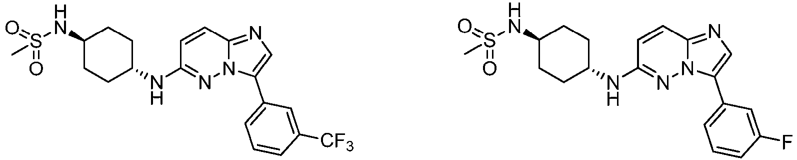

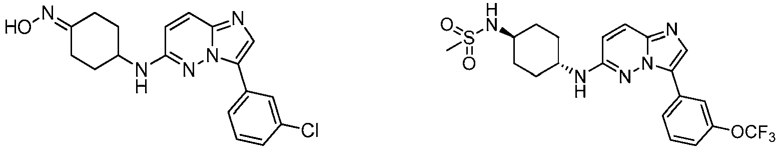

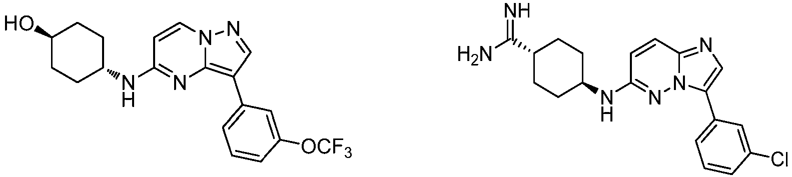

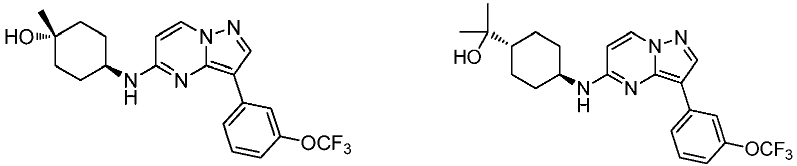

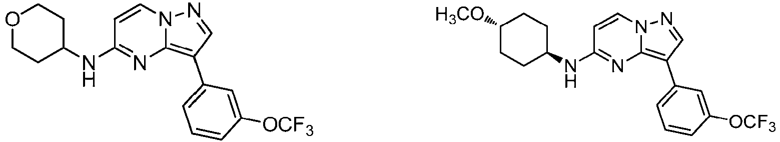

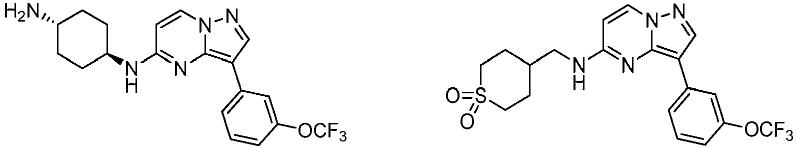

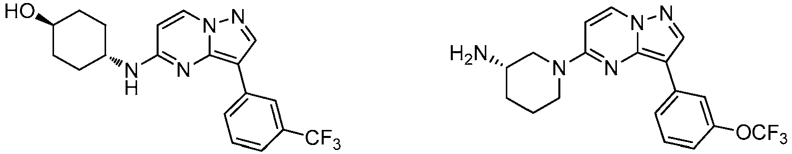

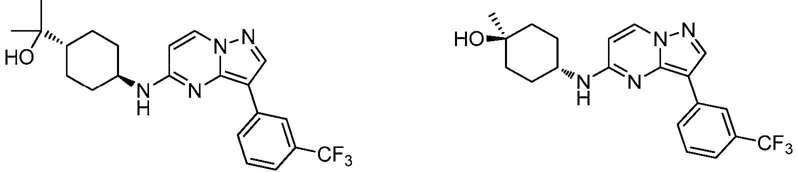

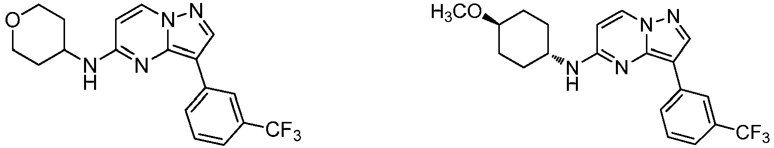

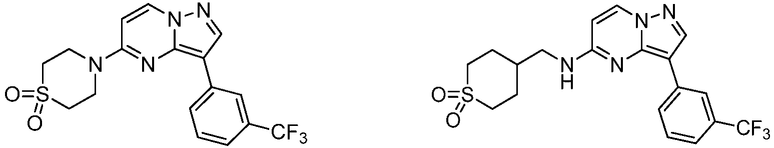

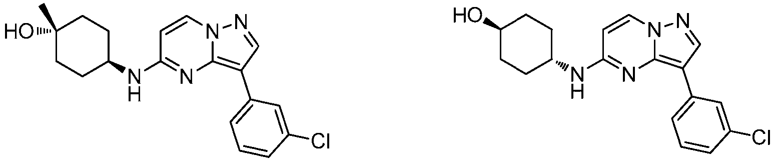

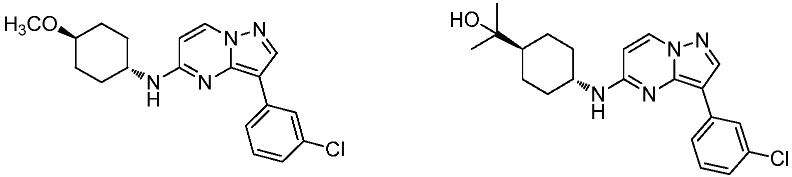

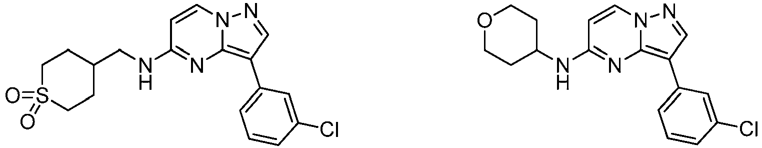

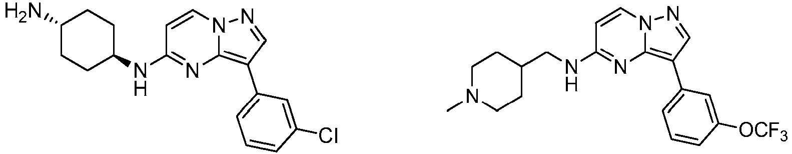

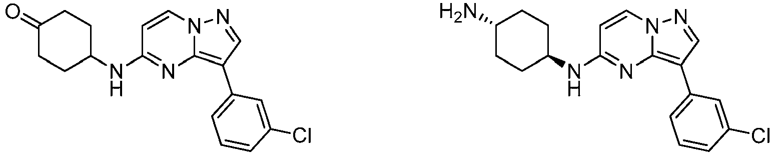

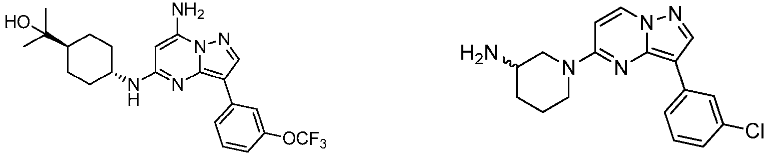

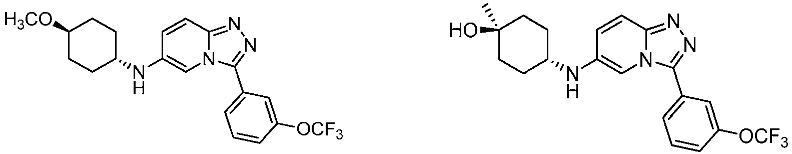

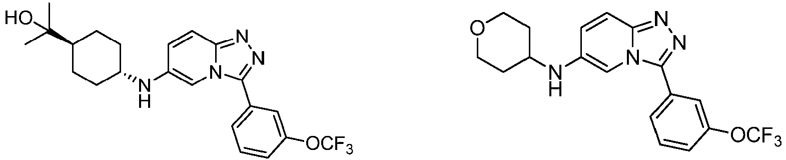

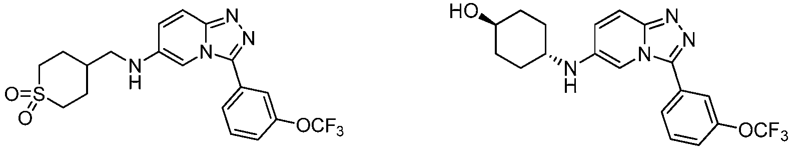

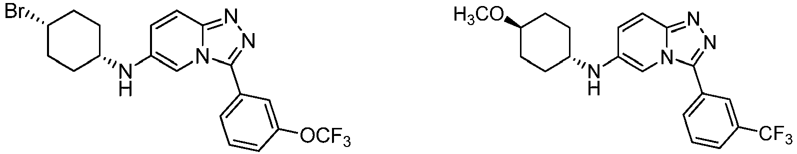

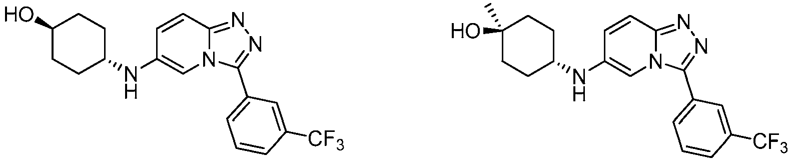

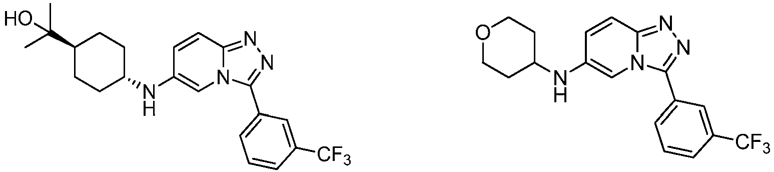

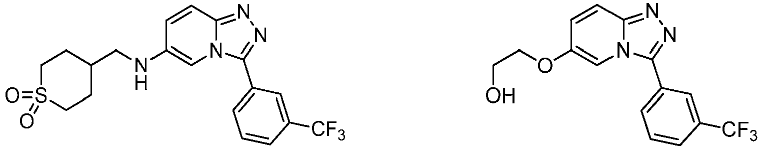

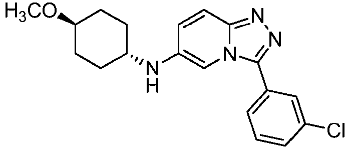

In some specific embodiments, the PIM kinase inhibitor has the following structure (*i.e.,* Compound 1 or 2-((1R,4R)-4-((3-(3-(trifluoromethyl)phenyl)imidazo[1,2-b]pyridazin-6-yl)amino)cyclohexyl)propan-2-ol):

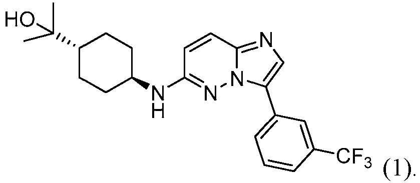

The structure of the JAK inhibitor for use in the present methods is also not particularly limited provided it has sufficient activity against JAK when used in combination with the PIM inhibitor. In some embodiments, the JAK inhibitor has sufficient activity against JAK2, when used in combination with the PIM inhibitor. In some embodiments, the JAK inhibitor has sufficient activity against JAK1, when used in combination with the PIM inhibitor. Exemplary JAK inhibitors, all of which are included within the scope of certain embodiments of the disclosure, are described in PCT Pub. Nos: WO 2015/157257; WO 2014/151871; WO 2014/026595; WO 2014/025128; WO 2014/025486; WO 2014/130411; WO 2014/124230; WO 2011/101161; WO 2011/076519; WO 2010/071885; WO 2010/017122; WO 2009/080638; WO 2009/143389; WO 2009/158571; WO 2009/017954; WO 2009/085913; WO 2009/155565; WO 2008/106635; WO 2008/128072; WO 2008/092199; WO 2005/026026130; WO 2004/046118WO 2004/074244; WO 2001/060816; 97/019065 and in U.S. Pub. Nos: 2015/0306112; 2013/0018034; 2012/0053208; 2008/0260754; and 2008/0214558, the full disclosures of which are hereby incorporated by reference.

In some other embodiments, the JAK inhibitor is ruxolitinib, tofacitinib, oclacitinib, baricitinib, filgotinib, gandotinib, lestaurtinib, momelotinib, pacritinib, PF-04965842, updacitinib, perficitinib, fedratinib, cucurbitacin I, CHZ868, decemotinib, CEP-33779, R348, fibotinib, ABT-494 which compounds are known in the art. In some embodiments, the JAK inhibitor is BMS-911543, ASN002, itacitinib, NS-018, AZD1480, gandotinib, and combinations thereof.

In some embodiments, the JAK inhibitor is a JAK1 inhibitor, a JAK2 inhibitor, or both. For example, in some embodiments, the JAK inhibitor is selected from the group consisting of ruxolitinib, gandotinib, lestaurtinib, momelotinib, pacritinib, and fedratinib. In more specific embodiments, the JAK inhibitor is ruxolitinib, or a pharmaceutically acceptable salt thereof (*e.g.,* ruxolitinib phosphate). In yet more specific embodiments, the JAK inhibitor is ruxolitinib.

Additional therapeutic agents may be used in combination with a PIM kinase inhibitor (*e.g.,* Compound 1) for treatment of a myeloproliferative neoplasm or fibrosis associated with cancer *(e.g.,* a solid tumor) according to embodiments of the disclosure. Combinations of additional therapeutic agents can be administered simultaneously (*e.g.,* in the same or different formulation) or sequentially with the PIM kinase inhibitor. For example, a PIM kinase inhibitor (*e.g.,* Compound 1, or a pharmaceutically acceptable salt thereof) can be administered before a JAK inhibitor (*e.g.,* ruxolitinib, or a pharmaceutically acceptable salt thereof). Alternatively, a PIM kinase inhibitor (*e.g.,* Compound 1, or a pharmaceutically acceptable salt thereof) can be administered after a JAK inhibitor (*e.g.,* ruxolitinib, or a pharmaceutically acceptable salt thereof). In some of these embodiments, the JAK inhibitor can be optionally administered in combination with the PIM kinase inhibitor and the additional therapeutic agent(s). That is, in some embodiments, the method comprises administering the PIM kinase inhibitor and an additional therapeutic agent.

In some embodiments, additional therapeutic agents include hydroxyurea, interferon alpha, cladribine, thalidomide (including derivatives thereof, *e.g.,* pomalidomide, lenolidamide), corticosteroids (*e.g.,* prednisone), everolimus, androgens (*e.g.,* testosterone) and combinations thereof. In some embodiments, the additional therapeutic agent is an additional PIM kinase inhibitor. In some embodiments, the additional PIM kinase inhibitor is PIM447 or INCB053914.

In some embodiments, the method further comprises administering an immune checkpoint inhibitor. In some embodiments, the immune checkpoint molecule is CTLA-4, PD-1 or PD-L1. In some embodiments, the method further comprises administering a CTLA-4 inhibitor. In certain embodiments, the CTLA-4 inhibitor is ipilimumab. In other embodiments, the CTLA-4 inhibitor is tremelimumab.

In some embodiments, the method further comprises administering a PD-1 inhibitor. Exemplary PD-1 inhibitors include, but are not limited to, Pembrolizumab, Nivolumab, CBT-501 (CBT Pharmaceuticals), CBT-502 (CBT Pharmaceuticals), JS001 (Junshi Biosciences), IBI308 (Innovent Biologics), SHR-1210 (Hengrui Medicine), BGB-A317 (Beigene), BAT-I306 (Bio-Thera Solutions), GLS-010 (Gloria Pharmaceuticals; WuXi Biologics), AK103, AK104, AK105 (Akesio Biopharma; Hangzhou Hansi Biologics; Hanzhong Biologics), LZM009 (Livzon), HLX-10 (Henlius Biotech), CS1003 (CStone Pharmaceuticals), or combinations thereof. In specific embodiments, the PD-1 inhibitor is Pembrolizumab, Nivolumab, or a combination thereof. In particular embodiments, the PD-1 inhibitor is Pembrolizumab. In particular embodiments, the PD-1 inhibitor is Nivolumab.

In some embodiments, the PD-1 inhibitor is a monoclonal antibody (*e.g.,* made by Genor Biopharma and in Phase I of clinical trials as of this filing; as made by Shenzhou Gongcheng and applying for clinical trials as of this filing; as made by Lunan Hope Pharmaceuticals and applying for clinical trials as of this filing).

In some embodiments, the method further comprises administering a PD-L1 inhibitor. Exemplary PD-L1 inhibitors include, but are not limited to, Atezolizumab, Avelumab, Durvalumab, or a combination thereof. In particular embodiments, the PD-L1 inhibitor is Atezolizumab. In particular embodiments, the PD-L1 inhibitor is Avelumab. In particular embodiments, the PD-L1 inhibitor is Durvalumab. In certain embodiments, the PD-L1 inhibitor is KN035 (Alphamab; 3DMed), CS1001 (CStone Pharmaceuticals), SHR-1316 (Hengrui Medicine), TQB2450 (Chiatai Tianqing), STI-A1014 (Zhaoke Pharm; Lee's Pharm), BGB-A333 (Beigene), MSB2311 (Mabspace Biosciences), HLX-20 (Henlius Biotech) or combinations thereof. In some embodiments, the PD-L1 inhibitor is a monoclonal antibody (*e.g.,* as made by Hisun Pharm and applying for clinical trials as of this filing).

In some embodiments, the method further comprises administering a FLT3 inhibitor, a caspase 3 activator, a BET inhibitor, an LSD1 inhibitor, a PI3K inhibitor, a PLK inhibitor, a cyclic AMP phosphodiesterase, a histone deacetylase inhibitor, an mTOR inhibitor, an iron chelator, a SYK inhibitor, an SMO antagonist or inhibitor, a hedgehog signaling pathway inhibitor, a BCR-ABL/Kit inhibitor, a BCR-ABL inhibitor, a DNA methylation inhibitor, an SMAC mimetic, an ACVR2a fusion protein, a thromopoeitin receptor agonist, a PI3K delta inhibitor, a tyrosine kinase inhibitor, a recombinant amyloid P/pentraxin 2 protein, a CDK4/6 inhibitor, a telomerase inhibitor, a TGF-β superfamily inhibitor, an LOXL2 inhibitor (e.g., an antibody), a BCL-2 inhibitor, a WNT signal inhibitor, a PD-L1 antibody, a VEGF1/2 inhibitor, a tubulin polymerization inhibitor, an aurora kinase inhibitor, a PNP inhibitor, an AKT inhibitor or combinations thereof. In some embodiments, the method further comprises administering a hypoxia activated prodrug of bromo-isophosphoramide mustard (Br-IPM). In more specific embodiments, the method further comprises administering alvocidib, plitidepsin, INCB054329, INCB057643, INCB053914, INCB059872, rigosertib, anagrelide, givinostat, ridaforolimus, deferasirox, ASN002, LDE225/sonidegib, gleevec, dasatinib, RAD001, azacytidine, pracinostat, CPI-0610, LCL-161, sotatercept, eltrombopag, INCB050465, vismodegib, Lestaurtinib (and other staurosporine analogs), PRM-151, PIM447, ribociclib, imetelstat, luspatercept, saridegib, simtuzumab, obatoclax, navitoclax, Buparlisib, idelalisib, Panobinostat, IMG-7289, Luitpold Azacitidine, CWP232291, Durvalumab, Vatalanib, MKC-1, TAK-901, evofosfamide, TXA127, glasdegib, AC220, Forodesine (and related purine analogs), triciribine or combinations thereof.

In addition, the methods described herein can be performed in conjunction with other medical procedures. Accordingly, in some embodiments, the method further comprises performing a transfusion, administering radiation therapy, performing a splenectomy, or performing a stem cell transplant. In some specific embodiments, the method further comprises administering an angiotensin mimetic (*e.g.,* TXA127).

Various different myeloproliferative neoplasms (MPN) can be treated by the methods disclosed herein. In some embodiments the MPN is polycythemia vera. In some embodiments, the MPN is essential thrombocythemia. In other embodiments, the MPN is myelofibrosis. In some embodiments, the MPN is chronic myelogenous leukemia.

Relatedly, various different blood cancers can be treated by the methods disclosed herein. In some embodiments, a method for treating blood cancer in a mammal in need thereof is provided, the method comprising administering to the mammal an effective amount of a PIM kinase inhibitor and optionally a JAK inhibitor. In some embodiments, a method for treating hematological malignancy (chronic and acute) in a mammal in need thereof is provided, the method comprising administering to the mammal an effective amount of a PIM kinase inhibitor and optionally a JAK inhibitor. In some embodiments, a method for treating or preventing myelodysplastic syndrome or acute myeloid leukemia in a mammal in need thereof is provided, the method comprising administering to the mammal an effective amount of a PIM kinase inhibitor and optionally a JAK inhibitor.

Some embodiments provide a method for decreasing proliferation of hematopoietic cells in a mammal, the method comprising contacting the cells with a PIM kinase inhibitor. In some more specific embodiments, the method further comprises administering to the mammal an effective amount of a JAK inhibitor. In some embodiments, the JAK inhibitor is a JAK inhibitor according to the embodiments described herein. In some embodiments, the JAK inhibitor is a JAK2 inhibitor. In some embodiments, the JAK inhibitor is a JAK1 inhibitor.

In some different embodiments, the disclosure is directed to a method for decreasing proliferation of cells expressing JAK2 V617F in a mammalian cell, the method comprising contacting the cell with a PIM kinase inhibitor and optionally a JAK inhibitor. In some different embodiments, the invention is directed to a method for decreasing proliferation of cells expressing MPL W515L in a mammalian cell, the method comprising contacting the cell with a PIM kinase inhibitor and optionally a JAK inhibitor. In some different embodiments, the invention is directed to a method for decreasing proliferation of cells expressing a calreticulin (CALR) mutation in a mammalian cell, the method comprising contacting the cell with a PIM kinase inhibitor and optionally a JAK inhibitor. In some different embodiments, the disclosure is directed to a method for decreasing proliferation or overproduction of red blood cells, white blood cells, or platelets in a mammalian cell, the method comprising contacting the cell with a PIM kinase inhibitor and optionally a JAK inhibitor. In some embodiments, the mammalian cell is a bone marrow cell.

Myeloproliferative neoplasms refer to a group of disorders in which bone marrow stem cells grow and reproduce abnormally. MPN abnormal stem cells produce excess numbers of one or more types of blood cells (*e.g.,* red blood cells, white blood cells, and/or platelets). As disclosed herein, myeloproliferative neoplasms include, but are not limited to, polycythemia vera (PV), primary or essential thrombocythemia (ET), primary or idiopathic myelofibrosis (MF), secondary myelofibrosis (*e.g.,* myelofibrosis secondary to polycythemia vera or essential thrombocythemia), chronic myelogenous (myelocytic) leukemia (CML), chronic myelomonocytic leukemia (CMML), chronic neutrophilic leukemia (CNL), juvenile myelomonocytic leukemia (JML), systemic mastocytosis, and chronic eosinophilic leukemia (CEL)/hyper eosinophilic syndrome (HES).

In some specific embodiments, the myeloproliferative neoplasm of the mammal treated for a myeloproliferative neoplasm according to the embodiments described herein comprises a JAK2 mutation, a thrombopoietin receptor (MPL) mutation, or a calreticulin (CALR) mutation. In some embodiments, a JAK2 mutation comprises a JAK2 V617 mutation. JAK2 V617F refers to a mutated JAK2 possessing a V→F amino acid substitution at position 617 with respect to the human, wildtype JAK2 (UniProt. 060674). In some embodiments, a MPL mutation comprises a MPL W515L mutation. MPL W515L refers to a mutated thrombopoietin receptor (MPL) possessing a W→L substitution at position 515 with respect to the human, wildtype MPL (UniProt. P40238). In some embodiments, the mutation in CALR comprises a CALR exon 9 indel.

International Prognostic Scoring System (IPSS) score is the main way that myelofibrosis patients are stratified. Risk factors using IPSS include age, constitutional symptoms (*e.g.,* weight loss, fever, or excessive sweating), white blood cell counts, hemoglobin, peripheral blasts, complex or abnormal karyotype, transfusion dependency, and platelet counts. Patients having low-risk myelofibrosis have an IPSS score of 0. An IPSS score of 0 is typically associated with a median survival of about 180 months. In some embodiments, the myelofibrosis is low-risk myelofibrosis.

Patients having intermediate-risk myelofibrosis have an IPSS score of 1, 2 or 3. An IPSS score of 1 is also referred to as intermediate-1 risk, and is typically associated with a median survival of about 80 months. An IPSS score of 2 or 3 is also referred to as intermediate-2 risk, and is typically associated with a median survival of about 35 months. In some embodiments, the myelofibrosis is intermediate-risk myelofibrosis (*e.g.,* intermediate-1 risk myelofibrosis, intermediate-2 risk myelofibrosis).

Patients having high-risk myelofibrosis have an IPSS score of 4 or more. An IPSS score of 4 or more is typically associated with a median survival of about 16 months. In some embodiments, the myelofibrosis is high-risk myelofibrosis.

In some embodiments, the MPN is a ruxolitinib-resistant MPN (*e.g.,* ruxolitinib-resistant myelofibrosis). In some embodiments, the MPN (*e.g.,* myelofibrosis) has been previously treated with ruxolitinib, *e.g.,* in the absence of a PIM kinase inhibitor (*e.g.,* Compound 1, or a pharmaceutically acceptable salt thereof).

A wide variety of cancers, including solid tumors and leukemias (*e.g.,* acute myeloid leukemia, chronic lymphocytic leukemia) are also amenable to the treatment methods disclosed herein. In some specific embodiments, a method for treating a solid tumor comprising administration of a therapeutically effective amount of a PIM kinase inhibitor, and optionally a JAK inhibitor, is provided. Examples of solid tumors include, for example, prostate, breast, colon, and pancreatic cancers.

In some embodiments, treating the MPN or cancer described herein results in complete remission in the mammal. When used to refer to a subject having an MPN, such as myelofibrosis, "complete remission" means a patient meets the following criteria for ≥12 weeks:
(i) bone marrow shows age-adjusted normocellularity, <5% blasts and ≤grade 1 myelofibrosis according to the European classification; and
(ii) hemoglobin ≥100 g/L and <UNL, and neutrophil count ≥ 1 × 10⁹/L and <UNL in peripheral blood; and
(iii) platelet count ≥100 × 10⁹/L and <UNL, and <2% immature myeloid cells, except that in splenectomized patients, <5% immature myeloid cells is allowed; and
(iv) resolution of disease symptoms, non-palpable spleen and liver, and no evidence of EMH.

In some embodiments, treating the MPN or cancer described herein results in the mammal being measurable residual disease (MRD)-negative.

In the context of myeloproliferative neoplasms, such as MF, measurable residual disease, minimal residual disease and MRD refer to the presence of cells possessing acquired mutations within the JAK2, CALR and MPL genes of a subject having a myeloproliferative neoplasm, such as MF. Common mutations in JAK2 include the V617F mutation and mutations (e.g., substitutions, deletions, insertions, duplications) of exon 12. Common mutations in CALR include exon 9 mutations. Common mutations in MPL include exon 10 mutations (e.g., W515L and W515K). MRD is used diagnostically in the context of myeloproliferative neoplasms, but can also be used quantitatively to indicate the depth of response to a therapeutic intervention. MRD testing for myeloproliferative neoplasms, such as MF, is typically conducted using allele-specific quantitative PCR (qPCR), digital PCR or next-generation sequencing. The foregoing methods are reviewed in Haslam, K. and Langabeer, S.E., "Monitoring Residual Disease in the Myeloproliferative Neoplasms: Current Applications and Emerging Approaches," Biomed. Res. Intl. 2016:7241591, the relevant teachings of which are incorporated herein by reference in their entireties.

When a subject having a myeloproliferative neoplasm, such as MF, is described herein as being "measurable residual disease negative," "minimal residual disease negative," "MRD-negative" or "MRD⁻," the subject lacks, or lacks to a measurable extent, cells having an acquired mutation associated with the myeloproliferative neoplasm in at least one of JAK2, CALR or MPL (*e.g.,* the JAK2 V617F mutation, mutations of JAK2 exon 12, CALR exon 9 mutations, MPL W515K/L mutations). For example, in some embodiments, an MRD-negative subject lacks, or lacks to a measurable extent, cells having the JAK2 V617F mutation. In some embodiments, an MRD-negative subject lacks, or lacks to a measurable extent, cells having a CALR exon 9 mutation. In some embodiments, an MRD-negative subject lacks, or lacks to a measurable extent, cells having an MPL exon 10 mutation. Acquired mutations associated with myeloproliferative neoplasms are known in the art, and described in Haslam, K. and Langabeer, S.E., "Monitoring Residual Disease in the Myeloproliferative Neoplasms: Current Applications and Emerging Approaches," Biomed. Res. Intl. 2016:7241591, the relevant teachings of which are incorporated herein by reference in their entireties.

In hematologic cancers, such as AML, measurable residual disease, minimal residual disease and MRD refer to the post-therapy persistence of leukemic cells at levels below morphologic detection. Although not wishing to be bound by any particular theory, MRD is thought to be a strong prognostic indicator of increased risk of relapse or shorter survival in patients with hematologic cancers, such as AML. MRD testing for AML is typically conducted using one of three techniques: immunophenotypic detection by multiparameter flow cytometry (MFC), real-time quantitative PCR (RT-qPCR) and next-generation sequencing technology. MFC uses panels of fluorochrome-labeled monoclonal antibodies to identify aberrantly expressed antigens of leukemic cells. RT-qPCR can be used to amplify leukemia-associated genetic abnormalities. Next-generation sequencing technology can be used to evaluate a few genes or an entire genome. Together, RT-qPCR and next-generation sequencing technology represent molecular approaches to MRD testing. Each of the foregoing methods of detecting MRD status in a subject is described in Ravandi, F., et al., Blood Advances 12 June 2018, vol. 2, no. 11, and Schuurhuis, G. J., et al., Blood 2018 March 22, 131(12): 1275-1291, the relevant contents of which are incorporated herein by reference in their entireties.

To guide the development of a standardized approach to MRD testing, the European LeukemiaNet (ELN) has issued consensus recommendations for the measurement of MRD in AML. According to the ELN, a percentage of cancer (*e.g.,* AML) cells to leukocytes of 0.1% or greater in a subject's bone marrow, measured by MFC according to the ELN's recommendations for MRD testing by MFC, indicates the subject is MRD positive (MRD+) by MFC according to the ELN's recommendations for MRD testing by MFC. A percentage of cancer cells to leukocytes of less than 0.1% in a subject's bone marrow, measured by MFC according to the ELN's recommendations for MRD testing by MFC, indicates the subject is MRD negative (MRD-) by MFC according to the ELN's recommendations for MRD testing by MFC.

The ELN has also issued guidelines for molecular MRD testing in AML. The ELN defines complete molecular remission as complete morphologic remission plus two successive negative MRD samples obtained within an interval of ≥4 weeks at a sensitivity level of at least 1 in 1,000, wherein the samples are collected and measured according to the ELN guidelines for molecular MRD testing. The ELN defines molecular persistence at low copy numbers, which is associated with a low risk of relapse, as MRD with low copy numbers (<100-200 copies/10⁴ ABL copies corresponding to <1-2% of target to reference gene or allele burden) in patients with morphologic CR, and a copy number or relative increase <1 log between any two positive samples collected at the end of treatment, wherein the samples are collected and measured according to the ELN guidelines for molecular MRD testing. The ELN defines molecular progression in patients with molecular persistence as an increase of MRD copy numbers ≥1 log 10 between any two positive samples collected and measured according to the ELN guidelines for molecular MRD testing. The ELN defines molecular relapse as an increase of the MRD level of ≥1 log 10 between two positive samples in a patient who previously tested negative, wherein the samples are collected and measured according to the ELN guidelines for molecular MRD testing. Both molecular persistence and molecular relapse are indicators of an MRD-positive subject by RT-qPCR conducted according to the ELN guidelines for MRD testing by RT-qPCR. Thus, patients in complete molecular remission and patients labelled as having molecular persistence at low copy numbers are MRD-negative by RT-qPCR conducted according to the ELN guidelines for MRD testing by RT-qPCR. The ELN does not currently recommend using next-generation sequencing to assess MRD status. Thus, RT-qPCR is the recommended molecular approach to MRD testing, as discussed in Ravandi, F., et al. and Schuurhuis, G.J., et al. Specific recommendations for collecting and measuring samples (e.g., bone marrow samples) for MRD testing are described in Ravandi, F., et al., Blood Advances 12 June 2018, vol. 2, no. 11 and Schuurhuis, G. J., et al., Blood 2018 March 22, 131(12): 1275-1291, the relevant contents of which are incorporated herein by reference in their entireties.

When a subject having a hematologic cancer, such as AML, is described herein as being "measurable residual disease negative," "minimal residual disease negative," "MRD-negative" or "MRD⁻" without a further modifier, such as by MFC or by RT-qPCR, the subject is MRD negative according to at least one of the ELN's criteria described herein (*e.g.,* MFC, molecular biology). In some embodiments, the subject is MRD-negative by MFC conducted according to ELN guidelines for MRD testing. In some embodiments, the subject is MRD-negative by RT-qPCR conducted according to ELN guidelines for MRD testing. In some embodiments, the subject is MRD-negative by both MFC and RT-qPCR conducted according to ELN guidelines for MRD testing. In some embodiments, the subject is MRD-negative by MFC conducted according to ELN guidelines for MRD testing, and is MRD-positive by RT-qPCR conducted according to ELN guidelines for MRD testing. In some embodiments, the subject is MRD-positive by MFC conducted according to ELN guidelines for MRD testing, and is MRD-negative by RT-qPCR conducted according to ELN guidelines for MRD testing. When a subject is MRD-negative according to one of the ELN's criterion described herein (*e.g.,* the criterion for MFC), but MRD-positive according to another of the ELN's criterion described herein (*e.g.,* the criterion for RT-qPCR), that subject can still be described as MRD-negative according to the use of that term herein because the subject is MRD negative according to at least one of the ELN's criteria described herein.

When a subject having a hematologic cancer, such as AML, is described herein as being "measurable residual disease positive," "minimal residual disease positive," "MRD-positive" or "MRD⁺," the subject is MRD positive by the ELN's criteria for MFC and RT-qPCR described herein. For example, a subject that is MRD positive for AML can be MRD-positive by MFC conducted according to ELN guidelines for MRD testing in AML, and MRD-positive by RT-qPCR conducted according to ELN guidelines for MRD testing in AML.

Types of cancer that may be treated in various other embodiments include, but are not limited to: adenocarcinoma of the breast, prostate, and colon; all forms of bronchogenic carcinoma of the lung; myeloid; melanoma; hepatoma; neuroblastoma; papilloma; apudoma; choristoma; branchioma; malignant carcinoid syndrome; carcinoid heart disease; and carcinoma (e.g., Walker, basal cell, basosquamous, Brown-Pearce, ductal, Ehrlich tumor, Krebs 2, merkel cell, mucinous, non-small cell lung, oat cell, papillary, scirrhous, bronchiolar, bronchogenic, squamous cell, and transitional cell). Additional types of cancers that may be treated include: histiocytic disorders; leukemia; histiocytosis malignant; Hodgkin's disease; immunoproliferative small; non-Hodgkin's lymphoma; plasmacytoma; reticuloendotheliosis; melanoma; chondroblastoma; chondroma; chondrosarcoma; fibroma; fibrosarcoma; giant cell tumors; histiocytoma; lipoma; liposarcoma; mesothelioma; myxoma; myxosarcoma; osteoma; osteosarcoma; chordoma; craniopharyngioma; dysgerminoma; hamartoma; mesenchymoma; mesonephroma; myosarcoma; ameloblastoma; cementoma; odontoma; teratoma; thymoma; trophoblastic tumor. Further, the following types of cancers are also contemplated as amenable to treatment: adenoma; cholangioma; cholesteatoma; cyclindroma; cystadenocarcinoma; cystadenoma; granulosa cell tumor; gynandroblastoma; hepatoma; hidradenoma; islet cell tumor; Leydig cell tumor; papilloma; sertoli cell tumor; theca cell tumor; leimyoma; leiomyosarcoma; myoblastoma; myomma; myosarcoma; rhabdomyoma; rhabdomyosarcoma; ependymoma; ganglioneuroma; glioma; medulloblastoma; meningioma; neurilemmoma; neuroblastoma; neuroepithelioma; neurofibroma; neuroma; paraganglioma; paraganglioma nonchromaffin. The types of cancers that may be treated also include, but are not limited to, angiokeratoma; angiolymphoid hyperplasia with eosinophilia; angioma sclerosing; angiomatosis; glomangioma; hemangioendothelioma; hemangioma; hemangiopericytoma; hemangiosarcoma; lymphangioma; lymphangiomyoma; lymphangiosarcoma; pinealoma; carcinosarcoma; chondrosarcoma; cystosarcoma phyllodes; fibrosarcoma; hemangiosarcoma; leiomyosarcoma; leukosarcoma; liposarcoma; lymphangiosarcoma; myosarcoma; myxosarcoma; ovarian carcinoma; rhabdomyosarcoma; sarcoma; neoplasms; nerofibromatosis; and cervical dysplasia.

In addition to cancers, embodiments of the treatment methods disclosed herein are also amenable to treatment of autoimmune diseases. For example, some embodiments provide a method for treating graft vs. host disease (GvHD) in a mammal in need thereof, the method comprising administering to the mammal an effective amount of a PIM kinase inhibitor and optionally a JAK inhibitor. In some specific embodiments, the GvHD is the result of a stem cell transplant (*e.g.,* bone marrow transplant), a blood transfusion, or organ transplant (*e.g.,* thymus transplant). In some embodiments, the GvHD is acute. In some embodiments, the GvHD is chronic. In some specific embodiments, the method further comprises administering an angiotensin mimetic (*e.g.,* TXA127).

Some embodiments provide a method for treating lupus in a mammal in need thereof, the method comprising administering to the mammal an effective amount of a PIM kinase inhibitor and optionally a JAK inhibitor. In some more specific embodiments, the method further comprises administering a non-steroidal antiinflammatory drug (*e.g.,* naproxen sodium, ibuprofen, *etc*.), an anti-malarial drug (*e.g.,* hydroxychloroquine), a corticosteroid (*e.g.,* prednisone, methylprednisolone), an immunosuppressant (*e.g.,* azathioprine, mycophenolate mofetil, methotrexate), a biologic (*e.g.,* belimumab, rituximab) or combinations thereof.

Other embodiments provide a method for treating irritable bowel disease in a mammal in need thereof, the method comprising administering to the mammal an effective amount of a PIM kinase inhibitor and optionally a JAK inhibitor. In some embodiments, the method further comprises administering fiber supplements (*e.g.,* psyllium), a laxative (*e.g.,* magnesium hydroxide / milk of magnesia, polyethylene glycol), an anti-diarrheal medication (*e.g.,* loperamide, cholestyramine, colestipol, colesevelam), anti-cholinergic medication (*e.g.,* dicyclomine), a tricyclic antidepressant (*e.g.,* imipramine, desipramine, nortriptyline), SSRI anti-depressants (*e.g.,* fluoxetine, paroxetine), a pain medication (*e.g.,* pregabalin, gabapentin), or combinations thereof. In certain more specific embodiments, the method further comprises administering alosetron, eluxadoline, rifaximin, lubiprostone, linaclotide or combinations thereof.

Certain embodiments provide a method for treating Crohn's disease in a mammal in need thereof, the method comprising administering to the mammal an effective amount of a PIM kinase inhibitor and optionally a JAK inhibitor. In certain more specific embodiments, the method further comprises administering an antiinflammatory drug (e.g., corticosteroids, oral 5-aminosalicylates), immune system suppressors (*e.g.,* azathioprine, mercaptopurine, infliximab, adalimumab, certolizumab pegol, methotrexate, natalizumab, vedolizumab, ustekinumab), an antibiotic (*e.g.,* ciprofloxacin, metronidazole), an anti-diarrheal, a pain reliever (*e.g.,* acetaminophen), an iron supplement, a vitamin B-12 shot, a calcium supplement, a vitamin D supplement or combinations thereof.

Some embodiments provide a method for treating a disease associated with interleukin-22 (IL-22) in a mammal in need thereof, the method comprising administering to the mammal an effective amount of a PIM kinase inhibitor and optionally a JAK inhibitor. In certain embodiments, the disease associated with interleukin-22 (IL-22) is ulcerative colitis, or acute polymicrobial sepsis.

### II. Pharmaceutical compositions

Other embodiments are directed to pharmaceutical compositions. The PIM kinase inhibitor (*e.g.,* Compound 1) and/or JAK inhibitor (*e.g.,* ruxolitinib) or other therapeutic agent may be formulated together or separately according to methods known in the art. Certain embodiments comprise a pharmaceutically acceptable carrier or excipient, a PIM kinase inhibitor and/or a JAK inhibitor. In some embodiments, the pharmaceutical composition comprises a PIM kinase inhibitor and/or JAK inhibitor according to any of the foregoing described embodiments.

In some embodiments, the pharmaceutical composition is formulated for oral administration. In other embodiments, the pharmaceutical composition is formulated for injection.

Suitable routes of administration include, but are not limited to, oral, intravenous, rectal, aerosol, parenteral, ophthalmic, pulmonary, transmucosal, transdermal, vaginal, otic, nasal, and topical administration. In addition, by way of example only, parenteral delivery includes intramuscular, subcutaneous, intravenous, intramedullary injections, as well as intrathecal, direct intraventricular, intraperitoneal, intralymphatic, and intranasal injections.

In certain embodiments, a compound as described herein is administered in a local rather than systemic manner, for example, via injection of the compound directly into an organ, often in a depot preparation or sustained release formulation. In specific embodiments, long acting formulations are administered by implantation (for example, subcutaneously or intramuscularly) or by intramuscular injection. Furthermore, in other embodiments, the drug is delivered in a targeted drug delivery system, for example, in a liposome coated with organ specific antibody. In such embodiments, the liposomes are targeted to and taken up selectively by the organ. In yet other embodiments, the compound as described herein is provided in the form of a rapid release formulation, in the form of an extended release formulation, or in the form of an intermediate release formulation. In yet other embodiments, the compound described herein is administered topically.

The compounds according to embodiments of the disclosure are effective over a wide dosage range. For example, in the treatment of adult humans, dosages from 0.01 to 10,000 mg, from 0.5 to 1000 mg, from 1 to 500 mg per day, and from 5 to 400 mg per day are examples of dosages that are used in some embodiments. In some embodiments the dosage ranges from about 1 to about 300 mg per day, from about 1 to about 200 mg per day, from about 1 to about 100 mg per day, from about 1 to about 50 mg per day, from about 10 to about 25 mg per day, from about 100 to about 15 mg per day, from about 1 to about 10,000 mg per day, from about 100 to about 10,000 mg per day, from about 1000 to about 10,000 mg per day, from about 5000 to about 10,000 mg per day, from about 100 to about 5000 mg per day, from about 100 to about 1000 mg per day, from about 10 to about 500 mg per day, from about 10 to about 400 mg per day, from about 10 to about 250 mg per day, from about 100 to about 250 mg per day, from about 100 to about 200 mg per day, from about 1 to about 500 mg per day, from about 1 to about 250 mg per day, from about 0.5 to about 250 mg per day, from about 0.5 to about 100 mg per day, from about 0.1 to about 100 mg per day or from about 0.1 to about 50 mg per day. The exact dosage will depend upon the route of administration, the form in which the compound is administered, the subject to be treated, the body weight of the subject to be treated, and the preference and experience of the attending physician.

In some embodiments, a PIM kinase inhibitor (*e.g.,* Compound 1, or a pharmaceutically acceptable salt thereof) is administered in a dosage of from about 250 mg to about 2.5 g per day, from about 300 mg to about 1.5 g per day, or from about 450 mg to about 1.5 g per day. In some embodiments, the PIM kinase inhibitor (*e.g.,* Compound, or a pharmaceutically acceptable salt thereof) is administered in a dosage of about 240 mg, about 480 mg, about 720 mg or about 960 mg. In some embodiments, the PIM kinase inhibitor (*e.g.,* Compound, or a pharmaceutically acceptable salt thereof) is administered in a dosage of about 180 mg, 360 mg, 540 mg, 720 mg, 900 mg or 1,080 mg.

In embodiments involving a JAK inhibitor (*e.g.,* ruxolitinib, or a pharmaceutically acceptable salt thereof), dosages range from about 1 mg to about 100 mg per day, from about 2.5 mg to about 60 mg per day, from about 5 mg to about 60 mg per day or from about 10 mg to about 50 mg per day. In some embodiments, a JAK inhibitor (*e.g.,* ruxolitinib, or a pharmaceutically acceptable salt thereof) is administered in a dosage of from about 5 mg to about 100 mg per day, or from about 10 mg to about 50 mg per day. Ruxolitinib, for example, is typically given as an oral formulation twice daily in an individual dose of about 5 mg, about 10 mg, about 15 mg, about 20 mg, about 25 mg or about 30 mg.

Typically, the starting dose of ruxolitinib is 20 mg given orally twice daily for patients with a platelet count greater than 200 X 10⁹/L, and 15 mg twice daily for patients with a platelet count between 100 X 10⁹/L and 200 X 10⁹/L. The dose of ruxolitinib can be increased based on patient response, up to a maximum of 25 mg twice daily. If a patient receiving ruxolitinib under these conditions for six months does not have spleen reduction or symptom improvement, ruxolitinib treatment is typically discontinued.

In some embodiments, a PIM kinase inhibitor and/or JAK kinase inhibitor is administered in a single dose. Typically, such administration will be by injection, *e.g.,* intravenous injection, in order to introduce the agent quickly. However, other routes are used as appropriate. Accordingly, in some embodiments, a PIM kinase inhibitor (*e.g.,* Compound 1, or a pharmaceutically acceptable salt thereof) and/or JAK kinase inhibitor (*e.g.,* ruxolitinib, or a pharmaceutically acceptable salt thereof) is administered orally. A single dose of a PIM kinase inhibitor may also be used for treatment of an acute condition.

In some embodiments, a PIM kinase inhibitor (*e.g.,* Compound 1, or a pharmaceutically acceptable salt thereof) and/or JAK kinase inhibitor (*e.g.,* ruxolitinib, or a pharmaceutically acceptable salt thereof) is administered in multiple doses. In some embodiments, dosing is about once, twice, three times, four times, five times, six times, or more than six times per day. In other embodiments, dosing is about once a month, once every two weeks, once a week, or once every other day. In another embodiment a PIM kinase inhibitor and another agent (*e.g.,* a JAK2 inhibitor) are administered together about once per day to about 6 times per day. In another embodiment the administration of a PIM kinase inhibitor and another agent (*e.g.,* JAK inhibitor) continues for less than about 7 days. In yet another embodiment, the administration continues for more than about 6, 10, 14, 28 days, two months, six months, or one year. In another embodiment, the administration continues for from about seven days to about five years (*e.g.,* from about seven days to about two years, from about seven days to about one year). In another embodiment, the administration continues for 28 days. In another embodiment, the administration continues for one year. In some cases, continuous dosing is achieved and maintained as long as necessary.

Administration of the PIM kinase inhibitor and optionally JAK kinase inhibitor may continue as long as necessary. In some embodiments, a PIM kinase inhibitor and optionally JAK kinase are administered for more than 1, 2, 3, 4, 5, 6, 7, 14, or 28 days. In some embodiments, a PIM kinase inhibitor and optionally JAK kinase inhibitor are administered for less than 28, 14, 7, 6, 5, 4, 3, 2, or 1 day. In some embodiments, a PIM kinase inhibitor and optionally JAK kinase inhibitor are administered chronically on an ongoing basis, e.g., for the treatment of chronic effects.

In some embodiments, one or more cycles (*e.g.,* 1, 2, 3, 4, 5, 6, 7, 8, 9, 10, 11, 12, *etc.* cycles) of the PIM kinase inhibitor (*e.g.,* Compound 1, or a pharmaceutically acceptable salt thereof) and/or JAK kinase inhibitor (*e.g.,* ruxolitinib, or a pharmaceutically acceptable salt thereof) are administered. In some embodiments, the PIM kinase inhibitor (*e.g.,* Compound 1, or a pharmaceutically acceptable salt thereof) and/or JAK kinase inhibitor (*e.g.,* ruxolitinib, or a pharmaceutically acceptable salt thereof) are administered on a cycle, for example, a 28-day cycle. Accordingly, in some embodiments, one or more cycles of the PIM kinase inhibitor and/or JAK kinase inhibitor (*e.g.,* PIM kinase inhibitor) are each independently administered once or twice per day for 28 days on a 28-day cycle.

In some embodiments, the PIM kinase inhibitor and optionally JAK kinase are administered in dosages. Due to intersubject variability in compound pharmacokinetics, individualization of dosing regimen is provided in certain embodiments. Dosing for a compound of embodiments of the disclosure may be found by routine experimentation in light of the instant disclosure and/or can be derived by one of ordinary skill in the art.

In some embodiments, the PIM kinase inhibitors and optionally JAK kinase inhibitor are formulated into pharmaceutical compositions. In specific embodiments, pharmaceutical compositions are formulated in a conventional manner using one or more physiologically acceptable carriers comprising excipients and auxiliaries which facilitate processing of the active compounds into preparations which can be used pharmaceutically. Proper formulation is dependent upon the route of administration chosen. Any pharmaceutically acceptable techniques, carriers, and excipients are used as suitable to formulate the pharmaceutical compositions described herein: Remington: The Science and Practice of Pharmacy, Nineteenth Ed (Easton, Pa.: Mack Publishing Company, 1995); Hoover, John E., Remington's Pharmaceutical Sciences, Mack Publishing Co., Easton, Pennsylvania 1975; Liberman, H.A. and Lachman, L., Eds., Pharmaceutical Dosage Forms, Marcel Decker, New York, N.Y., 1980; and Pharmaceutical Dosage Forms and Drug Delivery Systems, Seventh Ed. (Lippincott Williams & Wilkins1999).

A pharmaceutical composition, as used herein, refers to a mixture of an inhibitor targeting PIM kinase or a combination of an inhibitor of PIM kinase and an inhibitor of JAK kinase with other chemical components, such as carriers, stabilizers, diluents, dispersing agents, suspending agents, thickening agents, and/or excipients. In certain embodiments, the pharmaceutical composition facilitates administration of the compound(s) to an organism. In some embodiments, practicing the methods of treatment or use provided herein, therapeutically effective amount(s) of inhibitors of PIM kinase and optionally JAK kinase inhibitors are administered in a pharmaceutical composition to a mammal having a disease, disorder or medical condition to be treated. In specific embodiments, the mammal is a human. In certain embodiments, therapeutically effective amounts vary depending on the severity of the disease, the age and relative health of the subject, the potency of the compound used and other factors.

In one embodiment, the inhibitor(s) are formulated in an aqueous solution. In specific embodiments, the aqueous solution is selected from, by way of example only, a physiologically compatible buffer, such as Hank's solution, Ringer's solution, or physiological saline buffer. In other embodiments, inhibitors targeting at least two super-enhancer components are formulated for transmucosal administration. In specific embodiments, transmucosal formulations include penetrants that are appropriate to the barrier to be permeated. In still other embodiments wherein the compounds described herein are formulated for other parenteral injections; appropriate formulations include aqueous or non-aqueous solutions. In specific embodiments, such solutions include physiologically compatible buffers and/or excipients.

In another embodiment, compounds described herein are formulated for oral administration. Compounds described herein are formulated by combining the active compounds with, *e.g.,* pharmaceutically acceptable carriers or excipients. In various embodiments, the compounds described herein are formulated in oral dosage forms that include, by way of example only, tablets, powders, pills, dragees, capsules, liquids, gels, syrups, elixirs, slurries, suspensions and the like.

In certain embodiments, pharmaceutical preparations for oral use are obtained by mixing one or more solid excipient with one or more of the compounds described herein, optionally grinding the resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as: for example, maize starch, wheat starch, rice starch, potato starch, gelatin, gum tragacanth, methylcellulose, microcrystalline cellulose, hydroxypropylmethylcellulose, sodium carboxymethylcellulose; or others such as: polyvinylpyrrolidone (PVP or povidone) or calcium phosphate. In specific embodiments, disintegrating agents are optionally added. Disintegrating agents include, by way of example only, cross linked croscarmellose sodium, polyvinylpyrrolidone, agar, or alginic acid or a salt thereof such as sodium alginate.

In one embodiment, dosage forms, such as dragee cores and tablets, are provided with one or more suitable coating. In specific embodiments, concentrated sugar solutions are used for coating the dosage form. The sugar solutions, optionally contain additional components, such as and by way of example only, gum arabic, talc, polyvinylpyrrolidone, carbopol gel, polyethylene glycol, and/or titanium dioxide, lacquer solutions, and suitable organic solvents or solvent mixtures. Dyestuffs and/or pigments are also optionally added to the coatings for identification purposes. Additionally, the dyestuffs and/or pigments are optionally utilized to characterize different combinations of active compound doses.

In certain embodiments, therapeutically effective amounts of inhibitors targeting PIM kinase and optionally JAK kinase inhibitors are formulated into other oral dosage forms. Oral dosage forms include push fit capsules made of gelatin, as well as soft, sealed capsules made of gelatin and a plasticizer, such as glycerol or sorbitol. In specific embodiments, push fit capsules contain the active ingredients in admixture with one or more fillers. Fillers include, by way of example only, lactose, binders such as starches, and/or lubricants such as talc or magnesium stearate and, optionally, stabilizers. In other embodiments, soft capsules contain one or more active compounds that are dissolved or suspended in a suitable liquid. Suitable liquids include, by way of example only, one or more fatty oils, liquid paraffins, or liquid polyethylene glycols. In addition, stabilizers are optionally added.

A particular composition comprises a PIM kinase inhibitor (*e.g.,* Compound 1, or a pharmaceutically acceptable salt thereof), and a polyglycolized glyceride.

"Polyglycolized glycerides" refers to mixtures of monoesters, diesters and triesters of glycerols and monoesters and diesters of polyethylene glycols with a mean relative molecular mass between about 200 and 6000. Polyglycolized glycerides may be obtained by partial transesterification of triglycerides with polyethylene glycol or by esterification of glycerol and polyethylene glycol with fatty acids. In some embodiments, the fatty acid component contains between 8-22 carbon atoms, for example, between 10-18 carbon atoms. Examples of natural vegetable oils from which polyglycolized glycerides can be derived include palm kernel oil and palm oil. Suitable polyol compounds generally have a molecular weight ranging from about 200 to about 6000 g/mol and preferably contain polyethylene glycol, although other polyols may be employed, such as polyglycerols or sorbitol. Polyglycolized glycerides are available on the market under the trade name Gelucire^{®}. Examples of polyglycolized glycerides useful in various embodiments include WL 2514CS, LABRASOL, LABRAFIL, Gelucire 44/14 (lauroyl polyoxy-32 glycerides), Gelucire 33/01, Gelucire 35/10, Gelucire 37/02, Gelucire 50/13, Gelucire 44/11 and mixtures thereof.

"Gelucire^{®} 44/14" or "Gelucire 44/14" is a lipid-based excipient manufactured by Gattefosse Corporation, Westwood, N.J. comprising a mixture of pegylated fatty acid esters and glycerides. The number 44 denotes the melting point of the compound and 14 indicates hydrophile/lipophile balance (HLB) value. Other Gelucire excipients similarly indicate values for melting point and HLB values. For example, Gelucire 33/01, Gelucire 35/10, Gelucire 37/02, Gelucire 50/13 and Gelucire 44/11.

With respect to the desired application and delivery of the composition, the melting point of the polyglycolized glyceride can be selected such that the therapeutic effectiveness of the composition is optimized. Accordingly, in some embodiments, the polyglycolized glyceride has a melting point ranging from about 30 to about 50 °C. In related embodiments the polyglycolized glyceride has a melting point ranging from about 31 to about 49 °C, about 32 to about 48 °C, about 33 to about 48 °C, about 34 to about 48 °C, about 35 to about 48 °C, about 36 to about 48 °C, about 37 to about 48 °C, about 38 to about 47 °C, about 39 to about 46 °C, about 40 to about 45 °C, about 41 to about 45 °C, about 42 to about 45 °C or about 43 to about 45 °C. In certain specific embodiments, the polyglycolized glyceride has a melting point of about 44 °C.

Relatedly, the hydrophile/lipophile balance of the polyglycolized glyceride can also be selected to optimize embodiments of the composition. Thus, in certain embodiments, the polyglycolized glyceride has a hydrophile/lipophile balance (HLB) value ranging from about 8 to about 18, about 9 to about 17, about 9 to about 16, about 10 to about 16, about 11 to about 15, about 11 to about 15, about 12 to about 15, or about 13 to about 15. In certain specific embodiments, the polyglycolized glyceride has hydrophile/lipophile balance value of about 14.

A hydrophile/lipophile balance value can be determined by Griffin's method. The HLB value is determined according to the following equation: $HLB = 20 \times \left({M}_{h}/M\right)$ wherein, Mₕ is the molecular mass of the hydrophilic portion of the molecule, and M is the molecular mass of the entire molecule. Thus, the value of the HLB ranges from 0 to 20, with a value of 0 corresponding to a lipophilic (*i.e*., hydrophobic) molecule and a value of 20 corresponding to a hydrophilic (*i.e.,* lipophobic) molecule.

In some embodiments, the composition of the PIM kinase inhibitor and the polyglycolized glyceride is formulated for oral administration, *e.g.,* in the form of tablets, lozenges, aqueous or oily suspensions, dispersible powders or granules, emulsion, hard or soft capsules, or syrups or elixirs.

In some embodiments, the composition comprises Compound 1, or a pharmaceutically acceptable salt thereof, and the polyglycolized glyceride at a weight ratio ranging from about 1:1 to about 1:10, as determined using the molecular weight of Compound 1 as a free base (*i.e.,* having a molecular weight of 419.92). In some more specific embodiments, the composition comprises Compound 1, or a pharmaceutically acceptable salt thereof, and the polyglycolized glyceride at a weight ratio ranging from about 1:1.25 to about 1:10, from about 1:1.5 to about 1:10, from about 1:1.75 to about 1:10, from about 1:2 to about 1:10, from about 1:2 to about 1:9, from about 1:2.5 to about 1:8; from about 1:3 to about 1:7, from about 1:4 to about 1:6 as determined using the molecular weight of Compound 1 as a free base. In some specific embodiments, the composition comprises Compound 1, or a pharmaceutically acceptable salt thereof, and the polyglycolized glyceride at a weight ratio of about 1:5 as determined using the molecular weight of Compound 1 as a free base. In some specific embodiments, the composition comprises Compound 1, or a pharmaceutically acceptable salt thereof, and the polyglycolized glyceride at a weight ratio of about 1:2.6 as determined using the molecular weight of Compound 1 as a free base. In some other embodiments, the composition comprises Compound 1, or a pharmaceutically acceptable salt thereof, and the polyglycolized glyceride at a weight ratio of about of 1:3, 1:4, 1:4.5, 1:5.5, or 1:6, as determined using the molecular weight of Compound 1 as a free base.

In some specific embodiments, the concentration of Compound 1, or a pharmaceutically acceptable salt thereof, in the composition ranges from about 10 wt% to about 25 wt% as determined using the molecular weight of Compound 1 as a hydrochloride salt. In some other embodiments, the composition comprises Compound 1, or a pharmaceutically acceptable salt thereof, in a concentration ranging from about 14 wt% to about 22 wt% as determined using the molecular weight of Compound 1 as a hydrochloride salt. In some other embodiments, the composition comprises Compound 1, or a pharmaceutically acceptable salt thereof, in a concentration of about 18.38 wt% as determined using the molecular weight of Compound 1 as a hydrochloride salt. In some more specific embodiments, the composition comprises Compound 1, or a pharmaceutically acceptable salt thereof, in a concentration of about 18.38 ± 0.2 wt% as determined using the molecular weight of Compound 1 as a hydrochloride salt. In some more specific embodiments, the composition comprises Compound 1, or a pharmaceutically acceptable salt thereof, in a concentration of about 18.38 ± 0.4 wt% as determined using the molecular weight of Compound 1 as a hydrochloride salt. In some more specific embodiments, the composition comprises Compound 1, or a pharmaceutically acceptable salt thereof, in a concentration of about 18.38 ± 0.8 wt% as determined using the molecular weight of Compound 1 as a hydrochloride salt.

Similarly, in some embodiments, the composition comprises the polyglycolized glyceride in a concentration ranging from about 75 wt% to about 90 wt%. In some embodiments, the composition comprises the polyglycolized glyceride in a concentration ranging from about 78 wt% to about 84 wt%. In some more specific embodiments, the composition comprises the polyglycolized glyceride at a concentration of about 81.62 wt%. In some embodiments, the composition comprises the polyglycolized glyceride at a concentration of about 81.62 ± 0.5 wt%. In some embodiments, the composition comprises the polyglycolized glyceride at a concentration of about 81.62 ± 1 wt%. In some embodiments, the composition comprises the polyglycolized glyceride at a concentration of about 81.62 ± 2 wt%.

In some specific embodiments, the concentration of Compound 1, or a pharmaceutically acceptable salt thereof, in the composition ranges from about 15 wt% to about 35 wt% as determined using the molecular weight of Compound 1 as a hydrochloride salt. In some other embodiments, the composition comprises Compound 1, or a pharmaceutically acceptable salt thereof, in a concentration ranging from about 20 wt% to about 30 wt% as determined using the molecular weight of Compound 1 as a hydrochloride salt. In some other embodiments, the composition comprises Compound 1, or a pharmaceutically acceptable salt thereof, in a concentration of about 25 wt% as determined using the molecular weight of Compound 1 as a hydrochloride salt. In some more specific embodiments, the composition comprises Compound 1, or a pharmaceutically acceptable salt thereof, in a concentration of about 25 ± 0.2 wt% as determined using the molecular weight of Compound 1 as a hydrochloride salt. In some more specific embodiments, the composition comprises Compound 1, or a pharmaceutically acceptable salt thereof, in a concentration of about 25 ± 0.4 wt% as determined using the molecular weight of Compound 1 as a hydrochloride salt. In some more specific embodiments, the composition comprises Compound 1, or a pharmaceutically acceptable salt thereof, in a concentration of about 25 ± 0.8 wt% as determined using the molecular weight of Compound 1 as a hydrochloride salt.

Similarly, in some embodiments, the composition comprises the polyglycolized glyceride in a concentration ranging from about 65 wt% to about 85 wt%. In some embodiments, the composition comprises the polyglycolized glyceride in a concentration ranging from about 70 wt% to about 80 wt%. In some more specific embodiments, the composition comprises the polyglycolized glyceride at a concentration of about 75 wt%. In some embodiments, the composition comprises the polyglycolized glyceride at a concentration of about 75 ± 0.5 wt%. In some embodiments, the composition comprises the polyglycolized glyceride at a concentration of about 75 ± 1 wt%. In some embodiments, the composition comprises the polyglycolized glyceride at a concentration of about 75 ± 2 wt%.

In some specific embodiments, the concentration of Compound 1, or a pharmaceutically acceptable salt thereof, in the composition ranges from about 23.3 wt% to about 43.3 wt% as determined using the molecular weight of Compound 1 as a hydrochloride salt. In some other embodiments, the composition comprises Compound 1, or a pharmaceutically acceptable salt thereof, in a concentration ranging from about 28.3 wt% to about 38.3 wt% as determined using the molecular weight of Compound 1 as a hydrochloride salt. In some other embodiments, the composition comprises Compound 1, or a pharmaceutically acceptable salt thereof, in a concentration of about 33.3 wt% as determined using the molecular weight of Compound 1 as a hydrochloride salt. In some more specific embodiments, the composition comprises Compound 1, or a pharmaceutically acceptable salt thereof, in a concentration of about 33.3 ± 0.2 wt% as determined using the molecular weight of Compound 1 as a hydrochloride salt. In some more specific embodiments, the composition comprises Compound 1, or a pharmaceutically acceptable salt thereof, in a concentration of about 33.3 ± 0.4 wt% as determined using the molecular weight of Compound 1 as a hydrochloride salt. In some more specific embodiments, the composition comprises Compound 1, or a pharmaceutically acceptable salt thereof, in a concentration of about 33.3 ± 0.8 wt% as determined using the molecular weight of Compound 1 as a hydrochloride salt.

Similarly, in some embodiments, the composition comprises the polyglycolized glyceride in a concentration ranging from about 56.7 wt% to about 76.7 wt%. In some embodiments, the composition comprises the polyglycolized glyceride in a concentration ranging from about 61.7 wt% to about 71.7 wt%. In some more specific embodiments, the composition comprises the polyglycolized glyceride at a concentration of about 66.7 wt%. In some embodiments, the composition comprises the polyglycolized glyceride at a concentration of about 66.7 ± 0.5 wt%. In some embodiments, the composition comprises the polyglycolized glyceride at a concentration of about 66.7 ± 1 wt%. In some embodiments, the composition comprises the polyglycolized glyceride at a concentration of about 66.7 ± 2 wt%.

In some embodiments, the composition comprises from about 100 mg to about 300 mg of Compound 1, or a pharmaceutically acceptable salt thereof, as determined using the molecular weight of Compound 1 as a free base. In some embodiments, the composition comprises from about 100 mg to about 150 mg of Compound 1, or a pharmaceutically acceptable salt thereof, as determined using the molecular weight of Compound 1 as a free base. In some embodiments, the composition comprises from about 115 mg to about 125 mg of Compound 1, or a pharmaceutically acceptable salt thereof, as determined using the molecular weight of Compound 1 as a free base. In some specific embodiments, the composition comprises about 120 mg of Compound 1, or a pharmaceutically acceptable salt thereof, as determined using the molecular weight of Compound 1 as a free base. In some embodiments, the composition comprises about 120 ± 0.5 mg of Compound 1, or a pharmaceutically acceptable salt thereof, as determined using the molecular weight of Compound 1 as a free base. In some embodiments, the composition comprises about 120 ± 1 mg of Compound 1, or a pharmaceutically acceptable salt thereof, as determined using the molecular weight of Compound 1 as a free base. In some embodiments, the composition comprises about 120 ± 3 mg of Compound 1, or a pharmaceutically acceptable salt thereof, as determined using the molecular weight of Compound 1 as a free base.

Similarly, in some embodiments, the composition comprises from about 500 mg to about 700 mg of the polyglycolized glyceride. In some embodiments, the composition comprises from about 550 mg to about 650 mg of the polyglycolized glyceride. In some embodiments, the composition comprises from about 560 mg to about 600 mg of the polyglycolized glyceride. In some embodiments, the composition comprises about 587.7 mg of the polyglycolized glyceride. In some embodiments, the composition comprises about 587.7 ± 1 mg of the polyglycolized glyceride. In some embodiments, the composition comprises about 587.7 ± 2 mg of the polyglycolized glyceride. In some embodiments, the composition comprises about 587.7 ± 5 mg of the polyglycolized glyceride.

In some embodiments, the composition comprises from about 160 mg to about 200 mg of Compound 1, or a pharmaceutically acceptable salt thereof, as determined using the molecular weight of Compound 1 as a free base. In some embodiments, the composition comprises from about 175 mg to about 185 mg of Compound 1, or a pharmaceutically acceptable salt thereof, as determined using the molecular weight of Compound 1 as a free base. In some specific embodiments, the composition comprises about 180 mg of Compound 1, or a pharmaceutically acceptable salt thereof, as determined using the molecular weight of Compound 1 as a free base. In some embodiments, the composition comprises about 180 ± 0.5 mg of Compound 1, or a pharmaceutically acceptable salt thereof, as determined using the molecular weight of Compound 1 as a free base. In some embodiments, the composition comprises about 180 ± 1 mg of Compound 1, or a pharmaceutically acceptable salt thereof, as determined using the molecular weight of Compound 1 as a free base. In some embodiments, the composition comprises about 180 ± 3 mg of Compound 1, or a pharmaceutically acceptable salt thereof, as determined using the molecular weight of Compound 1 as a free base.

Similarly, in some embodiments, the composition comprises from about 520 mg to about 560 mg of the polyglycolized glyceride. In some embodiments, the composition comprises from about 535 mg to about 545 mg of the polyglycolized glyceride. In some embodiments, the composition comprises about 540 mg of the polyglycolized glyceride. In some embodiments, the composition comprises about 540 ± 1 mg of the polyglycolized glyceride. In some embodiments, the composition comprises about 540 ± 2 mg of the polyglycolized glyceride. In some embodiments, the composition comprises about 540 ± 5 mg of the polyglycolized glyceride.

In some embodiments, the composition comprises from about 220 mg to about 260 mg of Compound 1, or a pharmaceutically acceptable salt thereof, as determined using the molecular weight of Compound 1 as a free base. In some embodiments, the composition comprises from about 235 mg to about 245 mg of Compound 1, or a pharmaceutically acceptable salt thereof, as determined using the molecular weight of Compound 1 as a free base. In some specific embodiments, the composition comprises about 240 mg of Compound 1, or a pharmaceutically acceptable salt thereof, as determined using the molecular weight of Compound 1 as a free base. In some embodiments, the composition comprises about 240 ± 0.5 mg of Compound 1, or a pharmaceutically acceptable salt thereof, as determined using the molecular weight of Compound 1 as a free base. In some embodiments, the composition comprises about 240 ± 1 mg of Compound 1, or a pharmaceutically acceptable salt thereof, as determined using the molecular weight of Compound 1 as a free base. In some embodiments, the composition comprises about 240 ± 3 mg of Compound 1, or a pharmaceutically acceptable salt thereof, as determined using the molecular weight of Compound 1 as a free base.

Similarly, in some embodiments, the composition comprises from about 440 mg to about 500 mg of the polyglycolized glyceride. In some embodiments, the composition comprises from about 475 mg to about 485 mg of the polyglycolized glyceride. In some embodiments, the composition comprises about 480 mg of the polyglycolized glyceride. In some embodiments, the composition comprises about 480 ± 1 mg of the polyglycolized glyceride. In some embodiments, the composition comprises about 480 ± 2 mg of the polyglycolized glyceride. In some embodiments, the composition comprises about 480 ± 5 mg of the polyglycolized glyceride.

Compound 1 used in the composition may be in free-base form, or in a pharmaceutically acceptable salt form. In some embodiments, Compound 1 is present as a free base. In some embodiments, Compound 1 is present as a salt. In some embodiments, Compound 1 is present as a hydrochloride salt.

A composition comprising a PIM kinase inhibitor (*e.g.,* Compound 1, or a pharmaceutically acceptable salt thereof) and a polyglycolized glyceride can optionally be used in place of the PIM kinase inhibitor (*e.g.,* Compound 1, or a pharmaceutically acceptable salt thereof) in any of the methods disclosed herein.

In other embodiments, therapeutically effective amounts of PIM kinase inhibitors and/or JAK kinase inhibitors are formulated for buccal or sublingual administration. Formulations suitable for buccal or sublingual administration include, by way of example only, tablets, lozenges, or gels. In still other embodiments, the compounds described herein are formulated for parental injection, including formulations suitable for bolus injection or continuous infusion. In specific embodiments, formulations for injection are presented in unit dosage form (*e.g.,* in ampoules) or in multi dose containers. Preservatives are, optionally, added to the injection formulations. In still other embodiments, the pharmaceutical compositions are formulated in a form suitable for parenteral injection as sterile suspensions, solutions or emulsions in oily or aqueous vehicles. Parenteral injection formulations optionally contain formulatory agents such as suspending, stabilizing and/or dispersing agents. In specific embodiments, pharmaceutical formulations for parenteral administration include aqueous solutions of the active compounds in water soluble form. In additional embodiments, suspensions of the active compounds (*e.g.,* therapeutically effective amounts of PIM kinase inhibitors and/or JAK kinase inhibitors) are prepared as appropriate oily injection suspensions. Suitable lipophilic solvents or vehicles for use in the pharmaceutical compositions described herein include, by way of example only, fatty oils such as sesame oil, or synthetic fatty acid esters, such as ethyl oleate or triglycerides, or liposomes. In certain specific embodiments, aqueous injection suspensions contain substances which increase the viscosity of the suspension, such as sodium carboxymethyl cellulose, sorbitol, or dextran. Optionally, the suspension contains suitable stabilizers or agents which increase the solubility of the compounds to allow for the preparation of highly concentrated solutions. Alternatively, in other embodiments, the active ingredient is in powder form for constitution with a suitable vehicle, *e.g.,* sterile pyrogen-free water, before use.

In still other embodiments, therapeutically effective amounts of PIM kinase inhibitors and/or JAK kinase inhibitors are administered topically. The compounds described herein are formulated into a variety of topically administrable compositions, such as solutions, suspensions, lotions, gels, pastes, medicated sticks, balms, creams or ointments. Such pharmaceutical compositions optionally contain solubilizers, stabilizers, tonicity enhancing agents, buffers and preservatives.

In yet other embodiments, therapeutically effective amounts of PIM kinase inhibitors and/or JAK kinase inhibitors are formulated for transdermal administration. In specific embodiments, transdermal formulations employ transdermal delivery devices and transdermal delivery patches and can be lipophilic emulsions or buffered, aqueous solutions, dissolved and/or dispersed in a polymer or an adhesive. In various embodiments, such patches are constructed for continuous, pulsatile, or on demand delivery of pharmaceutical agents. In additional embodiments, the transdermal delivery of inhibitors is accomplished by means of iontophoretic patches and the like. In certain embodiments, transdermal patches provide controlled delivery of inhibitors. In specific embodiments, the rate of absorption is slowed by using rate-controlling membranes or by trapping the compound within a polymer matrix or gel. In alternative embodiments, absorption enhancers are used to increase absorption. Absorption enhancers or carriers include absorbable pharmaceutically acceptable solvents that assist passage through the skin. For example, in one embodiment, transdermal devices are in the form of a bandage comprising a backing member, a reservoir containing the compound optionally with carriers, optionally a rate controlling barrier to deliver the compound to the skin of the host at a controlled and predetermined rate over a prolonged period of time, and means to secure the device to the skin.

In other embodiments, therapeutically effective amounts of PIM kinase inhibitors and/or JAK kinase inhibitors are formulated for administration by inhalation. Various forms suitable for administration by inhalation include, but are not limited to, aerosols, mists or powders. Pharmaceutical compositions of inhibitors are conveniently delivered in the form of an aerosol spray presentation from pressurized packs or a nebulizer, with the use of a suitable propellant (*e.g.,* dichlorodifluoromethane, trichlorofluoromethane, dichlorotetrafluoroethane, carbon dioxide or other suitable gas). In specific embodiments, the dosage unit of a pressurized aerosol is determined by providing a valve to deliver a metered amount. In certain embodiments, capsules and cartridges of, such as, by way of example only, gelatin for use in an inhaler or insufflator are formulated containing a powder mix of the compound and a suitable powder base such as lactose or starch.

In still other embodiments, therapeutically effective amounts of PIM kinase inhibitors and/or JAK kinase inhibitors are formulated in rectal compositions such as enemas, rectal gels, rectal foams, rectal aerosols, suppositories, jelly suppositories, or retention enemas, containing conventional suppository bases such as cocoa butter or other glycerides, as well as synthetic polymers such as polyvinylpyrrolidone, PEG, and the like. In suppository forms of the compositions, a low-melting wax such as, but not limited to, a mixture of fatty acid glycerides, optionally in combination with cocoa butter is first melted.

In certain embodiments, pharmaceutical compositions are formulated in any conventional manner using one or more physiologically acceptable carriers comprising excipients and auxiliaries which facilitate processing of the active compounds into preparations which can be used pharmaceutically. Proper formulation is dependent upon the route of administration chosen. Any pharmaceutically acceptable techniques, carriers, and excipients are optionally used as suitable. Pharmaceutical compositions comprising inhibitors are manufactured in a conventional manner, such as, by way of example only, by means of conventional mixing, dissolving, granulating, dragee-making, levigating, emulsifying, encapsulating, entrapping or compression processes.

Pharmaceutical compositions include at least one pharmaceutically acceptable carrier, diluent or excipient and inhibitors, described herein as an active ingredient. The active ingredient is in free-acid or free-base form, or in a pharmaceutically acceptable salt form. In addition, the methods and pharmaceutical compositions described herein include the use of N-oxides, crystalline forms (also known as polymorphs), as well as active metabolites of these compounds having the same type of activity. All tautomers of the compounds described herein are included within the scope of the compounds presented herein. Additionally, the compounds described herein encompass unsolvated as well as solvated forms with pharmaceutically acceptable solvents such as water, ethanol, and the like. The solvated forms of inhibitors presented herein are also considered to be disclosed herein. In addition, the pharmaceutical compositions optionally include other medicinal or pharmaceutical agents, carriers, adjuvants, such as preserving, stabilizing, wetting or emulsifying agents, solution promoters, salts for regulating the osmotic pressure, buffers, and/or other therapeutically valuable substances.

Methods for the preparation of compositions comprising therapeutically effective amounts of PIM kinase inhibitors and/or JAK kinase inhibitors described herein include formulating the compounds with one or more inert, pharmaceutically acceptable excipients or carriers to form a solid, semi-solid or liquid. Solid compositions include, but are not limited to, powders, tablets, dispersible granules, capsules, cachets, and suppositories. Liquid compositions include solutions in which a compound is dissolved, emulsions comprising a compound, or a solution containing liposomes, micelles, or nanoparticles comprising a compound as disclosed herein. Semi-solid compositions include, but are not limited to, gels, suspensions and creams. The form of the pharmaceutical compositions described herein include liquid solutions or suspensions, solid forms suitable for solution or suspension in a liquid prior to use, or as emulsions. These compositions also optionally contain minor amounts of nontoxic, auxiliary substances, such as wetting or emulsifying agents, pH buffering agents, and so forth.

In some embodiments, pharmaceutical compositions comprising therapeutically effective amounts of PIM kinase inhibitors and/or JAK kinase inhibitors illustratively take the form of a liquid where the agents are present in solution, in suspension or both. Typically, when the composition is administered as a solution or suspension a first portion of the agent is present in solution and a second portion of the agent is present in particulate form, in suspension in a liquid matrix. In some embodiments, a liquid composition includes a gel formulation. In other embodiments, the liquid composition is aqueous.

In certain embodiments, useful aqueous suspensions contain one or more polymers as suspending agents. Useful polymers include water-soluble polymers such as cellulosic polymers, *e.g.,* hydroxypropyl methylcellulose, and water-insoluble polymers such as cross-linked carboxyl-containing polymers. Certain pharmaceutical compositions described herein comprise a mucoadhesive polymer, selected, for example, from carboxymethylcellulose, carbomer (acrylic acid polymer), poly(methylmethacrylate), polyacrylamide, polycarbophil, acrylic acid/butyl acrylate copolymer, sodium alginate and dextran.

Useful pharmaceutical compositions also, optionally, include solubilizing agents to aid in the solubility of inhibitors. The term "solubilizing agent" generally includes agents that result in formation of a micellar solution or a true solution of the agent. Certain acceptable nonionic surfactants, for example polysorbate 80, are useful as solubilizing agents, as are ophthalmically acceptable glycols, polyglycols, *e.g.,* polyethylene glycol 400, and glycol ethers.

Furthermore, useful pharmaceutical compositions optionally include one or more pH adjusting agents or buffering agents, including acids such as acetic, boric, citric, lactic, phosphoric and hydrochloric acids; bases such as sodium hydroxide, sodium phosphate, sodium borate, sodium citrate, sodium acetate, sodium lactate and tris-hydroxymethylaminomethane; and buffers such as citrate/dextrose, sodium bicarbonate and ammonium chloride. Such acids, bases and buffers are included in an amount required to maintain pH of the composition in an acceptable range.

Additionally, useful compositions also, optionally, include one or more salts in an amount required to bring osmolality of the composition into an acceptable range. Such salts include those having sodium, potassium or ammonium cations and chloride, citrate, ascorbate, borate, phosphate, bicarbonate, sulfate, thiosulfate or bisulfite anions; suitable salts include sodium chloride, potassium chloride, sodium thiosulfate, sodium bisulfite and ammonium sulfate.

Other useful pharmaceutical compositions optionally include one or more preservatives to inhibit microbial activity. Suitable preservatives include mercury-containing substances such as merfen and thiomersal; stabilized chlorine dioxide; and quaternary ammonium compounds such as benzalkonium chloride, cetyltrimethylammonium bromide and cetylpyridinium chloride.

Still other useful compositions include one or more surfactants to enhance physical stability or for other purposes. Suitable nonionic surfactants include polyoxyethylene fatty acid glycerides and vegetable oils, *e.g.,* polyoxyethylene (60) hydrogenated castor oil; and polyoxyethylene alkylethers and alkylphenyl ethers, *e.g.,* octoxynol 10, octoxynol 40.

Still other useful compositions include one or more antioxidants to enhance chemical stability where required. Suitable antioxidants include, by way of example only, ascorbic acid and sodium metabisulfite.

In certain embodiments, aqueous suspension compositions are packaged in single-dose non-reclosable containers. Alternatively, multiple-dose reclosable containers are used, in which case it is typical to include a preservative in the composition.

In alternative embodiments, other delivery systems for hydrophobic pharmaceutical compounds are employed. Liposomes and emulsions are examples of delivery vehicles or carriers useful herein. In certain embodiments, organic solvents such as *N*-methylpyrrolidone are also employed. In additional embodiments, the compounds described herein are delivered using a sustained release system, such as semipermeable matrices of solid hydrophobic polymers containing the therapeutic agent. Various sustained release materials are useful herein. In some embodiments, sustained release capsules release the compounds for a few weeks up to over 100 days. Depending on the chemical nature and the biological stability of the therapeutic reagent, additional strategies for protein stabilization are employed.

In certain embodiments, the therapeutically effective amount of the PIM kinase inhibitor (*e.g.,* Compound 1) and/or JAK inhibitor is encapsulated in a lipid nanoparticle (LNP), solid nanoparticle, or liposome. In some embodiments, the lipid nanoparticle (LNP), solid nanoparticle, or liposome comprises a cationic lipid. In some embodiments, the lipid nanoparticle (LNP), solid nanoparticle, or liposome is a multilamellar or unilamellar liposomal vesicle.

Cationic liposomes are readily available. For example, N[1-2,3-dioleyloxy)propyl]-N,N,N-triethylammonium (DOTMA) liposomes are available under the tradename Lipofectin^{®} (GIBCO BRL, Grand Island, N.Y.). Similarly, anionic and neutral liposomes are readily available as well, e.g., from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), and dioleoylphoshatidyl ethanolamine (DOPE), among others. These materials can also be mixed with DOTMA in appropriate ratios. Methods for making liposomes using these materials are well known in the art.

Lipid nanoparticles encapsulating a PIM kinase inhibitor (*e.g.,* Compound 1) and/or JAK inhibitor may further be provided in a formulation that contains a suitable gel or suspension, such as an aqueous suspension, which may include a tissue retention-enhancing or thickening agent such as, for example, hydroxyethyl cellulose or carboxymethyl cellulose.

In certain embodiments, the formulations described herein comprise one or more antioxidants, metal chelating agents, thiol containing compounds and/or other general stabilizing agents. Examples of such stabilizing agents, include, but are not limited to: (a) about 0.5% to about 2% w/v glycerol, (b) about 0.1% to about 1% w/v methionine, (c) about 0.1% to about 2% w/v monothioglycerol, (d) about 1 mM to about 10 mM EDTA, (e) about 0.01% to about 2% w/v ascorbic acid, (f) 0.003% to about 0.02% w/v polysorbate 80, (g) 0.001% to about 0.05% w/v. polysorbate 20, (h) arginine, (i) heparin, (j) dextran sulfate, (k) cyclodextrins, (l) pentosan polysulfate and other heparinoids, (m) divalent cations such as magnesium and zinc; or (n) combinations thereof.

In some embodiments, the concentration of one or more inhibitors provided in the pharmaceutical composition is less than 100%, 90%, 80%, 70%, 60%, 50%, 40%, 30%, 20%, 19%, 18%, 17%, 16%, 15%,14%, 13%, 12%, 11%, 10%, 9%, 8%, 7%, 6%, 5%, 4%, 3%, 2%, 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% w/w, w/v or v/v.

In some embodiments, the concentration of one or more inhibitors greater than 90%, 80%, 70%, 60%, 50%, 40%, 30%, 20%, 19.75%, 19.50%, 19.25% 19%, 18.75%, 18.50%, 18.25% 18%, 17.75%, 17.50%, 17.25% 17%, 16.75%, 16.50%, 16.25% 16%, 15.75%, 15.50%, 15.25% 15%, 14.75%, 14.50%, 14.25% 14%, 13.75%, 13.50%, 13.25% 13%, 12.75%, 12.50%, 12.25% 12%, 11.75%, 11.50%, 11.25% 11%, 10.75%, 10.50%, 10.25% 10%, 9.75%, 9.50%, 9.25% 9%, 8.75%, 8.50%, 8.25% 8%, 7.75%, 7.50%, 7.25% 7%, 6.75%, 6.50%, 6.25% 6%, 5.75%, 5.50%, 5.25% 5%, 4.75%, 4.50%, 4.25%, 4%, 3.75%, 3.50%, 3.25%, 3%, 2.75%, 2.50%, 2.25%, 2%, 1.75%, 1.50%, 125% , 1%, 0.5%, 0.4%, 0.3%, 0.2%, 0.1%, 0.09%, 0.08%, 0.07%, 0.06%, 0.05%, 0.04%, 0.03%, 0.02%, 0.01%, 0.009%, 0.008%, 0.007%, 0.006%, 0.005%, 0.004%, 0.003%, 0.002%, 0.001%, 0.0009%, 0.0008%, 0.0007%, 0.0006%, 0.0005%, 0.0004%, 0.0003%, 0.0002%, or 0.0001% w/w, w/v, or v/v.

In some embodiments, the concentration of one or more inhibitors is in the range from approximately 0.0001% to approximately 50%, approximately 0.001% to approximately 40 %, approximately 0.01% to approximately 30%, approximately 0.02% to approximately 29%, approximately 0.03% to approximately 28%, approximately 0.04% to approximately 27%, approximately 0.05% to approximately 26%, approximately 0.06% to approximately 25%, approximately 0.07% to approximately 24%, approximately 0.08% to approximately 23%, approximately 0.09% to approximately 22%, approximately 0.1% to approximately 21%, approximately 0.2% to approximately 20%, approximately 0.3% to approximately 19%, approximately 0.4% to approximately 18%, approximately 0.5% to approximately 17%, approximately 0.6% to approximately 16%, approximately 0.7% to approximately 15%, approximately 0.8% to approximately 14%, approximately 0.9% to approximately 12%, approximately 1% to approximately 10% w/w, w/v or v/v.

In some embodiments, the concentration of one or more inhibitors is in the range from approximately 0.001% to approximately 10%, approximately 0.01% to approximately 5%, approximately 0.02% to approximately 4.5%, approximately 0.03% to approximately 4%, approximately 0.04% to approximately 3.5%, approximately 0.05% to approximately 3%, approximately 0.06% to approximately 2.5%, approximately 0.07% to approximately 2%, approximately 0.08% to approximately 1.5%, approximately 0.09% to approximately 1%, approximately 0.1% to approximately 0.9% w/w, w/v or v/v.

In some embodiments, the amount of one or more inhibitors is equal to or less than 10 g, 9.5 g, 9.0 g, 8.5 g, 8.0 g, 7.5 g, 7.0 g, 6.5 g, 6.0 g, 5.5 g, 5.0 g, 4.5 g, 4.0 g, 3.5 g, 3.0 g, 2.5 g, 2.0 g, 1.5 g, 1.0 g, 0.95 g, 0.9 g, 0.85 g, 0.8 g, 0.75 g, 0.7 g, 0.65 g, 0.6 g, 0.55 g, 0.5 g, 0.45 g, 0.4 g, 0.35 g, 0.3 g, 0.25 g, 0.2 g, 0.15 g, 0.1 g, 0.09 g, 0.08 g, 0.07 g, 0.06 g, 0.05 g, 0.04 g, 0.03 g, 0.02 g, 0.01 g, 0.009 g, 0.008 g, 0.007 g, 0.006 g, 0.005 g, 0.004 g, 0.003 g, 0.002 g, 0.001 g, 0.0009 g, 0.0008 g, 0.0007 g, 0.0006 g, 0.0005 g, 0.0004 g, 0.0003 g, 0.0002 g, or 0.0001 g.

In some embodiments, the amount of one or more inhibitors is more than 0.0001 g, 0.0002 g, 0.0003 g, 0.0004 g, 0.0005 g, 0.0006 g, 0.0007 g, 0.0008 g, 0.0009 g, 0.001 g, 0.0015 g, 0.002 g, 0.0025 g, 0.003 g, 0.0035 g, 0.004 g, 0.0045 g, 0.005 g, 0.0055 g, 0.006 g, 0.0065 g, 0.007 g, 0.0075 g, 0.008 g, 0.0085 g, 0.009 g, 0.0095 g, 0.01 g, 0.015 g, 0.02 g, 0.025 g, 0.03 g, 0.035 g, 0.04 g, 0.045 g, 0.05 g, 0.055 g, 0.06 g, 0.065 g, 0.07 g, 0.075 g, 0.08 g, 0.085 g, 0.09 g, 0.095 g, 0.1 g, , 0.15 g, 0.2 g, , 0.25 g, 0.3 g, , 0.35 g, 0.4 g, , 0.45 g, 0.5 g, 0.55 g, 0.6 g, , 0.65 g, 0.7 g, 0.75 g, 0.8 g, 0.85 g, 0.9 g, 0.95 g, 1 g, 1.5 g, 2 g, 2.5, 3 g, 3.5, 4 g, 4.5 g, 5 g, 5.5 g, 6 g, 6.5g, 7 g, 7.5g, 8 g, 8.5 g, 9 g, 9.5 g, or 10 g.

In some embodiments, the amount of one or more inhibitors is in the range of 0.0001-10 g, 0.0005-9 g, 0.001-8 g, 0.005-7 g, 0.01-6 g, 0.05-5 g, 0.1-4 g, 0.5-4 g, or 1-3 g.

The PIM kinase inhibitor (e.g., Compound 1) and optional JAK inhibitor can be administered concurrently or separately. For example, one of the inhibitors may be administered via a bolus followed by a separate bolus of the second inhibitor after an appropriate period of time. Slower administration, such as a longer duration infusion can be used for administration of one or both of the inhibitors. The skilled clinician can determine appropriate administration methods and orders, which are all within the scope of the present disclosure.

Some embodiments thus provide a separate dosage form comprising a PIM kinase inhibitor (*e.g.,* Compound 1, or a pharmaceutically acceptable salt thereof) and an additional therapeutic agent (*e.g.,* an additional therapeutic agent described herein), wherein the PIM kinase inhibitor and the additional therapeutic agent are associated with one another. The term "associated with one another," as used herein, means that the separate dosage forms are packaged together or otherwise attached to one another such that it is readily apparent that the separate dosage forms are intended to be sold and/or administered together, for example, according to a method described herein. In some embodiments, the dosage form comprises a PIM kinase inhibitor (*e.g.,* Compound 1, or a pharmaceutically acceptable salt thereof) and a JAK kinase inhibitor (*e.g.,* ruxolitinib, or a pharmaceutically acceptable salt thereof). In some embodiments, the PIM kinase inhibitor is as described herein. In some embodiments, the JAK inhibitor is as described herein.

Some related embodiments provide a kit comprising a PIM kinase inhibitor (*e.g.,* Compound 1) and written instructions for administering the PIM kinase inhibitor for treatment of a myeloproliferative neoplasm is provided. In more specific embodiments, the kit further comprises a JAK inhibitor and written instructions for administering the JAK inhibitor in combination with the PIM kinase inhibitor. In some embodiments, the PIM kinase inhibitor is as described herein. In some embodiments, the JAK inhibitor is as described herein. In some embodiments, the myeloproliferative neoplasm is as described herein.

The PIM kinase inhibitors and optional JAK inhibitors can be prepared according to methods known in the art. Exemplary preparation procedures are provided in PCT Pub. Nos: WO 2016/161248; WO 2014/052365; WO 2015/048689; WO 2015/002894; WO 2014/168975; WO 2014/159745; WO 2014/130693; WO 2014/078578; WO 2014/018567; WO 2013/184572; WO 2013/173518; WO 2013/116382; WO 2013/102059; WO 2013/059738; WO 2013/010136; WO 2013/013188; WO 2011/153514; WO 2011/046964; WO 2010/009342; WO 2008/121742; WO 2008/054827; WO 2008/039218; WO 2008/058126; WO 2007/087068; and in U.S. Pub. Nos: 2015/0018336; 2014/0336206; 2014/0329807; 2014/0243355; 2014/0212485; 2014/0194446/ 2014/0187564; 2014/0135347; 2014/0128414; 2014/0187565; 2014/0171453; 2014/0163027; 2014/01663046; 2014/0142126; 2014/0142123; 2014/0128413; 2014/0079690; 2014/0080844; 2014/0057907; 2014/0039168; 2013/0338172; 2013/0310402; 2013/0273030; 2013/0197014; 2013/0035334; 2013/0012525; 2012/0283277; 2012/0283276; 2012/0277254; 2012/0252821; 2010/0331350, and U.S. Patent No. 7,750,007, the full disclosures of which are hereby incorporated by reference.

### EXAMPLE EMBODIMENTS OF THE INVENTION

Embodiment 1. A method for treating a myeloproliferative neoplasm in a mammal in need thereof, the method comprising administering to the mammal an effective amount of a compound represented by the following structural formula: 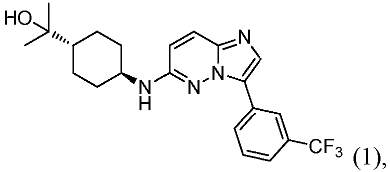 or a pharmaceutically acceptable salt thereof.

Embodiment 2. The method of Embodiment 1, comprising administering to the mammal from about 250 mg to about 2.5 g per day of the compound of structural formula 1, or a pharmaceutically acceptable salt thereof.

Embodiment 3. The method of Embodiment 2, comprising administering to the mammal from about 300 mg to about 1.5 g per day of the compound of structural formula 1, or a pharmaceutically acceptable salt thereof.

Embodiment 4. The method of Embodiment 3, comprising administering to the mammal from about 450 mg to about 1.5 g per day of the compound of structural formula 1, or a pharmaceutically acceptable salt thereof.

Embodiment 5. The method of any one of the preceding embodiments, wherein the myeloproliferative neoplasm is myelofibrosis.

Embodiment 6. The method of Embodiment 5, wherein the myelofibrosis is intermediate-risk myelofibrosis or high-risk myelofibrosis.

Embodiment 7. The method of Embodiment 5 or 6, wherein the myelofibrosis is primary myelofibrosis.

Embodiment 8. The method of Embodiment 5 or 6, wherein the myelofibrosis is secondary myelofibrosis.

Embodiment 9. The method of any one of the preceding embodiments, wherein treating the myeloproliferative neoplasm results in the mammal being measurable residual disease (MRD)-negative.

Embodiment 10. The method of any one of the preceding embodiments, wherein treating the myeloproliferative neoplasm results in in the mammal.

Embodiment 11. The method of any one of the preceding embodiments, wherein the compound of structural formula 1, or a pharmaceutically acceptable salt thereof, is administered orally.

Embodiment 12. The method of any one of the preceding embodiments, wherein the compound of structural formula 1, or a pharmaceutically acceptable salt thereof, is administered once daily.

Embodiment 13. The method of any one of Embodiments 1-11, wherein the compound of structural formula 1, or a pharmaceutically acceptable salt thereof, is administered twice daily.

Embodiment 14. The method of any one of the preceding embodiments, wherein the compound of structural formula 1, or a pharmaceutically acceptable salt thereof, is administered for from about 7 days to about one year.

Embodiment 15. The method of Embodiment 14, wherein the compound of structural formula 1, or a pharmaceutically acceptable salt thereof, is administered for 28 days.

Embodiment 16. The method of Embodiment 14, wherein the compound of structural formula 1, or a pharmaceutically acceptable salt thereof, is administered for one year.

Embodiment 17. The method of any one of the preceding embodiments, further comprising administering to the mammal an effective amount of an additional chemotherapeutic agent.

Embodiment 18. A method for treating a myeloproliferative neoplasm in a mammal in need thereof, the method comprising administering to the mammal an effective amount of each of:
a compound represented by the following structural formula: 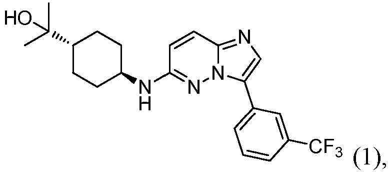
or a pharmaceutically acceptable salt thereof; and
ruxolitinib, or a pharmaceutically acceptable salt thereof.

Embodiment 19. The method of Embodiment 18, comprising administering to the mammal from about 250 mg to about 2.5 g per day of the compound of structural formula 1, or a pharmaceutically acceptable salt thereof.

Embodiment 20. The method of Embodiment 19, comprising administering to the mammal from about 300 mg to about 1.5 g per day of the compound of structural formula 1, or a pharmaceutically acceptable salt thereof.

Embodiment 21. The method of Embodiment 20, comprising administering to the mammal from about 450 mg to about 1.5 g per day of the compound of structural formula 1, or a pharmaceutically acceptable salt thereof.

Embodiment 22. The method of any one of Embodiments 18-21, wherein the myeloproliferative neoplasm is myelofibrosis.

Embodiment 23. The method of Embodiment 22, wherein the myelofibrosis is intermediate-risk myelofibrosis or high-risk myelofibrosis.

Embodiment 24. The method of any one of Embodiments 18-23, wherein treating the myeloproliferative neoplasm results in the mammal being measurable residual disease (MRD)-negative.

Embodiment 25. The method of any one of Embodiments 18-24, wherein treating the myeloproliferative neoplasm results in complete remission in the mammal.

Embodiment 26. The method of any one of Embodiments 18-25, wherein the compound of structural formula 1, or a pharmaceutically acceptable salt thereof, is administered orally.

Embodiment 27. The method of any one of Embodiments 18-26, wherein the compound of structural formula 1, or a pharmaceutically acceptable salt thereof is administered once daily.

Embodiment 28. The method of any one of Embodiments 18-26, wherein the compound of structural formula 1, or a pharmaceutically acceptable salt thereof is administered twice daily.

Embodiment 29. The method of any one of Embodiments 18-28, wherein the compound of structural formula 1, or a pharmaceutically acceptable salt thereof, is administered for from about seven days to about one year.

Embodiment 30. The method of Embodiment 29, wherein the compound of structural formula 1, or a pharmaceutically acceptable salt thereof, is administered for 28 days.

Embodiment 31. The method of Embodiment 29, wherein the compound of structural formula 1, or a pharmaceutically acceptable salt thereof, is administered for one year.

Embodiment 32. The method of any one of Embodiments 18-31, wherein the effective amount of ruxolitinib, or a pharmaceutically acceptable salt thereof, is from about 5 mg/day to about 100 mg/day.

Embodiment 33. The method of Embodiment 32, wherein the effective amount of ruxolitinib, or a pharmaceutically acceptable salt thereof, is from about 10 mg/day to about 50 mg/day.

Embodiment 34. The method of any one of Embodiments 18-33, wherein the ruxolitinib, or a pharmaceutically acceptable salt thereof, is administered orally.

Embodiment 35. The method of any one of Embodiments 18-34, wherein the ruxolitinib, or a pharmaceutically acceptable salt thereof, is administered twice daily.

Embodiment 36. The method of any one of Embodiments 18-35, wherein the ruxolitinib, or a pharmaceutically acceptable salt thereof, is administered for from about seven days to about one year.

Embodiment 37. A method for treating a solid tumor in a mammal in need thereof, the method comprising administering to the mammal an effective amount of:
a compound represented by the following structural formula: 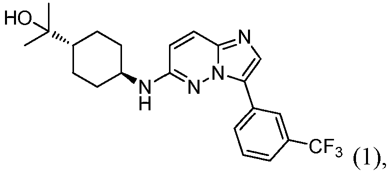
or a pharmaceutically acceptable salt thereof; and
ruxolitinib, or a pharmaceutically acceptable salt thereof.

Embodiment 38. The method of Embodiment 37, wherein the solid tumor is a prostate tumor.

Embodiment 39. The method of Embodiment 37 or 38, comprising administering to the mammal from about 300 mg to about 1.5 g per day of the compound of structural formula 1, or a pharmaceutically acceptable salt thereof.

Embodiment 40. The method of claim Embodiment 39, comprising administering to the mammal from about 450 mg to about 1.5 g per day of the compound of structural formula 1, or a pharmaceutically acceptable salt thereof.

Embodiment 41. The method of any one of Embodiments 38-40, wherein treating the solid tumor results in the mammal being measurable residual disease (MRD)-negative.

Embodiment 42. The method of any one of Embodiments 38-41, wherein treating the solid tumor results in complete remission in the mammal.

Embodiment 43. The method of any one of Embodiments 38-42, wherein the compound of structural formula 1, or a pharmaceutically acceptable salt thereof, is administered orally.

Embodiment 44. The method of any one of Embodiments 38-43, wherein the compound of structural formula 1, or a pharmaceutically acceptable salt thereof, is administered for from about seven days to about one year.

Embodiment 45. The method of Embodiment 44, wherein the compound of structural formula 1, or a pharmaceutically acceptable salt thereof, is administered for 28 days.

Embodiment 46. The method of Embodiment 44, wherein the compound of structural formula 1, or a pharmaceutically acceptable salt thereof, is administered for one year.

Embodiment 47. The method of any one of Embodiments 38-46, wherein the effective amount of ruxolitinib, or a pharmaceutically acceptable salt thereof, is from about 5 mg/day to about 100 mg/day.

Embodiment 48. The method of Embodiment 47, wherein the effective amount of ruxolitinib, or a pharmaceutically acceptable salt thereof, is from about 10 mg/day to about 50 mg/day.

Embodiment 49. The method of any one of Embodiments 38-48, wherein the ruxolitinib, or a pharmaceutically acceptable salt thereof, is administered orally.

Embodiment 50. The method of any one of Embodiments 38-49, wherein the ruxolitinib, or a pharmaceutically acceptable salt thereof, is administered twice daily.

Embodiment 51. The method of any one of Embodiments 39-50, wherein the ruxolitinib, or a pharmaceutically acceptable salt thereof, is administered for from about seven days to about one year.

Embodiment 52. A method for treating a previously treated cancer in a mammal in need thereof, the method comprising administering to the mammal an effective amount of a compound represented by the following structural formula: 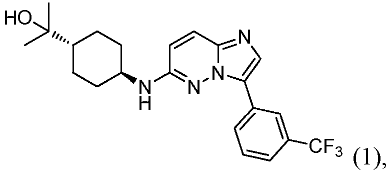 or a pharmaceutically acceptable salt thereof.

Embodiment 53. The method of Embodiment 52, wherein the cancer has been previously treated with ruxolitinib.

Embodiment 54. The method of Embodiment 53 or 54, wherein the cancer is a ruxolitinib-resistant cancer.

Embodiment 55. The method of any one of Embodiments 52-54, comprising administering to the mammal from about 250 mg to about 2.5 g per day of the compound of structural formula 1, or a pharmaceutically acceptable salt thereof.

Embodiment 56. The method of Embodiment 55, comprising administering to the mammal from about 300 mg to about 1.5 g per day of the compound of structural formula 1, or a pharmaceutically acceptable salt thereof.

Embodiment 57. The method of Embodiment 56, comprising administering to the mammal from about 450 mg to about 1.5 g per day of the compound of structural formula 1, or a pharmaceutically acceptable salt thereof.

Embodiment 58. The method of any one of Embodiments 52-57, wherein treating the cancer results in the mammal being measurable residual disease (MRD)-negative.

Embodiment 59. The method of any one of Embodiments 52-58, wherein treating the cancer results in complete remission in the mammal.

Embodiment 60. The method of any one of Embodiments 52-59, wherein the compound of structural formula 1, or a pharmaceutically acceptable salt thereof, is administered orally.

Embodiment 61. The method of any one of Embodiments 52-60, wherein the compound of structural formula 1, or a pharmaceutically acceptable salt thereof, is administered for from about seven days to about one year.

Embodiment 62. The method of Embodiment 61, wherein the compound of structural formula 1, or a pharmaceutically acceptable salt thereof, is administered for 28 days.

Embodiment 63. The method of Embodiment 61, wherein the compound of structural formula 1, or a pharmaceutically acceptable salt thereof, is administered for one year.

Embodiment 64. The method of any one of Embodiments 52-63, further comprising administering an effective amount of an additional chemotherapeutic agent to the mammal.

Embodiment 65. The method of any one of Embodiments 52-64, wherein the cancer is myelofibrosis.

Embodiment 66. A pharmaceutical composition comprising a pharmaceutically acceptable carrier or excipient; a compound represented by the following structural formula: 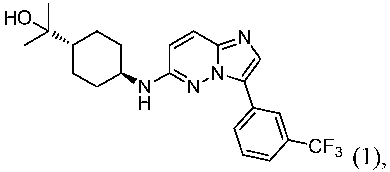 or a pharmaceutically acceptable salt thereof; and ruxolitinib, or a pharmaceutically acceptable salt thereof.

Embodiment 67. A kit, comprising a compound represented by the following structural formula: 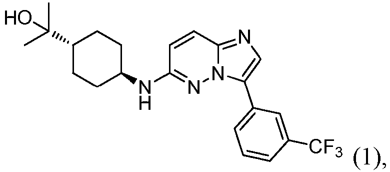 or a pharmaceutically acceptable salt thereof; and
written instructions for administering the compound of structural formula 1, or a pharmaceutically acceptable salt thereof, to treat a myeloproliferative neoplasm.

Embodiment 68. A kit, comprising a compound represented by the following structural formula: 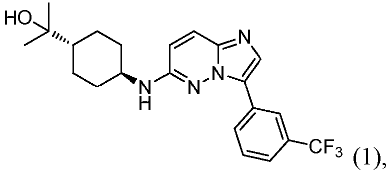
or a pharmaceutically acceptable salt thereof;
ruxolitinib, or a pharmaceutically acceptable salt thereof; and
written instructions for administering the compound of structural formula 1, or a pharmaceutically acceptable salt thereof, in combination with ruxolitinib, or a pharmaceutically acceptable salt thereof, to treat a myeloproliferative neoplasm.

Embodiment 69. A method for treating fibrosis associated with cancer in a mammal in need thereof, the method comprising administering to the mammal an effective amount of a compound represented by the following structural formula: 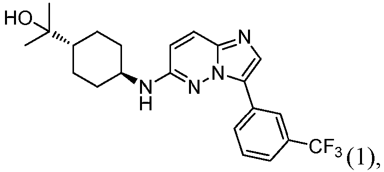 or a pharmaceutically acceptable salt thereof.

Embodiment 70. The method of Embodiment 69, comprising administering to the mammal from about 250 mg to about 2.5 g per day of the compound of structural formula 1, or a pharmaceutically acceptable salt thereof.

Embodiment 71. The method of Embodiment 70, comprising administering to the mammal from about 300 mg to about 1.5 g per day of the compound of structural formula 1, or a pharmaceutically acceptable salt thereof.

Embodiment 72. The method of Embodiment 71, comprising administering to the mammal from about 450 mg to about 1.5 g per day of the compound of structural formula 1, or a pharmaceutically acceptable salt thereof.

Embodiment 73. The method of any one of Embodiments 69-72, wherein treating the fibrosis results in complete remission in the mammal.

Embodiment 74. The method of any one of Embodiments 69-73, wherein the compound of structural formula 1, or a pharmaceutically acceptable salt thereof, is administered orally.

Embodiment 75. The method of any one of Embodiments 69-74, wherein the compound of structural formula 1, or a pharmaceutically acceptable salt thereof, is administered for from about seven days to about one year.

Embodiment 76. The method of Embodiment 75, wherein the compound of structural formula 1, or a pharmaceutically acceptable salt thereof, is administered for 28 days.

Embodiment 77. The method of Embodiment 75, wherein the compound of structural formula 1, or a pharmaceutically acceptable salt thereof, is administered for one year.

Embodiment 78. The method of any one of Embodiments 69-77, further comprising administering an effective amount of an additional chemotherapeutic agent to the mammal.

### ADDITIONAL EXAMPLE EMBODIMENTS

Embodiment 100. A method for treating a myeloproliferative neoplasm in a mammal in need thereof, the method comprising administering to the mammal an effective amount of a PIM kinase inhibitor, wherein the PIM kinase inhibitor is a compound having one of the following structures (I), (II) or (III): 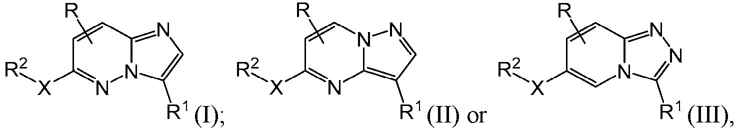 or a stereoisomer or pharmaceutically acceptable salt thereof, wherein:
X is a direct bond, N(R^{a}), S, O, SO or SO₂, wherein R^{a} is H or alkyl;
R is H, amino, cyano, hydroxyl, halo, alkyl, alkylaminyl, haloalkyl, alkoxy or haloalkoxy;
R¹ is phenyl, optionally substituted with 1, 2 or 3 R^{1'}, wherein R^{1'} is, at each occurrence, independently amino, cyano, alkyl, alkylaminyl, alkoxy, halo, haloalkyl, haloalkoxy, hydroxyl, nitro, alkylcarbonyl or alkylsulfonamidyl; and
R² has the following structure: 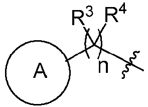 wherein:
   A is an optionally substituted 3-8 membered carbocyclic or heterocyclic ring;
   n is 0, 1, 2, 3 or 4; and
   R³ and R⁴ are, at each occurrence, independently H or alkyl.

Embodiment 101. The method of Embodiment 100, wherein the myeloproliferative neoplasm is polycythemia.

Embodiment 102. The method of Embodiment 100, wherein the myeloproliferative neoplasm is essential thrombocythemia.

Embodiment 103. The method of Embodiment 100, wherein the myeloproliferative neoplasm is myelofibrosis.

Embodiment 104. The method of Embodiments 100-103, wherein the mammal comprises a JAK2 mutation, a MPL mutation, or a CALR mutation.

Embodiment 105. The method of Embodiment 104, wherein the JAK2 mutation comprises a V617F mutation.

Embodiment 106. The method of Embodiment 104, wherein the MPL mutation comprises a W515L mutation.

Embodiment 107. The method of any one of Embodiments 100-106, wherein the myeloproliferative neoplasm is resistant to treatment with a JAK inhibitor.

Embodiment 108. The method of Embodiment 107, wherein the JAK inhibitor is a JAK1 inhibitor, a JAK2 inhibitor, or both.

Embodiment 109. The method of Embodiment 107 or 108, wherein the JAK inhibitor is selected from the group consisting of ruxolitinib, gandotinib, lestaurtinib, momelotinib, pacritinib, and fedratinib.

Embodiment 110. The method of any one of Embodiments 107-109, wherein the JAK inhibitor is ruxolitinib.

Embodiment 111. A method for decreasing proliferation of hematopoietic cells in a mammal, the method comprising contacting the cells with a PIM kinase inhibitor.

Embodiment 112. The method of any one of Embodiments 100-111, wherein the PIM kinase inhibitor has structure (I), wherein structure (I) and the values and variables for structure (I) are as defined anywhere herein (*e.g.,* in Embodiment 100).

Embodiment 113. The method of any one of Embodiments 100-110 and 112, wherein A is optionally substituted cyclohexyl.

Embodiment 114. The method of Embodiment 113, wherein the cyclohexyl is substituted with hydroxylalkyl.

Embodiment 115. The method of any one of Embodiments 100-110 and 112-114, wherein X is NH.

Embodiment 116. The method of any one of Embodiments 100-110 and 112-115, wherein at least one occurrence of R^{1'} is H.

Embodiment 117. The method of any one of Embodiments 100-110 and 112-116, wherein at least one occurrence of R^{1'} is trifluoromethyl.

Embodiment 118. The method of any one of Embodiments 100-117, wherein the PIM kinase inhibitor has the following structure 1:

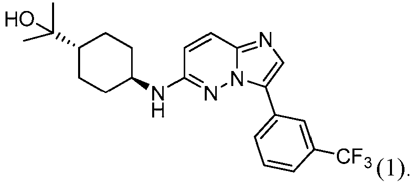

Embodiment 119. A method for treating a subject having or at risk of developing fibrosis associated with cancer, the method comprising administering to the subject a composition comprising a therapeutically effective amount of Compound 1: 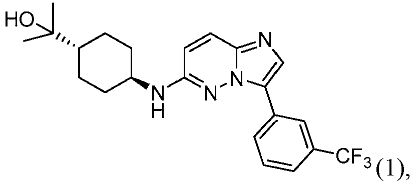 or a pharmaceutically acceptable salt thereof.

Embodiment 120. A method for treating one or more symptoms of fibrosis associated with cancer in a subject, the method comprising administering to the subject a composition comprising a therapeutically effective amount of Compound 1: 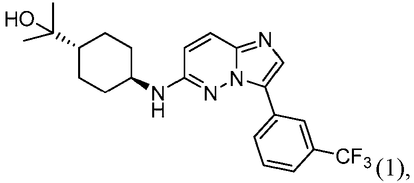 or a pharmaceutically acceptable salt thereof.

Embodiment 121. A method for treating fibrosis associated with cancer in a tissue, the method comprising contacting the tissue with Compound 1: 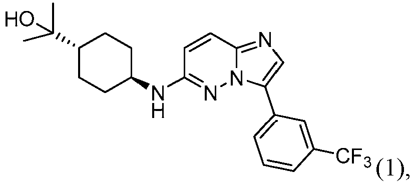 or a pharmaceutically acceptable salt thereof, in an amount sufficient to decrease or inhibit the further development of fibrosis.

Embodiment 122. A method for prophylactically treating a subject having or at risk of developing fibrosis associated with cancer, the method comprising administering to the subject a composition comprising a therapeutically effective amount of Compound 1: 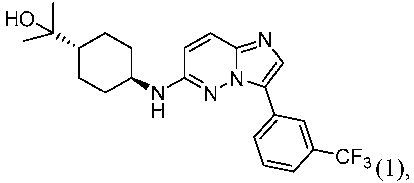 or a pharmaceutically acceptable salt thereof.

Embodiment 123. The method of any one of Embodiments 119-122, wherein the cancer is a solid tumor.

Embodiment 124. The method of any one of Embodiments 119-123, wherein the cancer is sarcoma of an internal organ.

Embodiment 125. The method of any one of Embodiments 119-124, wherein the cancer is pancreatic cancer, lung cancer, liver cancer, breast cancer, ovarian cancer, endometrial cancer, uterine sarcoma, renal cell cancer, or kidney cancer.

Embodiment 126. The method of any one of Embodiments 119-125, wherein the cancer is pancreatic cancer.

Embodiment 127. The method of Embodiment 126, wherein the pancreatic cancer is pancreatic ductal adenocarcinoma.

Embodiment 128. The method of Embodiment 125, wherein the cancer is liver cancer.

Embodiment 129. The method of Embodiment 125, wherein the cancer is lung cancer.

Embodiment 130. The method of Embodiment 125, wherein the cancer is breast cancer.

Embodiment 131. The method of Embodiment 130, wherein the breast cancer is inflammatory breast cancer.

Embodiment 132. The method of Embodiment 125, wherein the cancer is ovarian cancer.

Embodiment 133. The method of Embodiment 132, wherein the ovarian cancer is high grade serious ovarian cancer.

Embodiment 134. The method of Embodiment 125, wherein the cancer is endometrial cancer.

Embodiment 135. The method of Embodiment 125, wherein the cancer is uterine sarcoma.

Embodiment 136. The method of Embodiment 135, wherein the uterine sarcoma is uterine leiomyosarcoma.

Embodiment 137. The method of Embodiment 125, wherein the cancer is renal cell cancer.

Embodiment 138. The method of Embodiment 125, wherein the cancer is kidney cancer.

Embodiment 139. The method of any one of Embodiments 119-124, wherein the cancer is malignant fibrous histiocytoma, soft tissue sarcoma, fibrosarcoma, or dermatofibrosarcoma protuberans.

Embodiment 140. The method of any one of Embodiments 100-139, wherein the method further comprises administering to the mammal an effective amount of a JAK inhibitor.

Embodiment 141. The method of Embodiment 140, wherein the JAK inhibitor is a JAK1 inhibitor, a JAK2 inhibitor, or both.

Embodiment 142. The method of Embodiment 141, wherein the JAK inhibitor is selected from the group consisting of ruxolitinib, gandotinib, lestaurtinib, momelotinib, pacritinib, and fedratinib.

Embodiment 143. The method of any one of Embodiments 140-142, wherein the JAK inhibitor is ruxolitinib.

Embodiment 144. A method for reducing white blood cell count in a subject in need thereof, the method comprising administering an effective amount of a PIM kinase inhibitor of any one of Embodiments 100 and 112-118 and an effective amount of a JAK inhibitor.

Embodiment 145. The method of Embodiment 144, wherein the white blood cell count of the subject is elevated due to a myeloproliferative neoplasm.

Embodiment 146. The method of Embodiment 145, wherein the myeloproliferative neoplasm is as defined in any one of Embodiments 101-110.

Embodiment 147. The method of any one of Embodiments 146, wherein the JAK inhibitor is as defined in any one of Embodiments 141-143.

Embodiment 148. A pharmaceutical composition comprising a pharmaceutically acceptable carrier or excipient, a PIM kinase inhibitor and a JAK inhibitor.

Embodiment 149. The pharmaceutical composition of Embodiment 148, wherein the PIM kinase inhibitor is as defined in any one of Embodiments 100 and 112-118, and the JAK inhibitor is as defined in any one of Embodiments 141-143.

Embodiment 150. A kit comprising a PIM kinase inhibitor and written instructions for administering the PIM kinase inhibitor for treatment of a myeloproliferative neoplasm.

Embodiment 151. A kit comprising a PIM kinase inhibitor and written instructions for administering the PIM kinase inhibitor for treatment of fibrosis associated with cancer.

Embodiment 152. The kit of Embodiment 150 or 151, further comprising a JAK inhibitor and written instructions for administering the JAK inhibitor in combination with the PIM kinase inhibitor.

Embodiment 153. The kit of any one of Embodiments 150-152, wherein the PIM kinase inhibitor is Compound 1.

Embodiment 154. The kit of any one of Embodiments 150-153, wherein the JAK inhibitor is as defined in any one of Embodiments 141-143.

### FURTHER EXAMPLE EMBODIMENTS

Embodiment 201. A composition comprising: a polyglycolized glyceride; and Compound 1, or a pharmaceutically acceptable salt thereof.

Embodiment 202. The composition of Embodiment 201, wherein the polyglycolized glyceride has a melting point ranging from about 30 °C to about 50 °C.

Embodiment 203. The composition of Embodiment 202, wherein the polyglycolized glyceride has a melting point ranging from about 37 °C to about 48 °C.

Embodiment 204. The composition of Embodiment 203, wherein the polyglycolized glyceride has a melting point of about 44 °C.

Embodiment 205. The composition of any of Embodiments 201-204, wherein the polyglycolized glyceride has a hydrophile/lipophile balance (HLB) value ranging from about 8 to about 18.

Embodiment 206. The composition of Embodiment 205, wherein the polyglycolized glyceride has hydrophile/lipophile balance value ranging from about 10 to about 16.

Embodiment 207. The composition of Embodiment 206, wherein the polyglycolized glyceride has hydrophile/lipophile balance value of about 14.

Embodiment 208. The composition of any of Embodiments 201-207, wherein the composition further comprises a formulating agent, the formulating agent comprising polysorbate 20, polysorbate 60, polysorbate 80, glyceryl monocaprylate, glyceryl monocaprate, glyceryl monooleate, glyceryl dibehenate, propylene glycol dilaurate, propylene glycol monocaprylate, propylene glycol monolaurate, or combinations thereof.

Embodiment 209. The composition of Embodiment 208, wherein the formulating agent is polysorbate 20.

Embodiment 210. The composition of Embodiment 208, wherein the formulating agent is glyceryl monocaprylate.

Embodiment 211. The composition of any one of Embodiments 208-210, wherein the polyglycolized glyceride and formulating agent are present in a weight ratio ranging from 2:1 to 1:1.

Embodiment 212. The composition of any one of Embodiments 201-207, wherein the composition consists essentially of the compound and the polyglycolized glyceride.

Embodiment 213. The composition of any one of Embodiments 201-212, wherein the composition is a suspension.

Embodiment 214. The composition of any one of Embodiments 201-214, wherein the polyglycolized glyceride is Gelucire 44/14.

Embodiment 215. The composition of any one of Embodiments 201-214, comprising the hydrochloride salt of Compound 1.

Embodiment 216. The composition of any one of Embodiments 201-215, wherein the composition comprises from about 100 mg to about 300 mg of Compound 1, or a pharmaceutically acceptable salt thereof, as determined using the molecular weight of Compound 1 as the free base.

Embodiment 217. The composition of Embodiment 216, wherein the composition comprises from about 100 mg to about 150 mg of Compound 1, or a pharmaceutically acceptable salt thereof, as determined using the molecular weight of Compound 1 as the free base.

Embodiment 218. The composition of Embodiment 217, wherein the composition comprises from about 115 mg to about 125 mg of Compound 1, or a pharmaceutically acceptable salt thereof, as determined using the molecular weight of Compound 1 as the free base.

Embodiment 219. The composition of Embodiment 218, wherein the composition comprises about 120 mg of Compound 1, or a pharmaceutically acceptable salt thereof, as determined using the molecular weight of Compound 1 as the free base.

Embodiment 220. The composition of Embodiment 216, wherein the composition comprises from about 160 mg to about 200 mg of Compound 1, or a pharmaceutically acceptable salt thereof, as determined using the molecular weight of Compound 1 as the free base.

Embodiment 221. The composition of Embodiment 220, wherein the composition comprises from about 175 mg to about 185 mg of Compound 1, or a pharmaceutically acceptable salt thereof, as determined using the molecular weight of Compound 1 as the free base.

Embodiment 222. The composition of Embodiment 221, wherein the composition comprises about 180 mg of Compound 1, or a pharmaceutically acceptable salt thereof, as determined using the molecular weight of Compound 1 as the free base.

Embodiment 223. The composition of Embodiment 216, wherein the composition comprises from about 220 mg to about 260 mg of Compound 1, or a pharmaceutically acceptable salt thereof, as determined using the molecular weight of Compound 1 as the free base.

Embodiment 224. The composition of Embodiment 223, wherein the composition comprises from about 230 mg to about 250 mg of Compound 1, or a pharmaceutically acceptable salt thereof, as determined using the molecular weight of Compound 1 as the free base.

Embodiment 225. The composition of Embodiment 224, wherein the composition comprises about 240 mg of Compound 1, or a pharmaceutically acceptable salt thereof, as determined using the molecular weight of Compound 1 as the free base.

Embodiment 226. The composition of any one of Embodiments 201-225, wherein the composition comprises Compound 1, or a pharmaceutically acceptable salt thereof, in a concentration ranging from about 10 wt% to about 40 wt% as determined using the molecular weight of Compound 1 as a hydrochloride salt.

Embodiment 227. The composition of Embodiment 226, wherein the composition comprises Compound 1, or a pharmaceutically acceptable salt thereof, in a concentration ranging from about 14 wt% to about 22 wt% as determined using the molecular weight of Compound 1 as a hydrochloride salt.

Embodiment 228. The composition of Embodiment 227, wherein the composition comprises Compound 1, or a pharmaceutically acceptable salt thereof, in a concentration of about 18.38 wt% as determined using the molecular weight of Compound 1 as a hydrochloride salt.

Embodiment 229. The composition of Embodiment 226, wherein the composition comprises Compound 1, or a pharmaceutically acceptable salt thereof, in a concentration ranging from about 15 wt% to about 35 wt% as determined using the molecular weight of Compound 1 as a hydrochloride salt.

Embodiment 230. The composition of Embodiment 229, wherein the composition comprises Compound 1, or a pharmaceutically acceptable salt thereof, in a concentration ranging from about 20 wt% to about 30 wt% as determined using the molecular weight of Compound 1 as a hydrochloride salt.

Embodiment 231. The composition of Embodiment 230, wherein the composition comprises Compound 1, or a pharmaceutically acceptable salt thereof, in a concentration of about 25 wt% as determined using the molecular weight of Compound 1 as a hydrochloride salt.

Embodiment 232. The composition of any one of Embodiments 201-225, wherein the composition comprises Compound 1, or a pharmaceutically acceptable salt thereof, in a concentration ranging from about 23.3 wt% to about 43.3 wt% as determined using the molecular weight of Compound 1 as a hydrochloride salt.

Embodiment 233. The composition of Embodiment 232, wherein the composition comprises Compound 1, or a pharmaceutically acceptable salt thereof, in a concentration ranging from about 28.3 wt% to about 38.3 wt% as determined using the molecular weight of Compound 1 as a hydrochloride salt.

Embodiment 234. The composition of Embodiment 233, wherein the composition comprises Compound 1, or a pharmaceutically acceptable salt thereof, in a concentration of about 33.3 wt% as determined using the molecular weight of Compound 1 as a hydrochloride salt.

Embodiment 235. The composition of any one of Embodiments 201-234, wherein the composition comprises the polyglycolized glyceride in a concentration ranging from about 50 wt% to about 90 wt%.

Embodiment 236. The composition of any one of Embodiments 201-235, wherein the composition comprises the polyglycolized glyceride in a concentration ranging from about 75 wt% to about 90 wt%.

Embodiment 237. The composition of Embodiment 236, wherein the composition comprises the polyglycolized glyceride in a concentration ranging from about 78 wt% to about 84 wt%.

Embodiment 238. The composition of Embodiment 237, wherein the composition comprises the polyglycolized glyceride at a concentration of about 81.62 wt%.

Embodiment 239. The composition of Embodiments 201-235, wherein the composition comprises the polyglycolized glyceride in a concentration ranging from about 65 wt% to about 85 wt%.

Embodiment 240. The composition of Embodiment 239, wherein the composition comprises the polyglycolized glyceride in a concentration ranging from about 70 wt% to about 80 wt%.

Embodiment 241. The composition of Embodiment 240, wherein the composition comprises the polyglycolized glyceride at a concentration of about 75 wt%.

Embodiment 242. The composition of any one of Embodiments 201-235, wherein the composition comprises the polyglycolized glyceride in a concentration ranging from about 56.7 wt% to about 76.7 wt%.

Embodiment 243. The composition of Embodiment 242, wherein the composition comprises the polyglycolized glyceride in a concentration ranging from about 61.7 wt% to about 71.7 wt%.

Embodiment 244. The composition of Embodiment 243, wherein the composition comprises the polyglycolized glyceride at a concentration of about 66.7 wt%.

Embodiment 245. The composition of any one of Embodiments 201-244, wherein the composition is in the form of a capsule for oral administration.

Embodiment 246. The composition of any one of Embodiments 201-245, wherein the composition comprises Compound 1, or a pharmaceutically acceptable salt thereof, and the polyglycolized glyceride at a weight ratio ranging from about 1:1 to about 1:10 as determined using the molecular weight of Compound 1 as a free base.

Embodiment 247. The composition of any one of Embodiments 201-246, wherein the composition comprises Compound 1, or a pharmaceutically acceptable salt thereof, and the polyglycolized glyceride at a weight ratio ranging from about 1:4 to about 1:6 as determined using the molecular weight of the compound as a free base.

Embodiment 248. The composition of any one of Embodiments 201-247, wherein the composition comprises Compound 1, or a pharmaceutically acceptable salt thereof, and the polyglycolized glyceride at a weight ratio of about 1:5 as determined using the molecular weight of Compound 1 as a free base.

Embodiment 249. The composition of any one of Embodiments 201-246, wherein the composition comprises the Compound 1, or a pharmaceutically acceptable salt thereof, and the polyglycolized glyceride at a weight ratio ranging from about 1:1.6 to about 1:3.6 as determined using the molecular weight of Compound 1 as a free base.

Embodiment 250. The composition of Embodiment 249, wherein the composition comprises Compound 1, or a pharmaceutically acceptable salt thereof, and the polyglycolized glyceride at a weight ratio ranging from about 1:2.1 to about 1:3.1 as determined using the molecular weight of Compound 1 as a free base.

Embodiment 251. The composition of Embodiment 250, wherein the composition comprises Compound 1, or a pharmaceutically acceptable salt thereof, and the polyglycolized glyceride at a weight ratio of about 1:2.6 as determined using the molecular weight of Compound 1 as a free base.

Embodiment 252. The composition of any one of Embodiments 201-246, wherein the composition comprises Compound 1, or a pharmaceutically acceptable salt thereof, and the polyglycolized glyceride at a weight ratio ranging from about 1:1 to about 1:2.5 as determined using the molecular weight of Compound 1 as a free base.

Embodiment 253. The composition of Embodiment 252, wherein the composition comprises Compound 1, or a pharmaceutically acceptable salt thereof, and the polyglycolized glyceride at a weight ratio ranging from about 1:1.25 to about 1:2 as determined using the molecular weight of Compound 1 as a free base.

Embodiment 254. The composition of Embodiment 253, wherein the composition comprises Compound 1, or a pharmaceutically acceptable salt thereof, and the polyglycolized glyceride at a weight ratio of about 1: 1.76 as determined using the molecular weight of Compound 1 as a free base.

### EXAMPLES

As detailed in the Examples below, Applicant has discovered that PIM1 expression is significantly increased in mouse and human MPN/MF hematopoietic progenitor cells. Applicant has also discovered that PIM1 knockdown markedly inhibited proliferation in JAK2V617F-expressing cells but does not inhibit proliferation of wild-type JAK2-expressing cells.

These Examples provide details relating to the efficacy of an exemplary second-generation pan-PIM kinase inhibitor (i.e., Compound 1) in hematopoietic cells expressing JAK2 V617F and in a murine model of MF. Applicants have discovered that treatment using Compound 1 (0.25-1.0 µM) significantly reduces proliferation of murine Ba/F3-EpoR cells expressing JAK2V617F. Treatment with Compound 1 (0.5-1.0 µM) also significantly inhibited human JAK2 V617F-positive HEL and UKE-1 cells. Treatment also resulted in significant apoptosis in Ba/F3-EpoR-JAK2 V617F, HEL and UKE-1 cells but not in wild-type JAK2 expressing Ba/F3-EpoR cells.

Additionally, combination treatment with a PIM kinase inhibitor and a JAK2 inhibitor (e.g., Compound 1 and ruxolitinib) synergistically induces apoptosis in JAK2 V617F-expressing hematopoietic cells. Furthermore, Compound 1 significantly inhibits MPN/MF CD34+ hematopoietic progenitor colony growth as demonstrated using knock-in mice for the generation of JAK2 V617F expressing cells.

Specifically, heterozygous JAK2 V617F knock-in mice exhibit a polycythemia vera (PV) phenotype and mice expressing homozygous JAK2 V617F rapidly develop high-grade MF. Accordingly, these Examples describe testing that utilizes the homozygous JAK2 V617F mice to elucidate the in vivo efficacy of a PIM kinase inhibitor (Compound 1) alone or in combination with a JAK2 inhibitor (ruxolitinib) against MF.

Treatment using Compound 1 alone significantly reduces the increase in white blood cell (WBC) and neutrophil counts as well as spleen size in mice expressing homozygous JAK2 V617F compared with vehicle treatment. Combined treatment of Compound 1 and ruxolitinib almost completely normalized the WBC and neutrophil counts and the spleen size in homozygous JAK2 V617F mice (Example 9).

Histopathologic analysis revealed reduction in fibrosis in the bone marrow and spleens of mice treated with Compound 1 but treatment with ruxolitinib did not significantly reduce fibrosis. A combination of Compound 1 and ruxolitinib almost completely eliminated fibrosis in the bone marrow and spleens of homozygous JAK2 V617F mice.

Treatment with Compound 1 was well tolerated and did not cause any significant toxicity in wild type mice. RNA-sequencing analysis on purified LSK (Lin-Sca-1+c-kit+) cells from these drug-treated mice revealed that the genes related to TNFα and WNT signaling pathways were significantly downregulated when treated with Compound 1 alone or in combination with ruxolitinib compared with the control (Example 12).

### EXAMPLE 1

### COMPOUND 1 SHOWS ACTIVITY AGAINST MULTIPLE CELL TYPES AND LINES

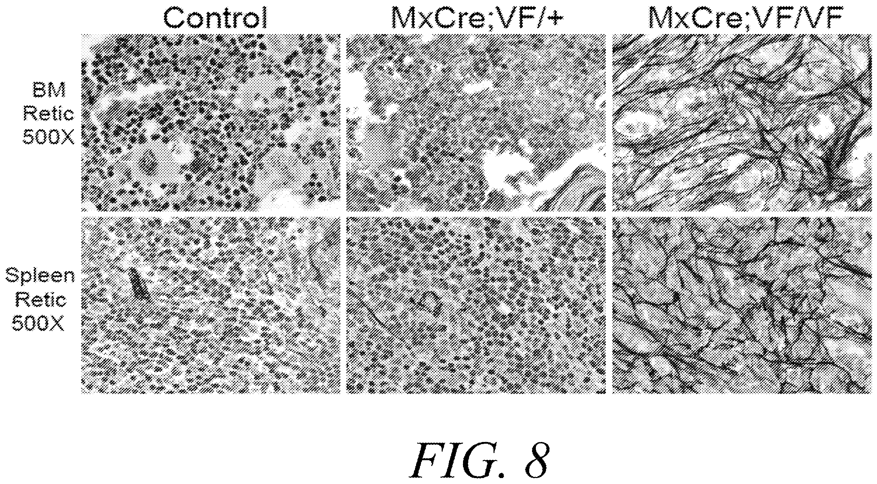

To determine the role of JAK2 V617F in the pathogenesis of MPNs, an inducible JAK2 V617F knock-in mouse was generated. Heterozygous JAK2 V617F knock-in mice exhibit all the features of human PV disease, including increase in red blood cells, hemoglobin and hematocrit, leukocytosis, thrombocytosis and splenomegaly (see Akada et al., Blood 2010). Homozygous JAK2 V617F knock-in mice, which rapidly develop high-grade MF (Grade 3 within 10-12 weeks after induction; FIG. 8) with 100% penetrance were also generated. These novel animal models allowed testing of the in vivo efficacy of Compound 1 or Compound 1/ruxolitinib drug combinations against MPN/MF.

Reticulin staining on the bone marrow and spleen sections from homozygous JAK2 V617F mice (MxCre;VF/VF) show high grade (Grade 3) myelofibrosis within 10 weeks after induction with pI-pC. BM and spleen sections from control or heterozygous JAK2 V617F mice (MxCre;VF/+) did not exhibit myelofibrosis at that time (FIG. 8).

### EXAMPLE 2

### MICROARRAY ANALYSIS OF PIM1 MRNA EXPRESSION

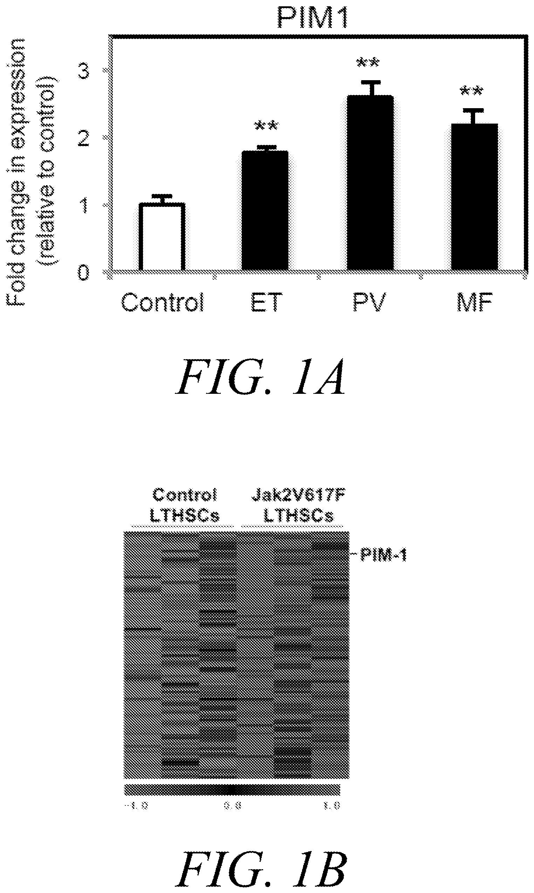

PIM1 mRNA expression is upregulated in hematopoietic progenitors of MPN patients. Analysis of published gene expression data on MPN patients (database series: GSE54646) revealed that PIM1 expression is significantly increased in MPN (i.e., PV, ET and MF) granulocytes compared with healthy control granulocytes. Microarray data analyzed from the Gene Expression Omnibus Database-series number: GSE54646. Note that expression of PIM1 is significantly increased in MPN (PV, ET and MF) granulocytes compared with healthy control granulocytes (in FIG. 1A, ** indicates p<0.005).

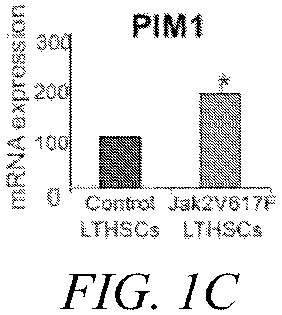

It was found that PIM1 is significantly upregulated in hematopoietic progenitors of JAK2 V617F knock-in mice and patients with MF. The presence of PIM1 is evidenced by comparing long-term hematopoietic stem cells with and without JAK2 V617F (FIG. 1B). The level of mRNA expression for JAK2 V617F is also increased compared to a control sample (FIG. 1C).

### EXAMPLE 3

### IMMUNOASSAY ANALYSIS OF PIM1 PROTEIN EXPRESSION

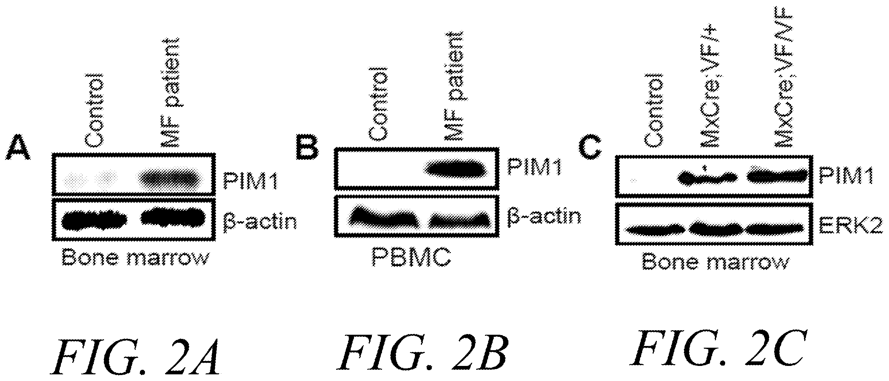

PIM1 protein expression in human and mouse MPN hematopoietic cells was assessed by immunoblotting. Significantly increased levels of PIM1 protein was observed in the bone marrow and peripheral blood mononuclear cells (PBMC) of MF patents and bone marrow of heterozygous (MxCre;VF/+) and homozygous (MxCre;VF/VF) JAK2 V617F knock-in mice compared to controls (FIGs. 2A-C).

### EXAMPLE 4

### PIM1 KNOCKDOWN ON HEMATOPOIETIC CELLS EXPRESSING JAK2 V617F

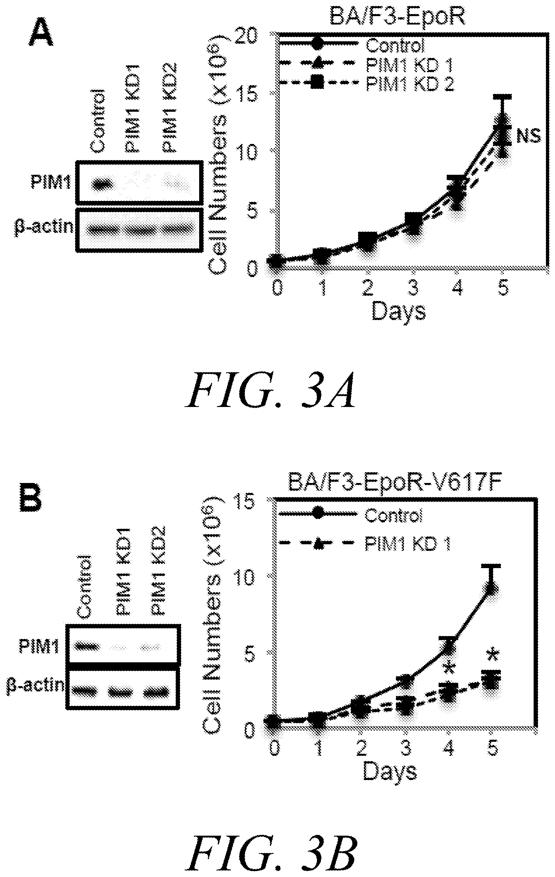

Murine BA/F3-EpoR cells expressing wild type JAK2 or JAK 2V617F (BA/F3-EpoR-JAK2 V617F) and human JAK2 V617F-positive leukemia cells (HEL) were transduced with lentiviral PIM1 shRNA or control shRNA. Infected cells were selected with puromycin. Knockdown of PIM1 significantly inhibited proliferation of BA/F3-EpoR-JAK2 V617F and HEL cells expressing JAK2 V617F but not wild-type JAK2 expressing BA/F3-EpoR cells (FIGs. 3A-C). These data show that PIM1 plays an important role in survival/proliferation of MPN cells expressing JAK2 V617F.

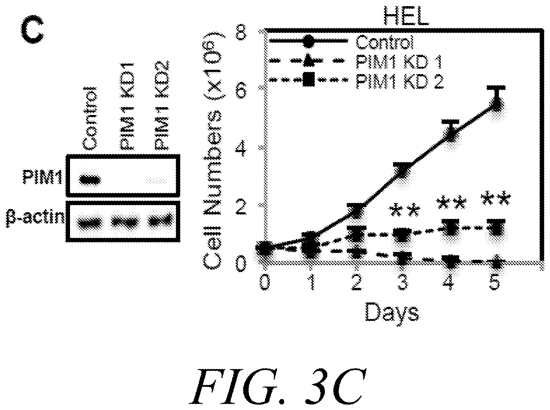

Knockdown of PIM1 inhibits proliferation of hematopoietic cells expressing JAK2 V617F but not wild-type JAK2 expressing cells. The data in FIG. 3A was obtained from BA/F3-EpoR cells expressing wild type JAK2. The data in FIG. 3B was obtained from BA/F3-EpoR cells expressing JAK2 V617F compared to a control sample. The data in FIG. 3C shows HEL cells expressing JAK2 V617F compared to a control sample. Expression was obtained by transducing cells with lentiviral scramble shRNA (control) or PIM1 shRNAs (KD1 and KD2) and selected using puromycin.

Cell proliferation was determined by viable cell counts every 24 hours for 5 days. Knockdown of PIM1 significantly inhibited proliferation of BA/F3-EpoR-JAK2 V617F and HEL cells expressing JAK2 V617F but not BA/F3-EpoR cells expressing wild type JAK2 (in FIGs. 3A-C, * indicates p<0.05 and ** indicates p<0.005).

### EXAMPLE 5

### SELECTIVE INHIBITION OF JAK2 V617F

The effects of Compound 1 on proliferation of murine BA/F3 cells expressing wild type JAK2 or BA/F3-EpoR-JAK2 V617F cells expressing JAK2 V617F as well as in human JAK2 V617F-positive leukemia cells HEL and UKE-1 were assessed.

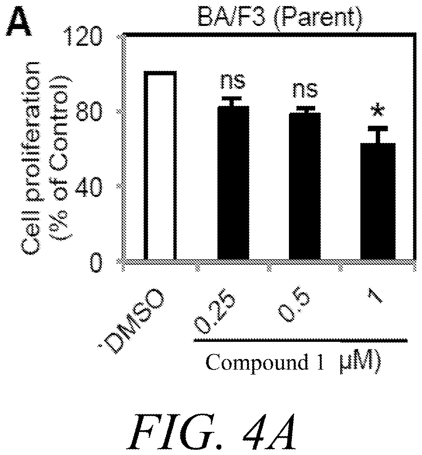

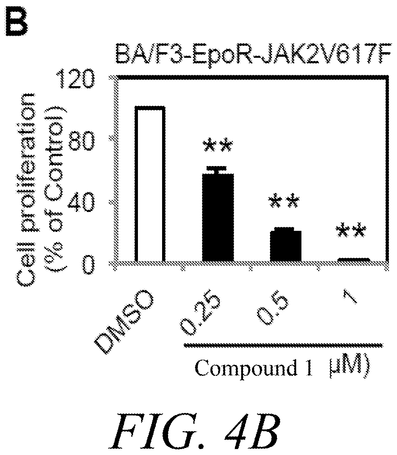

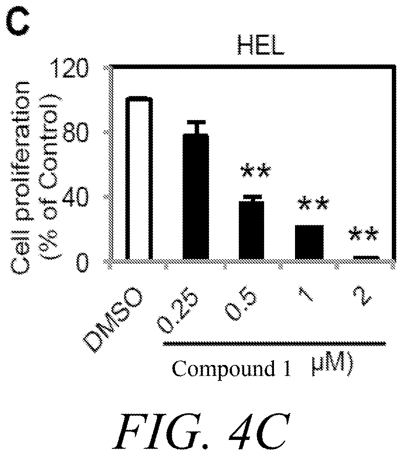

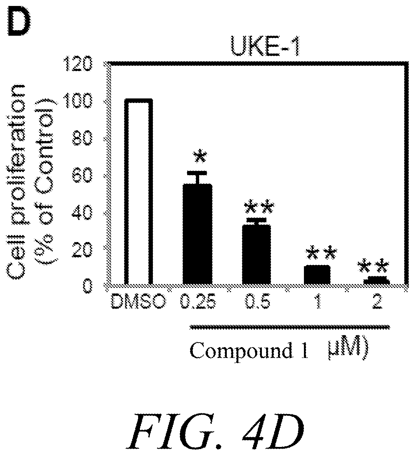

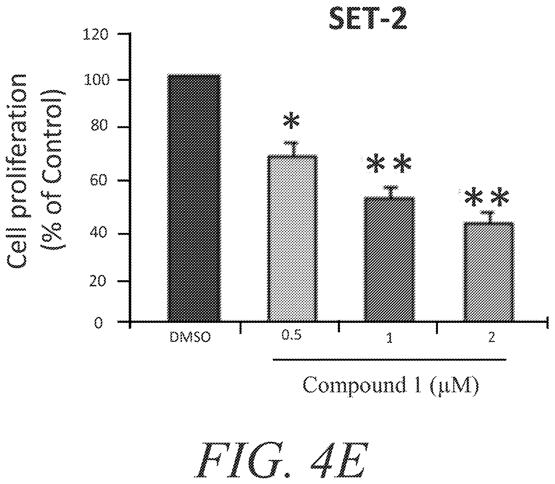

Treatment using Compound 1 over the concentration range of 0.5-1 µM significantly reduced (~90%) proliferation of BA/F3-EpoR-JAK2 V617F cells whereas wild-type JAK2-expressing BA/F3 cells was only modestly inhibited by Compound 1 at higher concentrations (FIGs. 4A, B). Treatment using Compound 1 also markedly inhibited proliferation of human JAK2 V617F-positive HEL and UKE-1 cells (FIGs. 4C, D). Treatment using Compound 1 showed dose dependent inhibition of proliferation for SET-2 cells (FIG. 4E)

Cells were treated with varying concentrations of Compound 1 for 5 days and cell proliferation was assessed by viable cell counts. Compound 1 in the concentration range of 0.25-1 µM or 0.5 - 2 µM markedly inhibited proliferation of cells expressing JAK2 V617F but only minimally inhibited wild-type JAK2-expressing cells at a higher concentration (in FIGs. 4A-E, * indicates p<0.05; ** indicates p<0.005; and "ns" indicates a difference that is not statistically significant).

### EXAMPLE 6

### COMPOUND 1 TREATMENT OF CD34+ HEMATOPOIETIC PROGENITOR CELLS

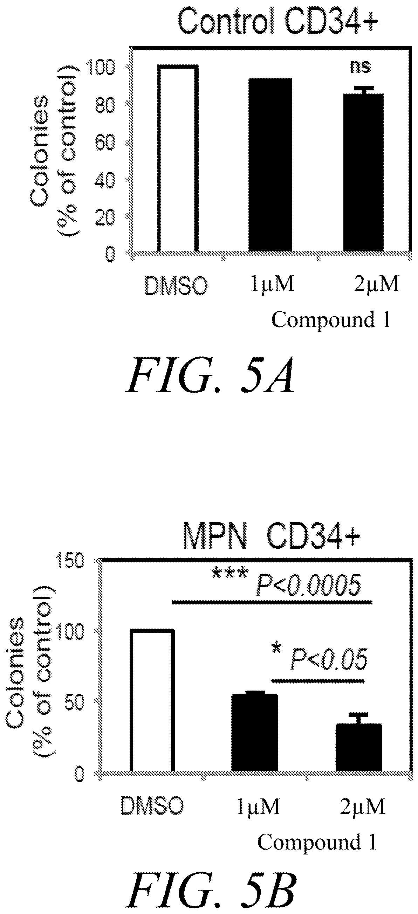

The effects of Compound 1 were assessed on MPN patient CD34+ hematopoietic progenitor cells. It was observed that treatment using Compound 1 significantly inhibited hematopoietic progenitor colonies in MPN patient CD34+ cells but showed minimal effect on healthy control CD34+ cells (in FIGs. 5A-B * indicates p<0.05; *** indicates p<0.0005; and "ns" indicates a difference that is not statistically significant).

CD34+ cells isolated from the peripheral blood of healthy control and MPN patients (n = 4) and plated in methylcellulose medium containing cytokines in the presence of DMSO or Compound 1 (1-2 µM). Hematopoietic progenitor colonies were counted after 14 days. Treatment using Compound 1 at a concentration of 1-2 µM significantly inhibited hematopoietic progenitor colonies in MPN CD34+ cells (FIG. 5B) but not in healthy control CD34+ cells (FIG. 5A).

### EXAMPLE 7

### SYNERGISM OF COMPOUND 1 WITH RUXOLITINIB

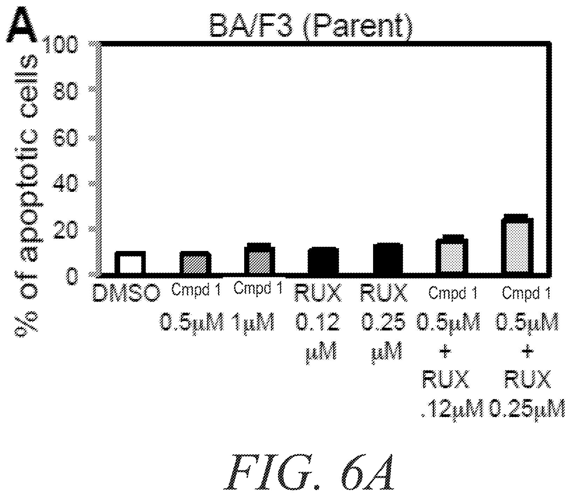

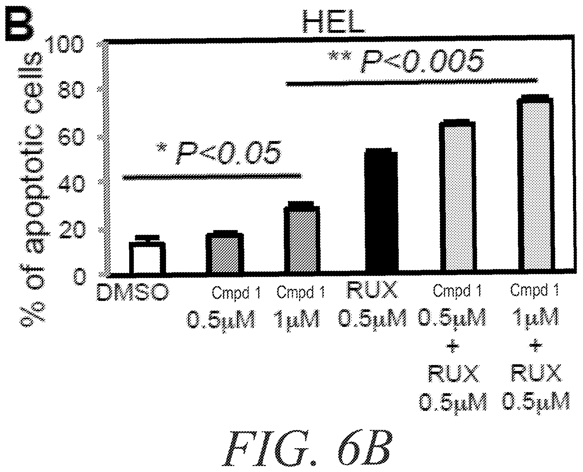

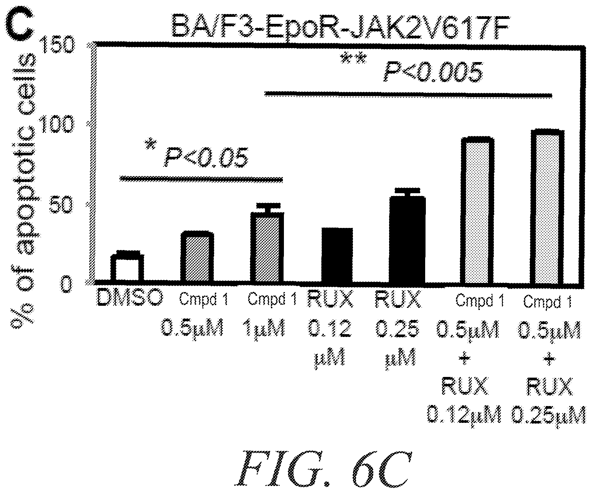

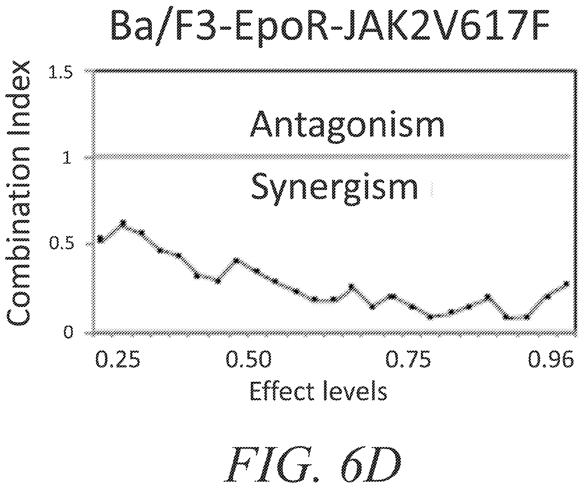

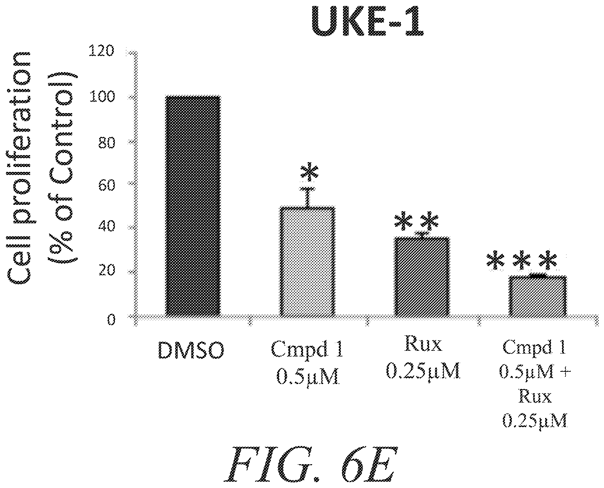

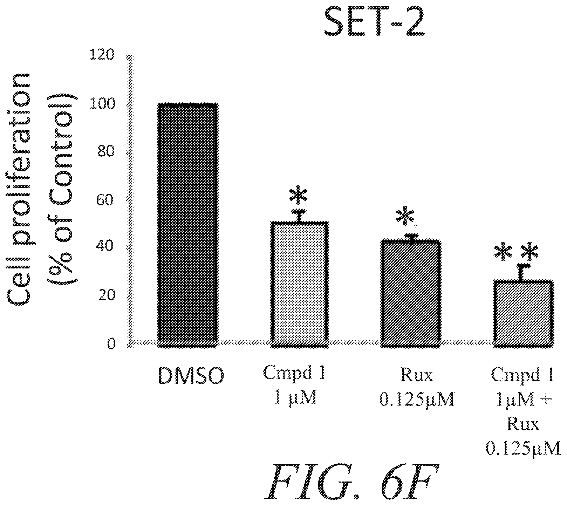

BA/F3 cells expressing wild type JAK2, as well as HEL cells, BA/F3-EpoR-JAK2 V617F cells, UKE-1 cells and SET-2 cells expressing JAK2 V617F were treated with Compound 1 alone or in combination with ruxolitinib at various concentrations as indicated in FIGs. 6A-C and 6E-F. Apoptosis was determined 48 hours after treatment using Annexin V staining followed by flow cytometry. Compound 1 alone or in combination with ruxolitinib at the indicated concentrations exhibited significant apoptosis in each cell line tested but not in wild type JAK2-expressing BA/F3 cells (FIGs. 6A-C and 6E-F). A combination of Compound 1 and ruxolitinib synergistically induced apoptosis in BA/F3-EpoR-JAK2 V617F cells (FIG. 6D) with combination index values less than 0.3 indicates strong synergy.

### EXAMPLE 8

### COMPOUND 1 OVERCOMES RESISTANCE TO JAK2 INHIBITION

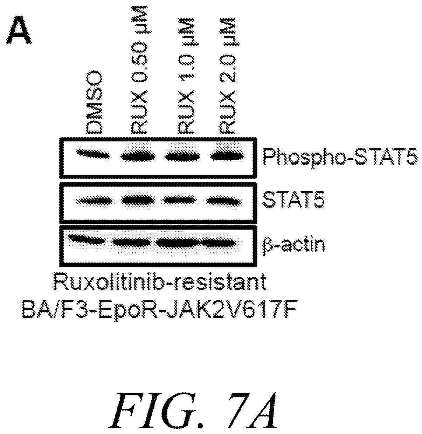

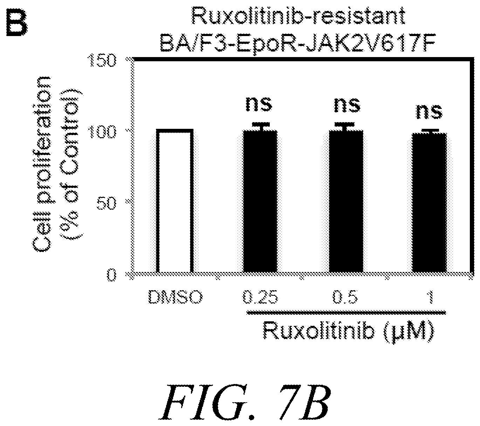

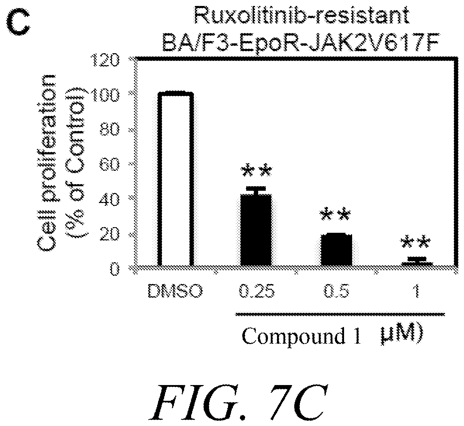

It was assessed whether treatment using Compound 1 would overcome resistance to JAK2 inhibition in cells expressing JAK2 V617F. To generate JAK2 inhibitor-resistant cells, BA/F3-EpoR-JAK2 V617F cells were cultured in the presence of increasing concentrations of ruxolitinib (up to 2 µM) for over 3 months. Unexpectedly, significant increases in the expression of PIM1 was observed in ruxolitinib-resistant BA/F3-EpoRJAK2 V617F cells compared to BA/F3-EpoR-JAK2 V617F cells that were sensitive to ruxolitinib. Phosphorylation of STAT5 was not inhibited by ruxolitinib treatment (0.5-2 µM) in ruxolitinib-resistant BA/F3-EpoR-JAK2 V617F cells (FIG. 7A). Similarly, cell proliferation was not significantly inhibited by ruxolitinib treatment (0.5-2 µM) in ruxolitinib-resistant BA/F3-EpoRJAK2 V617F cells (FIG. 7B). In contrast, treatment using Compound 1 markedly inhibited the proliferation of ruxolitinib-resistant BA/F3-EpoR-JAK2 V617F cells (FIG. 7C), suggesting that Compound 1 treatment can overcome the resistance to JAK2 inhibition in MPN cells, an important finding that has significant clinical implication.

Immunoblot showed no inhibition of STAT5 phosphorylation upon ruxolitinib treatment (FIG. 7A), indicating resistance to ruxolitinib treatment. Ruxolitinib-resistant BA/F3-EpoR-JAK2 V617F cells were treated with varying concentrations of ruxolitinib or Compound 1 (FIGs. 7B-C) for 5 days and cell proliferation was assessed by viable cell counts. Ruxolitinib treatment did not cause inhibition of ruxolitinib-resistant BA/F3-EpoR-JAK2 V617F cells but Compound 1 (0.25-1 µM) markedly inhibited proliferation of these cells (in FIGs. 7A-C, * indicates p<0.05; ** indicates p<0.005; "ns" indicates a difference that is not statistically significant).

### EXAMPLE 9

### BLOOD CELL COUNT IMPROVEMENT FROM TREATMENT USING COMPOUND 1

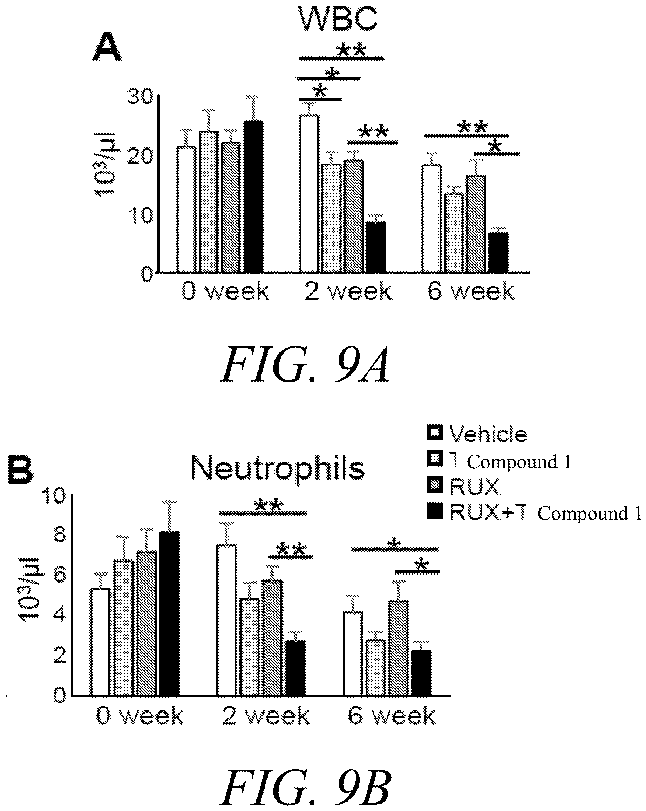

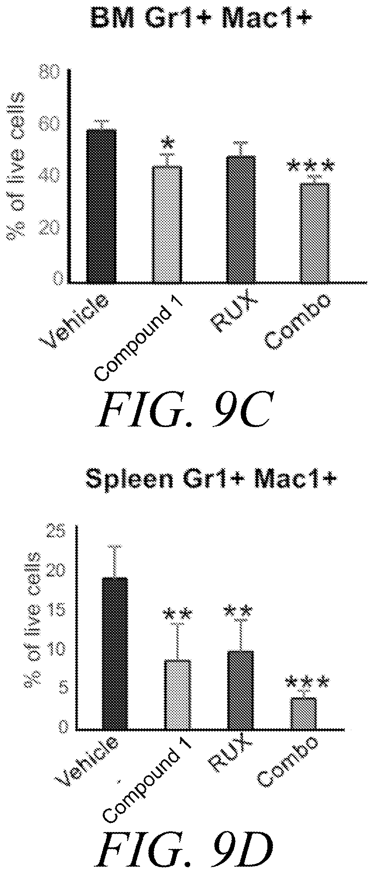

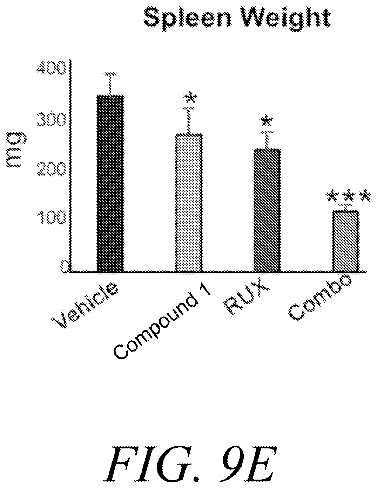

The efficacy of Compound 1 in combination with ruxolitinib was tested using our homozygous JAK2 V617F knock-in mouse model of MF (see Example 1). Bone marrow cells from homozygous JAK2 V617F knock-in mice were transplanted into lethally irradiated C57BL/6 recipients to obtain a cohort of MF mice with similar age. Eight weeks after transplantation, peripheral blood counts were measured and the mice were then randomized to receive treatment with vehicle, Compound 1 (150 mg/kg), ruxolitinib (60 mg/kg) or Compound 1 (150 mg/kg) plus ruxolitinib (60 mg/kg) by oral gavage once daily. All mice were treated for 6 weeks. Peripheral blood white blood cells (WBC) and neutrophil counts were reduced to almost normal levels upon combined treatment of Compound 1 and ruxolitinib (FIGs. 9A-B). Treatment with Compound 1 alone caused reduction in WBC and neutrophil counts in the blood.

In all, treatment with Compound 1 in combination with ruxolitinib reduces peripheral blood WBC and neutrophil counts, spleen size and eliminates fibrosis in the JAK2 V617F mouse model of myeloproliferative neoplasm/myelofibrosis; treatment with Compound 1 alone reduces WBC and neutrophil counts, spleen size and inhibits fibrosis in JAK2 V617F mice (in FIGs. 9A-E, * indicates p<0.05; ** indicates p<0.005).

### EXAMPLE 10

### FIBROSIS REDUCTION USING COMPOUND 1

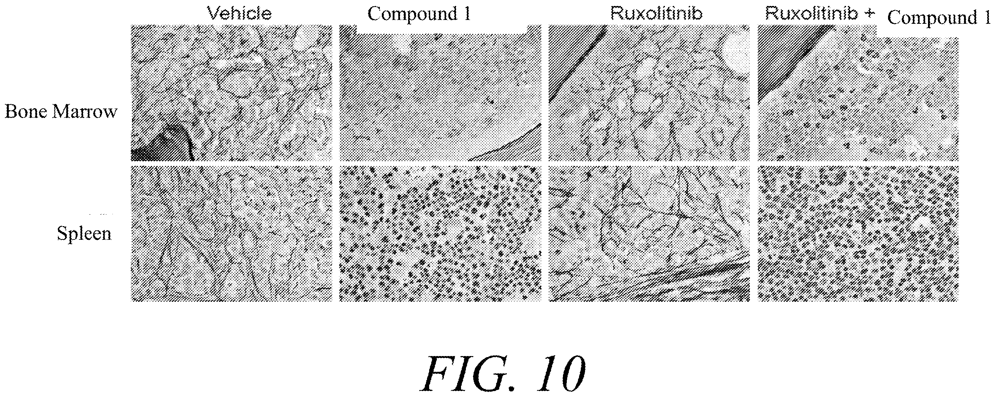

Homozygous JAK2 V617F mice exhibiting MF were treated with vehicle, Compound 1 alone (150 mg/kg), ruxolitinib alone (60 mg/kg) or Compound 1 (150 mg/kg) in combination with ruxolitinib (60 mg/kg) for 6 weeks. Reticulin staining show marked reduction of fibrosis when mice were treated with Compound 1 alone. Combined treatment with Compound 1 and ruxolitinib effectively eliminated fibrosis in the bone marrow (BM) and spleens (SPL) of homozygous JAK2 V617F mice (FIG. 10).

In all, retic staining of bone marrow and spleen sections show marked reduction in fibrosis in Compound 1-treated mice whereas representative JAK2 inhibitor ruxolitinib treatment did not cause any significant reduction in fibrosis. These data suggest that PIM kinase inhibitor Compound 1 in combination with JAK2 inhibitor ruxolitinib may have therapeutic efficacy against MF.

### EXAMPLE 11

### DOSING CALCULATIONS - TOXICITY STUDY

A daily dosing range for Compound 1 was calculated to be in the equivalent range of 10-10,000 mg in humans for the treatment of myelofibrosis. Dose calculations were determined from GLP toxicology studies in rats and non-GLP toxicology studies in dogs. In the rat, the severely toxic dose in 10 percent of animals was observed at 500 mg/kg, which equates to 5286 mg average per person, calculated as:
500 mg/kg in rats/7 human eq. dose factor = 71.4 mg/kg in humans
71.4 mg/kg × 37 human Km factor = 2643 mg/m2 in humans
2643 mg/m¬2 in humans × 2 avg. m2 per person = 5286 mg/average human

In dogs, the highest non-severely toxic dose was not observed in 7-day repeat studies at doses as high as 200 mg/kg, which equates to 7400 mg per person or greater, calculated as:
200 mg/kg in dogs/2 human eq. dose factor = 100 mg/kg in humans
100 mg/kg × 37 human Km factor = 3700 mg/m2 in humans
3700 mg/m¬2 in humans x 2 avg. m2 per person= 7400 mg/average human

The efficacious dose level obtained in the mouse myelofibrosis model (150 mg/kg), would equate to 925 mg per person calculated as:
150 mg/kg in mice/12 human eq. dose factor = 12.5 mg/kg in humans
12.5 mg/kg × 37 human Km factor = 462.5 mg/m2 in humans
462.5 mg/m¬2 in humans × 2 avg. m2 per person = 925 mg/average human

A dose range of 10-10,000 mg accounts for higher tolerance in humans compared to dogs or rats, as well as lower efficacious dose levels compared mice due to possible unknown differences in absorption and clearance between species. Additionally, formulation changes could enhance absorption and result in a lower required efficacious dose for humans than what was observed in mice.

### EXAMPLE 12

### DOWNREGULATION OF TNFA AND WNT SIGNALING PATHWAYS

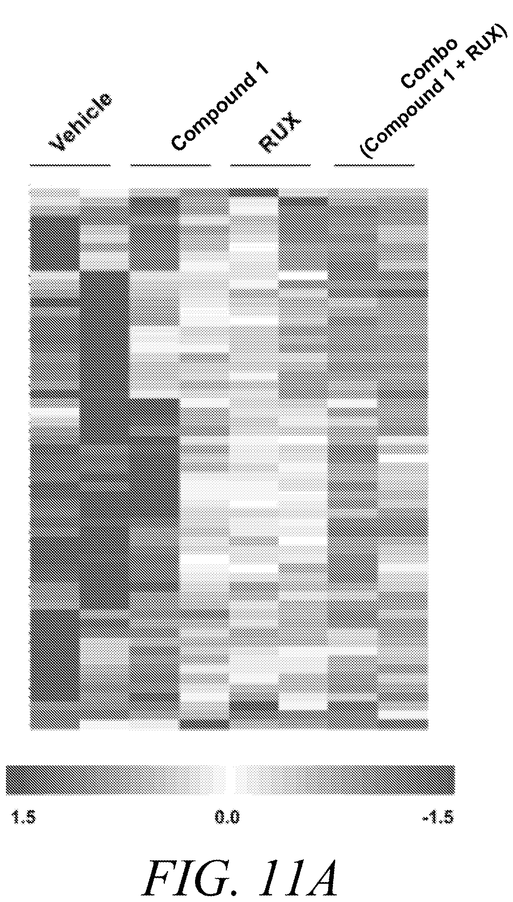

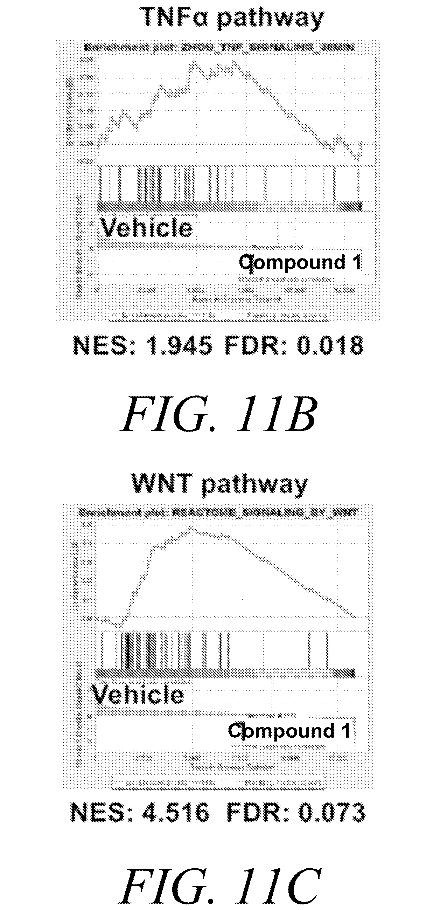

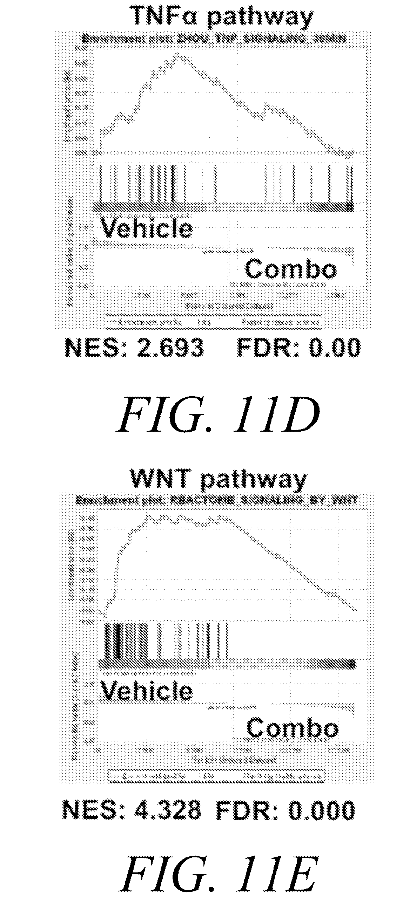

Purified LSK (Lin-·Sca-1+c-kit+) cells from homozygous JAK2 V617F mice treated with vehicle alone, Compound 1 alone, ruxolitinib alone or a combination of Compound 1 and ruxolitinib were analyzed using RNA-sequencing. The RNA-sequence analysis showed that the genes relating to hematopoietic stem cell (HSC) maintenance, TNFα and WNT signaling pathways were significantly downregulated for the samples treated with Compound 1 alone or Compound 1 in combination with ruxolitinib when compared with the vehicle (FIGs. 11A-E).

### EXAMPLE 13

### COMPOUND 1 TREATMENT OF MPL W515L CELLS

The effects of Compound 1 on proliferation of murine BA/F3 cells expressing wild type MPL or BA/F3 expressing MPL W515L were assessed.

Treatment using Compound 1 over the concentration range of 0.25-1 µM significantly reduced proliferation of BA/F3 MPL W515L cells whereas the parental BA/F3 cells was greater than 1 µM (FIG. 4A). Additionally, the in vivo efficacy of Compound 1 and Compound 1/ruxolitinib combination was tested using the transplantation mouse model where MPL mutant (MPL W515L) bone marrow cells were injected into irradiated mice (as described in PMID: 16834459). These animals rapidly develop marked thrombocytosis and leukocytosis. At the time of sacrifice, MPL W515L expressing mice show splenomegaly and bone marrow fibrosis. A similar decrease in white blood cell and neutrophil counts in MPL mutant mice upon treatment with Compound 1 alone or in combination with ruxolitinib was observed. A significant decrease in splenomegaly in MPL mutant mice treated with Compound 1 alone and in combination with ruxolitinib was observed. Combined treatment of Compound 1 plus ruxolitinib demonstrated a synergetic response, almost completely ablating the fibrosis in the bone marrow and spleens of MPL mutant mice.

The above Examples show that expression of PIM1 is significantly increased in hematopoietic progenitors of mouse and human MPN. Compound 1 alone or in combination with ruxolitinib significantly inhibits proliferation and induces apoptosis in JAK2 V617F expressing cells. Treatment with Compound 1 significantly reduces WBC and neutrophil counts, spleen size and inhibits fibrosis in JAK2 V617F mice and combination of ruxolitinib with Compound 1 further reduces WBC and neutrophil counts, spleen size and eliminates fibrosis in JAK2 V617F mouse model of MPN/MF. Genes related to TNFα and WNT signaling pathways are downregulated in JAK2 V617F hematopoietic progenitors upon combination treatment using Compound 1 and ruxolitinib.

Overall, Examples 1-13 suggest treatment using a PIM1 inhibitor alone or in combination with a JAK inhibitor is effective as therapy for myeloproliferative neoplasms.

### EXAMPLE 14

### COMPOUND 1 - BIOCHEMICAL PROFILE

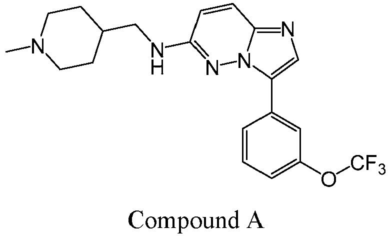

Compound 1 has the following biochemical profile compared to another known PIM kinase inhibitor, Compound A:

**Table 1: Pharmacokinetic profile of Compound 1 compared to Compound A**

| | Kᵢ (nM) | | | IC₅₀ (nM) | hERG IC₅₀ (µM) |
|---|---|---|---|---|---|
| | PIM 1 | PIM 2 | PIM 3 | Flt3 | Patch Clamp |
| Compound A | 12 | 980 | 20 | 3 | < 1 |
| Compound 1 | 5 | 239 | 42 | 279 | > 30 |

As the data in Table 1 show, Compound 1 is relatively selective for PIM-1, has improved selectivity vs. FLT-3 and improved metabolic stability. Additionally, Compound 1 does not show activity as a hERG inhibitor.

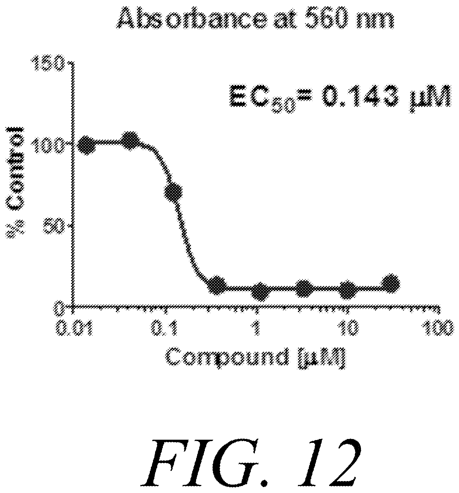

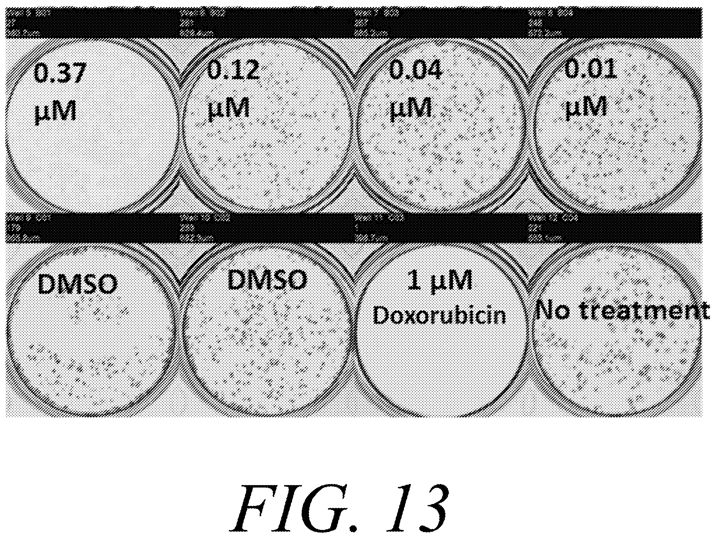

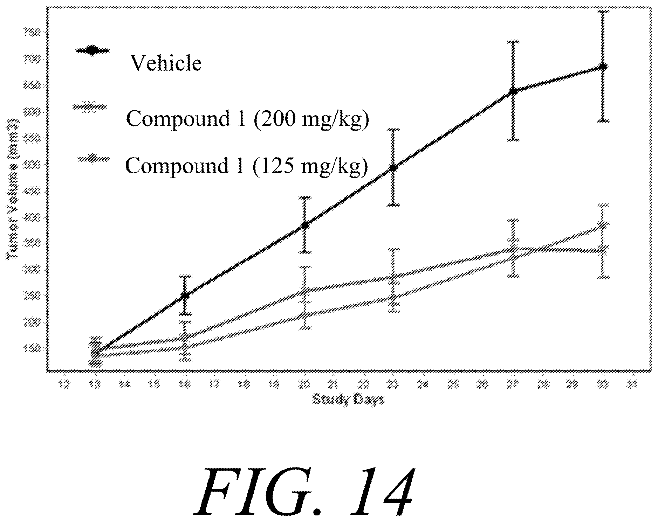

Compound 1 inhibits colony formation of prostate adenocarcinoma. PC3 cells were seeded in Roswell Park Memorial Institute (RPMI) media with pyruvate and 10% fetal bovine serum (FBS). Cells were treated in RPMI, pyruvate and 0.5% FBS and 0.37 µM, 0.12 µM, 0.04 µM, and 0.01 µM doses of Compound 1 compared to DMSO, 1 µM, and no treatment controls (see FIG. 13). The EC₅₀ for Compound 1 against PC3 cells was calculated to be 0.143 µM (see FIG. 12). Compound 1 also shows efficacy in vivo against prostate adenocarcinoma (see FIG. 14) in xenograft models (PC3 prostate adenocarcinoma mouse xenograft models). The percentage of tumor growth inhibition (% TGI) was determined as 40 and 59 for the 200 mg/kg and 125 mg/kg doses, respectively.

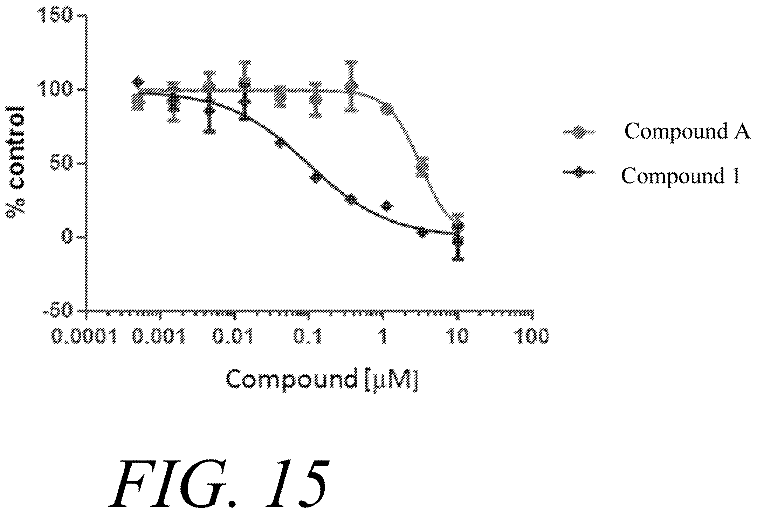

Additionally, Compound 1 is more potent at reducing phospho-BAD (an internally validated PIM-1 biomarker used as a pharmacodynamic biomarker for Compound A). The results of this assay are illustrated as a plot of the % of the control against the compound concentration (FIG. 15).

All of the U.S. patents, U.S. patent application publications, U.S. patent applications, foreign patents, foreign patent applications and non-patent publications referred to in this specification are incorporated herein by reference, in their entirety to the extent not inconsistent with the present description.

From the foregoing it will be appreciated that, although specific embodiments of the disclosure have been described herein for purposes of illustration, various modifications may be made without deviating from the spirit and scope of the disclosure.

### EMBODIMENTS

1. A method for treating a myeloproliferative neoplasm in a mammal in need thereof, the method comprising administering to the mammal:
   from about 250 mg to about 2.5 g per day of a compound represented by the following structural formula: 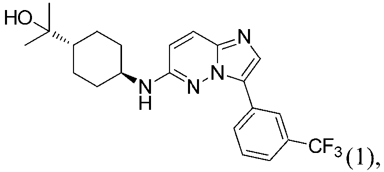 or a pharmaceutically acceptable salt thereof; and
   an effective amount of ruxolitinib, or a pharmaceutically acceptable salt thereof.
2. The method of embodiment 1, comprising administering to the mammal from about 300 mg to about 1.5 g per day of the compound of structural formula 1, or a pharmaceutically acceptable salt thereof.
3. The method of embodiment 1, comprising administering to the mammal from about 450 mg to about 1.5 g per day of the compound of structural formula 1, or a pharmaceutically acceptable salt thereof.
4. The method of any one of embodiments 1-3, wherein the myeloproliferative neoplasm is myelofibrosis.
5. The method of embodiment 4, wherein the myelofibrosis is intermediate-risk myelofibrosis or high-risk myelofibrosis.
6. The method of any one of embodiments 1-5, wherein the myelofibrosis is primary myelofibrosis.
7. The method of any one of embodiments 1-5, wherein the myelofibrosis is secondary myelofibrosis.
8. The method of any one of embodiments 1-7, wherein treating the myeloproliferative neoplasm results in the mammal being measurable residual disease (MRD)-negative.
9. The method of any one of embodiments 1-8, wherein treating the myeloproliferative neoplasm results in complete remission in the mammal.
10. The method of any one of embodiments 1-9, wherein the compound of structural formula 1, or a pharmaceutically acceptable salt thereof, is administered orally.
11. The method of any one of embodiments 1-10, wherein the compound of structural formula 1, or a pharmaceutically acceptable salt thereof, is administered once daily.
12. The method of any one of embodiments 1-10, wherein the compound of structural formula 1, or a pharmaceutically acceptable salt thereof, is administered twice daily.
13. The method of any one of embodiments 1-12, wherein the compound of structural formula 1, or a pharmaceutically acceptable salt thereof, is administered for from about seven days to about one year.
14. The method of embodiment 13, wherein the compound of structural formula 1, or a pharmaceutically acceptable salt thereof, is administered for 28 days.
15. The method of embodiment 13, wherein the compound of structural formula 1, or a pharmaceutically acceptable salt thereof, is administered for one year.
16. The method of any one of embodiments 1-15, wherein the effective amount of ruxolitinib, or a pharmaceutically acceptable salt thereof, is from about 5 mg/day to about 100 mg/day.
17. The method of embodiment 16, wherein the effective amount of ruxolitinib, or a pharmaceutically acceptable salt thereof, is from about 10 mg/day to about 50 mg/day.
18. The method of any one of embodiments 1-17, wherein the ruxolitinib, or a pharmaceutically acceptable salt thereof, is administered orally.
19. The method of any one of embodiments 1-18, wherein the ruxolitinib, or a pharmaceutically acceptable salt thereof, is administered twice daily.
20. The method of any one of embodiments 1-19, wherein the ruxolitinib, or a pharmaceutically acceptable salt thereof, is administered for from about seven days to about one year.
21. The method of any one of embodiments 1-20, wherein the myeloproliferative neoplasm has been previously treated with ruxolitinib in the absence of the compound of structural formula 1, or a pharmaceutically acceptable salt thereof.
22. The method of any one of embodiments 1-21, wherein the myeloproliferative neoplasm is a ruxolitinib-resistant myeloproliferative neoplasm.

## Claims

1. A compound represented by the following structural formula (1): 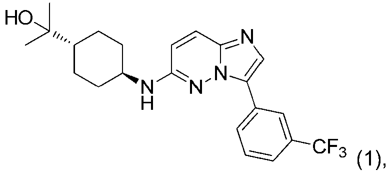
or a pharmaceutically acceptable salt thereof,
for use in a method for treating a myeloproliferative neoplasm in a mammal in need thereof, the method comprising administering to the mammal an effective amount of the compound of structural formula (1) or the pharmaceutically acceptable salt thereof.

2. The compound for use according to claim 1, comprising administering to the mammal from about 250 mg to about 2.5 g per day of the compound of structural formula (1), or the pharmaceutically acceptable salt thereof,
optionally comprising administering to the mammal from about 300 mg to about 1.5 g per day of the compound of structural formula (1), or the pharmaceutically acceptable salt thereof,
further optionally comprising administering to the mammal from about 450 mg to about 1.5 g per day of the compound of structural formula (1), or the pharmaceutically acceptable salt thereof.

3. The compound for use according to claim 1 or 2, wherein the mammal is on a JAK inhibitor regimen.

4. The compound for use according to claim 3, wherein the JAK inhibitor is a JAK1 inhibitor, a JAK2 inhibitor, a JAK3 inhibitor, or a combination of any of the above.

5. The compound for use according to claim 3 or 4, wherein the JAK inhibitor is ruxolitinib, tofacitinib, oclacitinib, baricitinib, filgotinib, gandotinib, lestaurtinib, momelotinib, pacritinib, PF-04965842, updacitinib, perficitinib, fedratinib, cucurbitacin I, CHZ868, decernotinib, CEP-33779, R348, fibotinib, ABT-494, BMS-911543, ASN002, itacitinib, NS-018, AZD1480, or a pharmaceutically acceptable salt thereof.

6. The compound for use according to any of claims 3, 4 or 5, wherein the JAK inhibitor is selected from the group consisting of ruxolitinib, gandotinib, lestaurtinib, momelotinib, pacritinib, fedratinib, or a pharmaceutically acceptable salt thereof.

7. The compound for use according to any of claims 3 to 6, wherein the JAK inhibitor is momelotinib or a pharmaceutically acceptable salt thereof.

8. The compound for use according to any of claims 3 to 6, wherein the JAK inhibitor is ruxolitinib or a pharmaceutically acceptable salt thereof.

9. The compound for use according to any of claims 3 to 7, wherein the JAK inhibitor is at a dose of
(i) about 1 mg to about 200 mg per day,
(ii) about 1 mg to about 100 mg per day,
(iii) about 5 mg to about 60 mg per day, or
(iv) about 10 mg to about 50 mg per day.

10. The compound for use according to any of the preceding claims, wherein the myeloproliferative neoplasm is myelofibrosis,
optionally wherein the myelofibrosis is intermediate-risk myelofibrosis or high-risk myelofibrosis,
further optionally wherein the myelofibrosis is primary myelofibrosis or secondary myelofibrosis.

11. The compound for use according to any of the preceding claims, wherein the compound of structural formula (1), or the pharmaceutically acceptable salt thereof, is administered orally.

12. The compound for use according to any of the preceding claims, wherein the compound of structural formula (1), or the pharmaceutically acceptable salt thereof, is administered
(i) once daily, or
(ii) twice daily.

13. The compound for use according to any of the preceding claims, wherein the compound of structural formula (1), or the pharmaceutically acceptable salt thereof, is administered for about 7 days to about one year.
optionally wherein the compound of structural formula (1), or the pharmaceutically acceptable salt thereof, is administered for
(i) 28 days, or
(ii) one year.

14. The compound for use according to any of the preceding claims, wherein the compound of structural formula (1) is the pharmaceutically acceptable salt, optionally wherein the pharmaceutically acceptable salt is an HCl salt.

15. The compound for use according to any of the preceding claims, wherein the mammal is a human.
